# NEUE EUROPÄISCHE PATENTSCHRIFT

(11) **EP 3 225 112 B2**
(45) Veröffentlichungstag und Bekanntmachung des Hinweises auf die Entscheidung über den Einspruch: **26.03.2025**
(45) Hinweis auf die Patenterteilung: 18.08.2021
(21) Anmeldenummer: 16163551.1
(22) Anmeldetag: 01.04.2016
(51) Int. Cl.: A61P 31/04, A61P 31/10, A61P 33/00, A61Q 11/00, A01N 25/04, A01N 25/30, A01N 25/00, A61K 8/49, A61K 8/41, A61K 8/43, A61K 6/52, A61K 6/62, A61K 6/69, A61P 1/02, A01P 1/00, A61Q 11/02

(54) **PHOTOSENSIBILISATOR-DISPERSION UND VERWENDUNG DERSELBEN**
PHOTOSENSITISER DISPERSION AND USE OF THE SAME
DISPERSION DE PHOTOSENSIBILISANT ET SON UTILISATION

(43) Veröffentlichungstag der Anmeldung: 04.10.2017
(73) Patentinhaber: TriOptoTec GmbH, 93053 Regensburg (DE)
(72) Erfinder: Bäumler, Wolfgang, 84085 Langquaid (DE); Jung, Christiane, 91731 Dorfkemmathen (DE); König, Burkhard, 93138 Lappersdorf (DE); Kunz, Werner, 93051 Regensburg (DE); Müller, Eva, 93444 Bad Kötzting (DE); Späth, Andreas, 93055 Regensburg (DE)
(74) Vertreter: Louis Pöhlau Lohrentz

(56) Entgegenhaltungen:
- EP-A1- 0 390 743
- WO-A1-2006/135344
- WO-A1-2009/123575
- WO-A2-2012/035480
- DE-T2- 60 222 421
- DATABASE WPI Week 201379, Derwent World Patents Index; AN 2013-N15563, XP002759899

## Beschreibung

Die vorliegende Erfindung betrifft eine Photosensibilisator-haltige Dispersion sowie deren Verwendung.

Durch das Auftreten immer neuer multiresistenter Bakterien-Isolate ist die Therapie bakterieller Erkrankungen schwieriger geworden. Zunehmend strengere Hygienestandards und eine weltweite Ausbreitung an nosokomialen Infektionen haben das Interesse für neue Präparate, Methoden und Anwendungen, die eine Ausbreitung von multiresistenten Keimen verhindern könnten, geweckt.

Die Forschung nach Alternativen zur Antibiotikatherapie ist von enormer Bedeutung für die Behandlung von Infektionen, die beispielsweise durch Bakterien verursacht werden, insbesondere infolge der Identifizierung und dem vermehrtem Auftreten vacomycinresistenter Bakterienstämme (VRSA) bereits im Jahr 2002 in Japan und den USA. In Europa wurde 2013 das erste VRSA Patientenisolat in Portugal erhalten.

Auch die Zunahme von Resistenzen bei Pilzinfektionen gegenüber Antipilzpräparaten verschärft zusehends die Problematik bei der Behandlung von oberflächlichen Infektionen. Die klinische Konsequenz der Resistenz gegenüber Antipilzpräparaten zeigt sich durch ein Versagen der Behandlung, ganz besonders bei immunsupprimierten Patienten.

Neue Ansätze zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei photooxidative Prozesse eine entscheidende Rolle.

Ein Photosensibilisator wird durch Licht einer bestimmten Wellenlänge angeregt. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff vorzugsweise oxidationsempfindliche Moleküle angegriffen. Oxidationsempfindliche Moleküle sind beispielsweise Moleküle, die Doppelbindungen oder oxidationsempfindliche Gruppen wie Phenole, Sulfide oder Thiole enthalten. Besonders anfällig für eine Schädigung sind insbesondere ungesättigte Fettsäuren in den Membranen von Bakterien.

Die DE 602 22 421 T2 offenbart eine injizierbare galenische Formulierung zur Verwendung in einer photodynamischen Diagnose oder Therapie sowie ihr Herstellungsverfahren.

Die WO 2006/135344 A1 beschreibt eine lichtsensibilisierende Zusammensetzung und ihre Verwendungen. Die WO 2009/123575 A1 offenbart ebenfalls eine lichtsensibilisierende Zusammensetzung und ihre Verwendungen.

Viele aus dem Stand der Technik bekannte Photosensibilisatoren weisen jedoch nachteiligerweise eine nicht ausreichende Benetzungsfähigkeit von hydrophoben Oberflächen auf.

Somit soll eine Photosensibilisator-haltige Zusammensetzung bereitgestellt werden, die eine einfache Auftragbarkeit gewährleistet und insbesondere zugleich eine gute Benetzungsfähigkeit auch von hydrophoben Oberflächen zeigt.

Weiterhin soll die Photosensibilisator-haltige Zusammensetzung nach dem Auftragen auf eine Oberfläche, vorzugsweise eine verbesserte Adhäsion des Photosensibilisators gewährleisten.

Weiterhin soll vorzugsweise die Wirksamkeit des Photosensibilisators verbessert werden.

Dabei soll die Photosensibilisator-haltige Zusammensetzung im Wesentlichen nicht die Anregung der in der Zusammensetzung enthaltenen Photosensibilisator-Moleküle durch Licht einer bestimmten Wellenlänge behindern.

Die Aufgabe der vorliegenden Erfindung wird gelöst durch die Bereitstellung einer Photosensibilisator-haltigen Dispersion nach Anspruch 1, wobei die Dispersion:
(a) wenigstens einen Photosensibilisator, der aus der Gruppe, die aus Phenalenonen, Flavinen und Mischungen davon besteht, ausgewählt wird,
(b) wenigstens eine flüssige, polare Phase, und
(c) wenigstens ein Tensid
umfasst, und wobei die Dispersion bei einer Temperatur aus einem Bereich von 2 bis 50°C und einem Druck in einem Bereich von 800 bis 1200 mbar eine Mikroemulsion, ein Gel oder eine Mischung davon umfasst.

Bevorzugte Ausführungsformen der erfindungsgemäßen Dispersion sind in den Ansprüchen 1 bis 14 angegeben.

Die Aufgabe der vorliegenden Erfindung wird weiterhin gelöst durch die nicht-medizinische Verwendung einer Dispersion nach einem der Ansprüche 1 bis 14 zur Inaktivierung von Mikroorganismen, die vorzugsweise aus der Gruppe, die aus Viren, Archäeen, Bakterien Bakteriensporen, Pilzen, Pilzsporen, Protozoen, Algen und blutübertragbaren Parasiten besteht, ausgewählt werden.

Bevorzugte Ausführungsformen der erfindungsgemäßen Verwendung sind in den abhängigen Ansprüchen 16 bis 18 spezifiziert.

Die Aufgabe der vorliegenden Erfindung wird weiterhin gelöst durch die Bereitstellung eines nicht-medizinischen Verfahrens nach Anspruch 19 zur Inaktivierung von Mikroorganismen, die vorzugsweise aus der Gruppe, die aus Viren, Archäeen, Bakterien Bakteriensporen, Pilzen, Pilzsporen, Protozoen, Algen und blutübertragbaren Parasiten besteht, ausgewählt werden, wobei das Verfahren folgende Schritte umfasst:
(A) Inkontaktbringen der Mikroorganismen mit einer Photosensibilisator-haltigen Dispersion nach einem der Ansprüche 1 bis 14, und
(B) Bestrahlen der Mikroorganismen und des wenigstens einen Photosensibilisators mit elektromagnetischer Strahlung geeigneter Wellenlänge und Energiedichte.

Die Aufgabe der vorliegenden Erfindung wird weiterhin gelöst durch die Bereitstellung einer Dispersion nach einem der Ansprüche 1 bis 14 zur Anwendung nach Anspruch 20 bei der photodynamischen Therapie zur Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden.

Eine bevorzugte Ausführungsform der erfindungsgemäßen Dispersion nach einem der Ansprüche 1 bis 14 zur Anwendung nach Anspruch 20 ist im abhängigen Anspruch 21 spezifiziert.

Vorzugsweise wird das erfindungsgemäße Verfahren zur Inaktivierung von Mikroorganismen bei der photodynamischen Therapie eines Patienten oder bei der photodynamischen Entkeimung einer Oberfläche eines Gegenstandes oder eines Raumes oder bei der photodynamischen Entkeimung einer Flüssigkeit, vorzugsweise bei der photodynamischen Entkeimung einer Oberfläche eines Gegenstandes oder bei der photodynamischen Entkeimung einer Flüssigkeit, durchgeführt.

Eine erfindungsgemäße Photosensibilisator-haltige Dispersion umfasst:
(a) wenigstens einen Photosensibilisator,
(b) wenigstens eine flüssige, polare Phase, und
(c) wenigstens ein Tensid,
wie in Anspruch 1 spezifiziert.

Die Erfinder haben festgestellt, dass durch die Bereitstellung einer erfindungsgemäßen Photosensibilisator-haltigen Dispersion eine Vielzahl unterschiedlicher Photosensibilisator-Klassen in effizienter Weise auch auf hydrophoben Oberflächen aufgebracht werden können. Vorzugsweise kann dabei eine für die photodynamische Inaktivierung notwendige Menge an Photosensibilisator auf der zu behandelnden Oberfläche aufgebracht werden.

Weiterhin weist die erfindungsgemäße Dispersion für unterschiedliche Photosensibilisator-Klassen eine ausreichende Benetzbarkeit auch von hydrophoben Oberflächen auf, so dass die erfindungsgemäße Photosensibilisator-haltige Dispersion und somit der darin enthaltene wenigstens eine Photosensibilisator vorzugsweise gleichmäßig auf der zu entkeimenden Oberfläche verteilt werden kann und vorzugsweise nach dem Aufbringen haften bleibt.

Dadurch ist vorzugsweise gewährleistet, dass nach Bestrahlung der zu entkeimenden Oberfläche mit elektromagnetischer Strahlung geeigneter Wellenlänge und Energiedichte, vorzugsweise in Gegenwart von Sauerstoff und/oder einer sauerstoffabgebenden Verbindung, auf der zu entkeimenden Oberfläche anhaftende Mikroorganismen zuverlässig inaktiviert werden.

Darüber hinaus haben die Erfinder überrascht festgestellt, dass die erfindungsgemäße Dispersion die photodynamische Aktivität des darin enthaltenden wenigstens einen Photosensibilisators nicht verringert.

Die erfindungsgemäße Photosensibilisator-haltige Dispersion weist eine geringe bis keine Trübung auf, wodurch eingestrahlte elektromagnetische Strahlung kaum oder nicht abgeschwächt wird.

Daher führt die Verwendung des wenigstens einen Photosensibilisators in der erfindungsgemäßen Dispersion im Vergleich zum Einsatz des reinen Photosensibilisators überraschenderweise zu keiner signifikanten Absenkung der Ausbeute an reaktiven Sauerstoffspezies und/oder Singulett-Sauerstoff.

Eine möglichst hohe Ausbeute an reaktiven Sauerstoffspezies oder Singulett-Sauerstoff ist für eine antimikrobielle Wirksamkeit bei der photodynamischen Therapie oder bei der photodynamischen Reinigung von Oberflächen oder Flüssigkeiten erwünscht.

Eine stärkere Trübung führt zu einer signifikanten Reduzierung der Energie der eingestrahlten elektromagnetischen Strahlung. Darüber hinaus führt das Auftreten von Auslöschungsvorgängen ("Quenching") innerhalb einer Photosensibilisator-Zusammensetzung nach Bestrahlung mit elektromagnetischer Strahlung geeigneter Wellenlänge und Energiedichte zu einer Abgabe der aufgenommen Energie, üblicherweise durch das Auftreten von Fluoreszenzeffekten, strahlungsloser Relaxation und/oder Abgabe von Wärme an die Umgebung.

Dadurch kommt es zu einer signifikanten Verringerung der photodynamischen Effizienz, d.h. zu einer Verringerung der durch photodynamische Prozesse gebildeten reaktiven Sauerstoffspezies (ROS) und/oder des durch photodynamische Prozesse gebildeten angeregten molekularen Sauerstoffs.

Bei einer bevorzugten Ausführungsform der erfindungsgemäßen Photosensibilisator-Dispersion umfasst diese neben dem wenigstens einen Photosensibilisator keine weiteren organischen Verbindungen, die ungesättigte Gruppen, beispielsweise in Form von Doppelbindungen und/oder Dreifachbindungen aufweisen, sowie keine weiteren organischen Verbindungen, die oxidierbare Gruppen, beispielsweise in Form von Thiol-Gruppen und/oder Aldehyd-Gruppen, aufweisen, da diese Reste oder Gruppen mit Singulett-Sauerstoff oder den gebildeten reaktiven Sauerstoff-Spezies reagieren und die Quantenausbeute reduzieren können.

Beispielsweise umfasst die erfindungsgemäßen Photosensibilisator-Dispersion neben dem wenigstens einen Photosensibilisator keine weiteren oxidierbaren Aromaten wie Phenole, Polyphenole, Anilin oder Phenylendiamine, sowie keine weiteren aktivierten Aminosäuren, wie Histidin oder Tryptophan, kein Imidazol, keine Alkylsulfide oder keine Thioether.

Als "Photosensibilisator" im Sinn der Erfindung werden Verbindungen verstanden, die elektromagnetische Strahlung, vorzugsweise sichtbares Licht, UV-Licht und/oder Infrarotlicht, absorbieren und sodann reaktive Sauerstoffspezies (ROS), vorzugsweise freie Radikale und/oder Singulett-Sauerstoff, aus Triplett-Sauerstoff erzeugen.

Unter dem Begriff "photodynamische Therapie" wird erfindungsgemäß die lichtinduzierte Inaktivierung von Zellen oder Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, auf und/oder in Patienten verstanden.

Unter dem Begriff "photodynamische Entkeimung" wird erfindungsgemäß die lichtinduzierte Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, auf Oberflächen von Gegenständen, Räumen und/oder Lebensmitteln und/oder in Flüssigkeiten, insbesondere in Wasser, Brauchwasser, Grauwasser, Regenwasser, Prozesswasser etc. verstanden.

Unter dem Begriff "Oberflächenreinigung" wird erfindungsgemäß die Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, auf Oberflächen von Gegenständen, Räumen und/oder Lebensmitteln verstanden. Der Begriff "Oberflächenreinigung und/oder -beschichtung" umfasst erfindungsgemäß nicht Oberflächen auf einem menschlichen oder tierischen Körper, wie beispielsweise die Haut; und/oder in einem menschlichen oder tierischen Körper, wie beispielsweise die äußere, apikale Seite des Epithels eines Hohlorgans.

Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart einer erfindungsgemäßen Dispersion bestimmt werden.

Erfindungsgemäß wird unter einer Reduzierung der Lebensfähigkeit verstanden, dass die Anzahl der Mikroorganismen um wenigstens 80,0 %, vorzugsweise wenigstens 99,0 %, vorzugsweise wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 % verringert wird.

Vorzugsweise wird die Reduzierung der Anzahl der Mikroorganismen gemäß Boyce, J. M. und Pittet, D. ("Guidelines for hand hygiene in healthcare settings. Recommendations of the Healthcare Infection Control Practices Advisory Committee and the HIPAC/SHEA/APIC/IDSA Hand Hygiene Task Force", Am.J.Infect.Control 30 (8), 2002, Seite 1 -46) als log₁₀-Reduktionsfaktor angegeben.

Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart einer erfindungsgemäßen Dispersion verstanden.

Geeignete Methoden zur Bestimmung des log₁₀-Reduktionsfaktors sind beispielsweise in der DIN EN 14885:2007-01 "Chemische Desinfektionsmittel und Antiseptika - Anwendung Europäischer Normen für chemische Desinfektionsmittel und Antiseptika" oder in Rabenau, H. F. und Schwebke, I. ("Leitlinie der Deutschen Vereinigung zur Bekämpfung der Viruskrankheiten (DVV) e. V. und der Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitsschutz 51(8), (2008), Seiten 937 - 945) beschrieben.

Vorzugsweise beträgt der log₁₀-Reduktionsfaktor nach einer Bestrahlung von Mikroorganismen mit elektromagnetischer Strahlung in Gegenwart einer erfindungsgemäßen Dispersion mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀, noch weiter bevorzugt mindestens 7,5 log₁₀.

Beispielsweise bedeutet eine Reduktion der Anzahl der Mikroorganismen nach einer Bestrahlung dieser Mikroorganismen mit elektromagnetischer Strahlung in Gegenwart einer erfindungsgemäßen Dispersion um 2 Zehnerpotenzen, bezogen auf die Ausgangsmenge dieser Mikroorganismen, einen log₁₀-Reduktionsfaktor von 2 log₁₀.

Weiter bevorzugt wird die Anzahl der Mikroorganismen nach einer Bestrahlung dieser Mikroorganismen mit elektromagnetischer Strahlung in Gegenwart einer erfindungsgemäßen Dispersion um mindestens 1 Zehnerpotenz, weiter bevorzugt um mindestens 2 Zehnerpotenzen, weiter bevorzugt um mindestens 3 Zehnerpotenzen, vorzugsweise um mindestens 4 Zehnerpotenzen, weiter bevorzugt um mindestens 5 Zehnerpotenzen, weiter bevorzugt um mindestens 6 Zehnerpotenzen, noch weiter bevorzugt um mindestens 7 Zehnerpotenzen, jeweils bezogen auf die Ausgangsmenge dieser Mikroorganismen, verringert.

Unter dem Begriff "Mikroorganismen" werden im Sinne der Erfindung insbesondere Viren, Archäeen, prokaryote Mikroorganismen, wie Pilze, Protozoen, Pilzsporen, einzellige Algen, verstanden. Die Mikroorganismen können dabei einzellig oder mehrzellig, beispielsweise als Pilzmycel, auftreten.

Eine erfindungsgemäße Photosensibilisator-Dispersion umfasst (a) wenigstens einen Photosensibilisator.

Bei einer bevorzugten Ausführungsform ist der wenigstens eine Photosensibilisator positiv geladen, negativ geladen, ungeladen oder eine Mischung davon. Weiter bevorzugt weist der wenigstens eine Photosensibilisator wenigstens einen organischen Rest mit a) wenigstens einem neutralen, protonierbaren Stickstoffatom und/oder b) wenigstens einem positiv geladenen Stickstoffatom auf.

Erfindungsgemäß wird der wenigstens eine Photosensibilisator aus der Gruppe, die aus Phenalenonen, Flavinen und Mischungen davon, besteht, ausgewählt.

Geeignete Phenalenone werden beispielsweise in der EP 2 678 035 A2 offenbart, deren Inhalt hiermit hinsichtlich Struktur und Synthese geeigneter Phenalenone unter Bezugnahme aufgenommen wird.

Vorzugsweise wird ein geeignetes Phenalenon-Derivat aus der Gruppe, die aus den Verbindungen mit den Formeln (2) bis (25) und Mischungen davon besteht, ausgewählt:

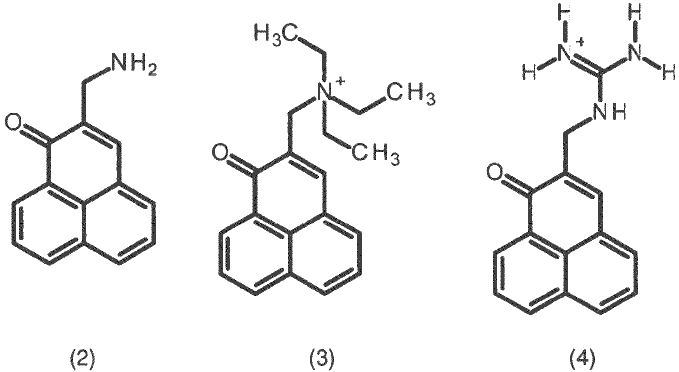

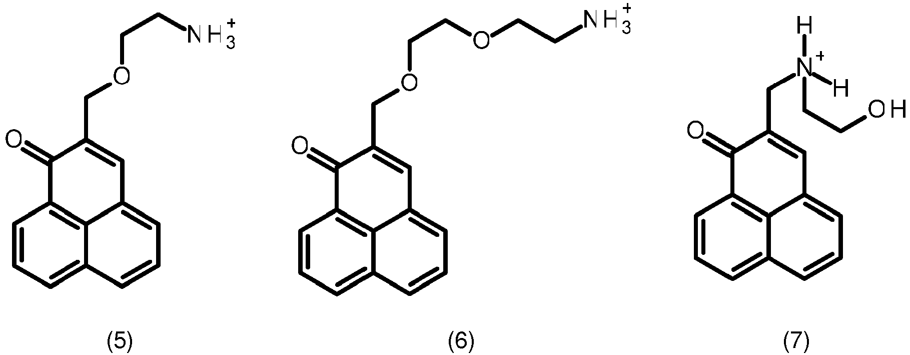

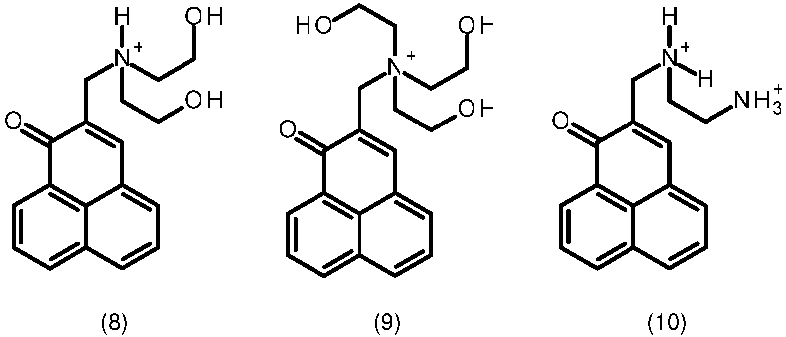

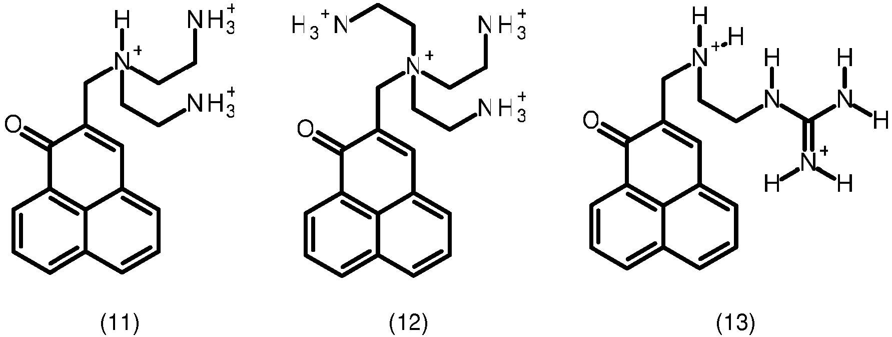

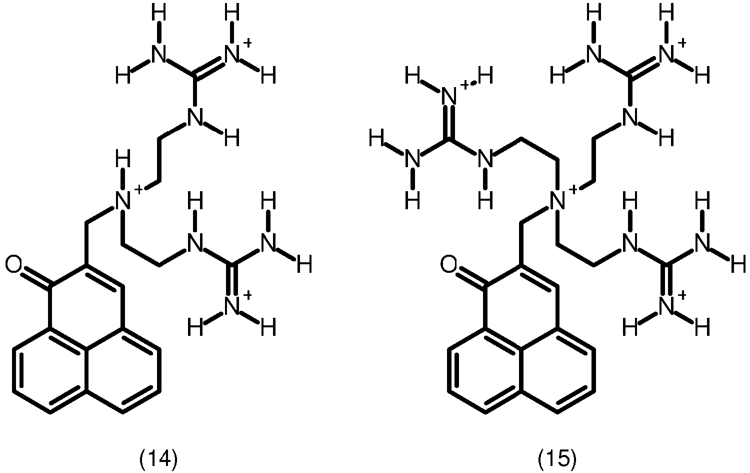

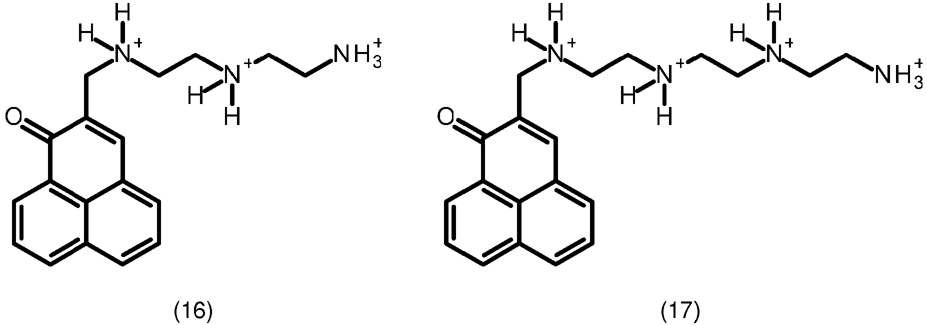

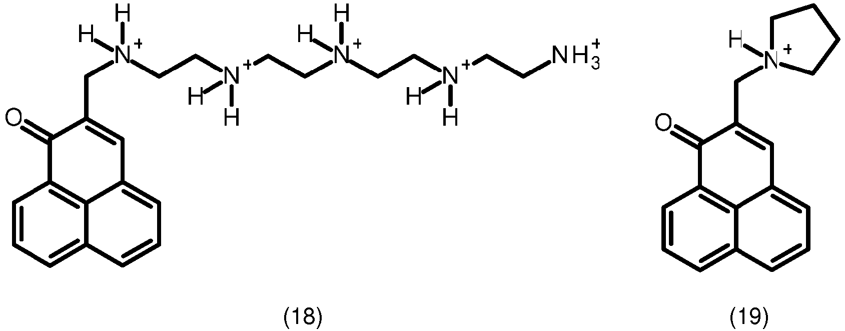

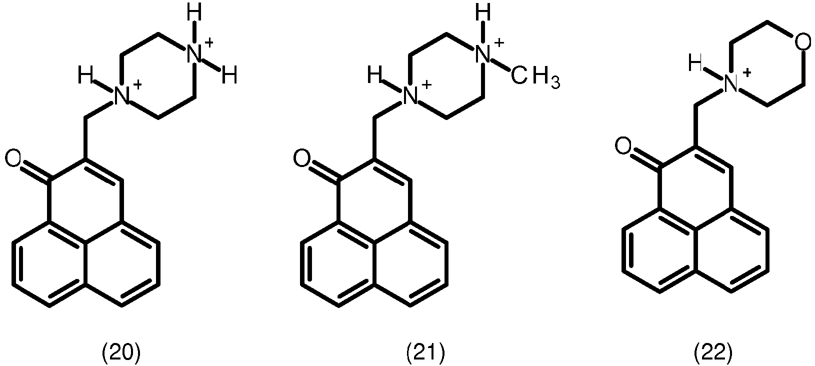

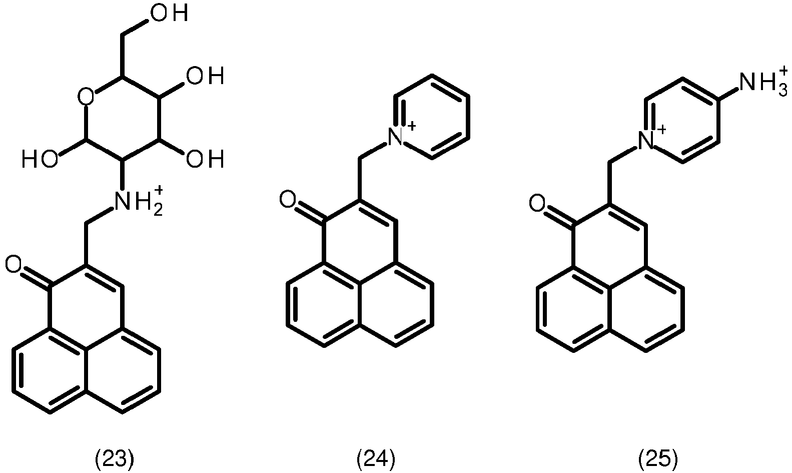

Vorzugsweise wird ein geeignetes Phenalenon-Derivat weiterhin aus der Gruppe, die aus den Verbindungen mit den Formeln (26) bis (28) und Mischungen davon besteht, ausgewählt:

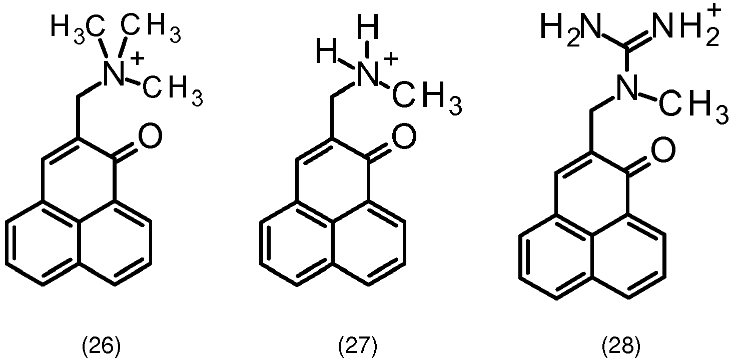

Weiter bevorzugt wird ein geeignetes Phenalenon-Derivat aus der Gruppe, die aus den Verbindungen mit den Formeln (2) bis (28) und Mischungen davon besteht, ausgewählt.

Geeignete Flavine werden beispielsweise in EP 2 723 342 A1, EP 2 723 743 A1 und EP 2 723 742 A1 offenbart, deren Inhalt hinsichtlich Struktur und Synthese geeigneter Flavine hiermit unter Bezugnahme aufgenommen wird.

Vorzugsweise wird ein geeignetes Flavin-Derivat aus der Gruppe, die aus den Verbindung mit den Formeln (32) bis (49), (51) bis (64) und Mischungen davon besteht, ausgewählt:

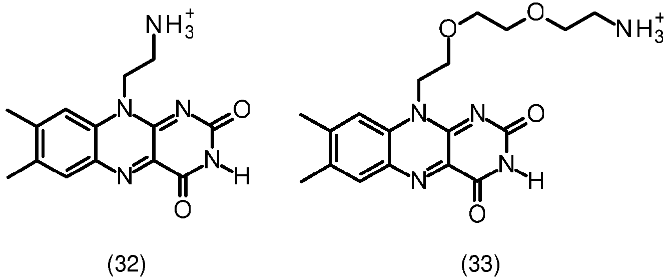

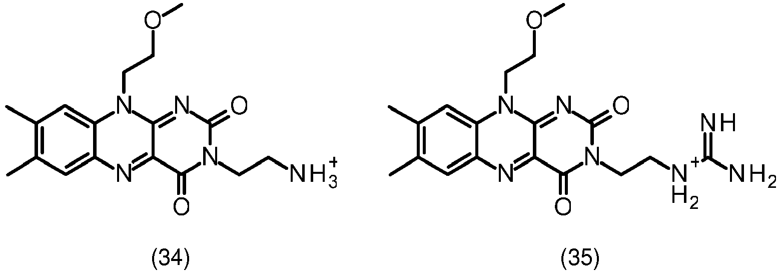

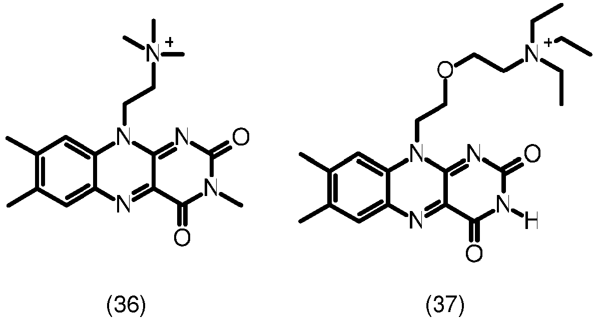

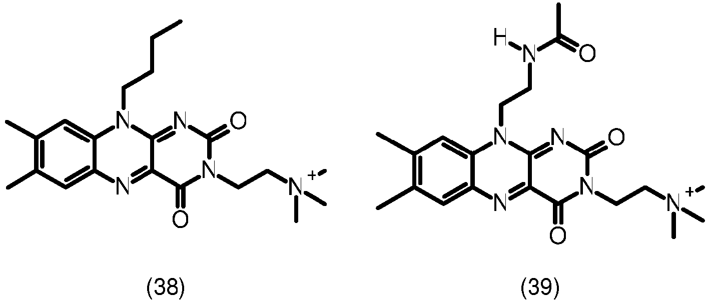

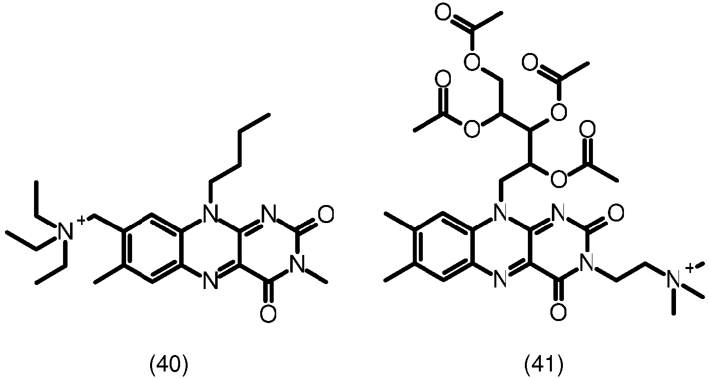

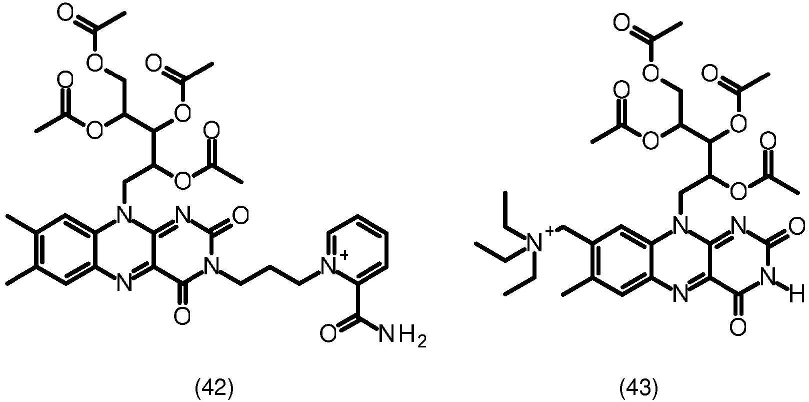

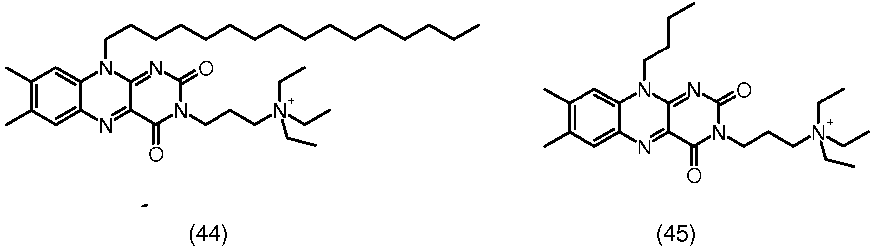

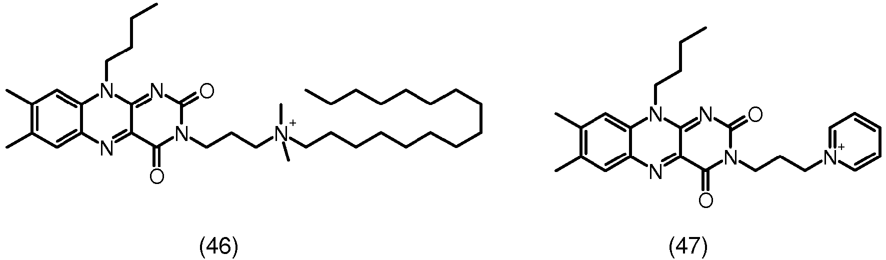

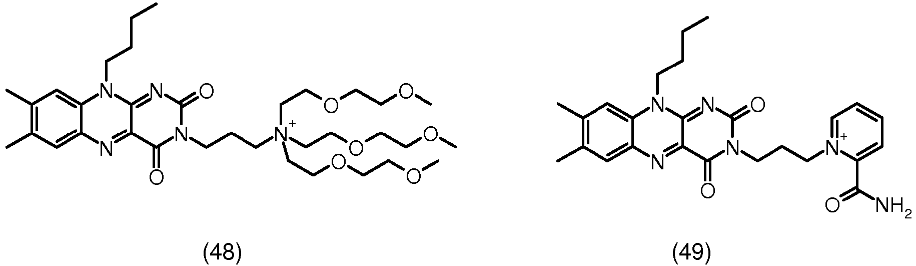

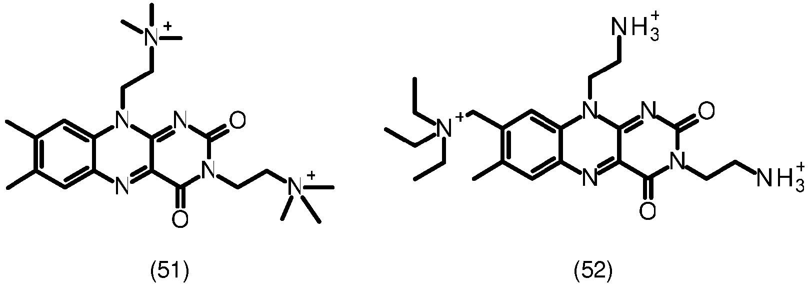

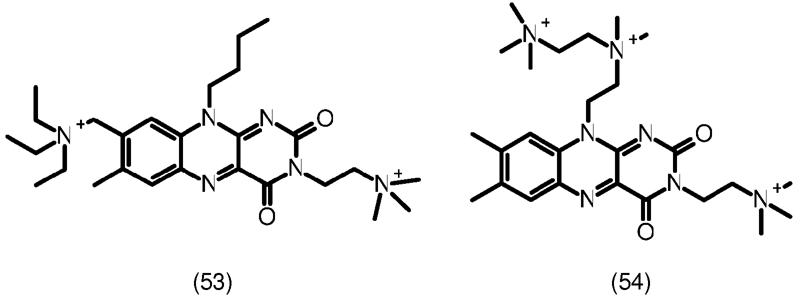

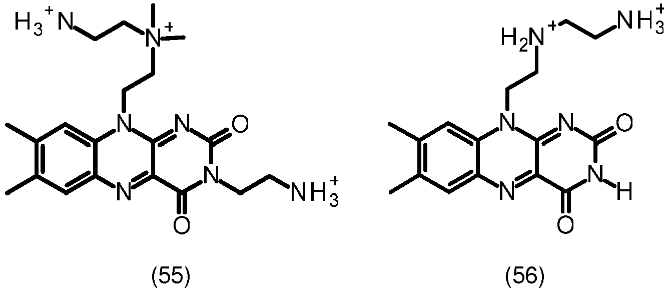

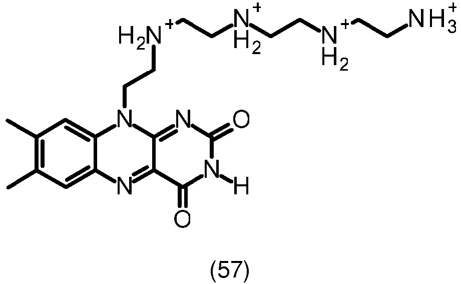

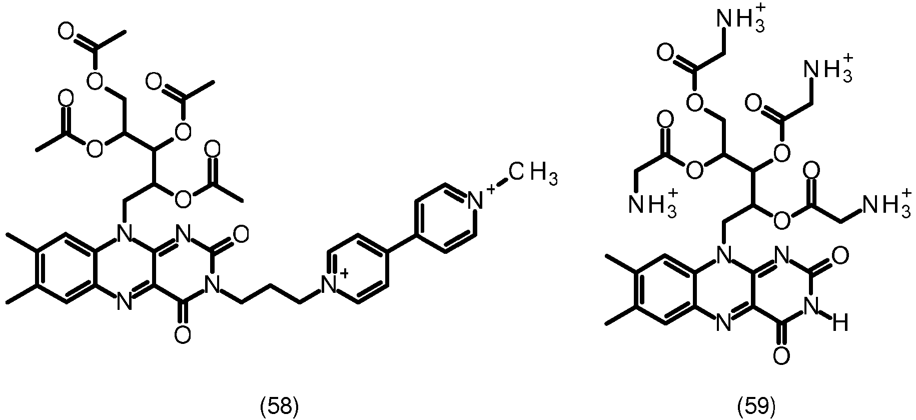

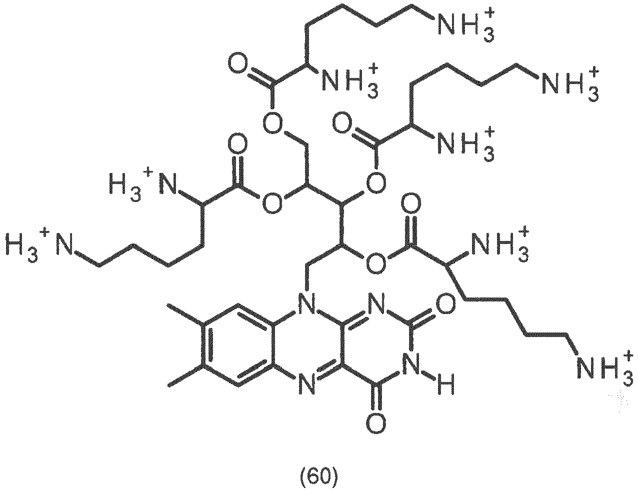

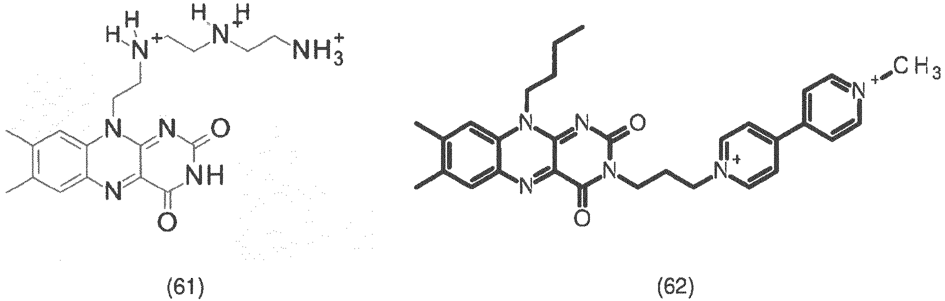

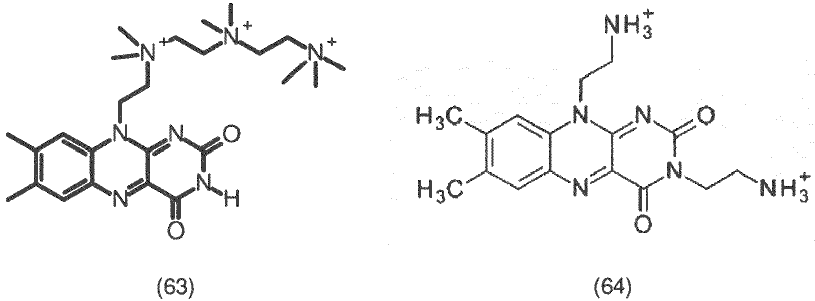

Kommerziell erhältliche Phenothiazinium-Farbstoffe sind beispielsweise Neu-Methylenblau (NMB; 3,7-Bis(ethylamino)-2,8-dimethylphenothiazin-5-iumchlorid), 1,9-Dimethyl-Methylenblau (DMMB; 3,7-Bis-(dimethylamino)-1,9-dimethyl-diphenothiazin-5-ium - Zinkchlorid) oder Methylengrün (Basic Green 5, [7-(Dimethylamino)-4-nitrophenothiazin-3-yliden]-dimethylazaniumchlorid).

Kommerziell erhältliche Polymethin-Farbstoffe sind beispielsweise Cyanin-5 (Cy5), Cyanin-3 (Cy3) oder Indocyaningrün (ICG).

Kommerziell erhältliche Xanthen-Farbstoffe sind beispielsweise Pyronin G, Eosin B, Eosin Y, Rose Bengal, Erythrosin (E127) oder Phloxin B,
Kommerziell erhältliche Triphenylmethan-Farbstoffe sind beispielsweise Patentblau V (4-[4,4'-Bis(diethylamino)-α-hydroxy-benzhydryl]-6-hydroxy-benzol-1,3-disulfonsäure), Malachitgrün (N,N,N',N'-Tetramethyl-4,4'-diaminotriphenylcarbeniumchlorid), Fuchsin (4-[(4-Aminophenyl)-(4-imino-1-cyclohexa-2,5-dienylidene)methyl]anilinhydrochlorid), Parafuchsin (4,4'-(4-Iminocyclohexa-2,5- dienylidenmethylen)dianilinhydrochlorid), Kristallviolett ((4-(4,4'-Bis(dimethylaminophenyl)benzhydryliden)cyclohexa-2,5-dien-1-yliden)dimethylammoniumchlorid).

Kommerziell erhältliche Anthrachinon-Farbstoffe sind beispielsweise (1,2-Dihydroxyanthrachinon) oder Indanthren (6,15-Dihydro-5,9,14,18-anthrazinetetron). Kommerziell erhältliche Porphyrin-Farbstoffe sind beispielsweise 5,10,15,20-Tetrakis(1-methyl-4-pyridinio)porphyrin-tetra(p-toluenesulfonat) (TMPyP), oder Tetrakis(p-trimethylammoniumphenyl)porphyrinchlorid.

Kommerziell erhältliche Phthalocyanin-Farbstoffe sind beispielsweise Zinkphthalocyanintetrasulfonat oder Tetrakis(p-trimethylammonium)phthalocyanin-Zinkchlorid,

Kommerziell erhältliche Indamin-Farbstoffe sind beispielsweise Safranin T (3,7-Diamino-2,8-dimethyl-5-phenylphenaziniumchlorid) oder Phenosafranin (3,7-Diamino-5-phenylphenaziniumchloride)
Kommerzielle Bezugsquelle für vorgenannte Farbstoffe sind beispielsweise AppliChem GmbH (Darmstadt, DE), Frontier Scientific Inc. (Logan, UT, USA), GE Healthcare Europe GmbH (Freiburg, DE), Sigma-Aldrich Corporation (St. Louis, MO, USA) oder Merck KGaA (Darmstadt, DE).

Als Gegenion zum positiv geladenen Stickstoffatom kann jedes geeignete Anion verwendet werden. Vorzugsweise werden als Gegenion zum positiv geladenen Stickstoffatom Anionen verwendet, die aus der Gruppe, die aus Fluorid, Chlorid, Bromid, Iodid, Sulfat, Hydrogensulfat, Phosphat, Dihydrogenphosphat, Hydrogenphosphat, Tosylat, Mesylat, Formiat, Acetat, Propionat, Butanoat, Oxalat, Tartrat, Fumarat, Benzoat, Citrat und/oder Mischungen davon besteht.

Vorzugsweise wird der wenigstens eine Photosensibilisator aus der Gruppe, die aus den Verbindungen mit den Formeln (2) bis (25), (32) bis (49), (51) bis (64), und Mischungen davon besteht, ausgewählt.

Vorzugsweise umfasst die Dispersion den wenigstens einen Photosensibilisator in einer Konzentration aus einem Bereich von 0,1 µM bis 1000 µM.

Eine erfindungsgemäße Dispersion umfasst weiterhin (b) wenigstens eine flüssige, polare Phase.

Vorzugsweise liegt die wenigstens eine flüssige, polare Phase bei einer Temperatur aus einem Bereich von 0°C bis 100°C und einem Druck aus einem Bereich von 800 bis 1200 mbar im flüssigen Aggregatzustand vor.

Vorzugsweise umfasst die wenigstens eine flüssige, polare Phase wenigstens ein polares Lösungsmittel, vorzugsweise Wasser.

Vorzugsweise umfasst eine erfindungsgemäße Dispersion das wenigstens eine polare Lösungsmittel, vorzugsweise Wasser, in einem Anteil von wenigstens 0,1 Gew.-%, vorzugsweise von wenigstens 0,5 Gew.-%, weiter bevorzugt von wenigstens 1 Gew.-%, weiter bevorzugt von wenigstens 4 Gew.-%, weiter bevorzugt von wenigstens 10 Gew.-%, weiter bevorzugt von wenigstens 35 Gew.-%, weiter bevorzugt von wenigstens 50 Gew.-%, weiter bevorzugt von wenigstens 51 Gew.-%, jeweils bezogen auf das Gesamtgewicht der Dispersion.

Vorzugsweise umfasst eine erfindungsgemäße Dispersion das wenigstens eine polare Lösungsmittel, vorzugsweise Wasser, in einem Anteil von aus einem Bereich von 0,1 Gew.-% bis 99,8 Gew.-%, vorzugsweise aus einem Bereich von 0,5 Gew.-% bis 99 Gew.-%, weiter bevorzugt aus einem Bereich von 4 Gew.-% bis 98 Gew.-%, weiter bevorzugt aus einem Bereich von 10 Gew.-% bis 97 Gew.-%, weiter bevorzugt aus einem Bereich von 35 Gew.-% bis 96 Gew.-%, weiter bevorzugt aus einem Bereich von 50 Gew.-% bis 95 Gew.-%, weiter bevorzugt aus einem Bereich von 51 Gew.-% bis 94 Gew.-%, weiter bevorzugt aus einem Bereich von 53 Gew.-% bis 93 Gew.-%, weiter bevorzugt aus einem Bereich von 70 Gew.-% bis 92 Gew.-%, jeweils bezogen auf das Gesamtgewicht der Dispersion.

Eine erfindungsgemäße Dispersion umfasst weiterhin (c) wenigstens ein Tensid.

Vorzugsweise umfasst eine erfindungsgemäße Dispersion das wenigstens eine Tensid in einem Anteil aus einem Bereich von 0,1 Gew.-% bis 65 Gew.-%, vorzugsweise aus einem Bereich von 1 Gew.-% bis 55 Gew.-%, weiter bevorzugt aus einem Bereich von 3 Gew.-% bis 50 Gew.-%, weiter bevorzugt aus einem Bereich von 5 Gew.-% bis 41 Gew.-%, weiter bevorzugt aus einem Bereich von 7 Gew.-% bis 37 Gew.-%, weiter bevorzugt aus einem Bereich von 9 Gew.-% bis 30 Gew.-%, weiter bevorzugt aus einem Bereich von 10 Gew.-% bis 27 Gew.-%, jeweils bezogen auf das Gesamtgewicht der Dispersion.

Das wenigstens eine Tensid wird vorzugsweise aus der Gruppe, die aus nichtionischen Tensiden, anionischen Tensiden, kationischen Tensiden, amphoteren Tensiden und Mischungen davon, vorzugsweise nichtionischen Tensiden, anionischen Tensiden und Mischungen davon, besteht, ausgewählt.

Das wenigstens eine Tensid weist vorzugsweise einen HLB-Wert aus einem Bereich von 4 bis 40, vorzugsweise aus einem Bereich von 5 bis 20, auf. Der HLB-Wert eines Tensids kann beispielsweise gemäß der in Griffin, W.C. (1949) ("Classification of Surface-Active Agents by 'HLB'", J. Soc. Cosmet. Chem. 1 (5), Seiten 311 bis 326) oder der in Griffin, W. C. (1954) ("Calculation of HLB Values of Non-Ionic Surfactants", J. Soc. Cosmet. Chem. 5 (4): Seiten 249 bis 256) beschriebenen Methoden bestimmt werden.

Vorzugsweise werden geeignete nichtionische Tenside aus der Gruppe, die aus Polyalkylenglycolether, Alkylglucoside, Alkylpolyglycoside, Alkylglycosidester, und Mischungen davon besteht, ausgewählt.

Geeignete Polyalkylenglycolether weisen vorzugsweise die allgemeine Formel (I):

CH₃-(CH₂)ₘ-(O-[CH₂]ₓ)ₙ-OH, (I)

auf,
wobei m = 8 - 20, vorzugsweise 10 - 16, bedeutet, wobei n = 1 - 25 bedeutet, wobei x = 1, 2, 3 oder 4 bedeutet.

Vorzugsweise wird eine Kombination unterschiedlicher Polyalkylenglycolether verwendet, beispielsweise mit unterschiedlichen Alkyloxy-Einheiten (-(O-[CH₂]ₓ)ₙ-).

Geeignete Polyalkylenglycolether sind beispielsweise Polyoxyethylenether des Laurylalkohols (Dodecan-1-ol), Polyoxyethylenether des Cetylalkohols (Hexadecan-1-ol), Polyoxyethylenether des Stearylalkohols (1-Octadecanol), Polyoxyethylenether des Oleylalkohols ((E)-Octadec-9-en-1-ol) oder Polyoxyethylenether von Gemischen des Stearylalkohols und des Cetylalkohols, (Cetylstearylalkohol).

Geeignete Polyalkylenglycolether sind beispielsweise unter den Handelsnamen: Brij, Thesit, Cremophor, Genapol, Magrogol, Lutensol etc. kommerziel erhältlich.

Geeignete Polyalkylenglycolether sind beispielsweise:

| Chemische Bezeichnung | INCI - Bezeichnung | Handelsname |
|---|---|---|
| Polyoxyethylen (4) laurylether | Laureth-4 (INCI) | Brij^{®} 30 |
| Polyoxyethylen (9) laurylether | Laureth-9 (INCI) | Thesit^{®} |
| Polyoxyethylen (23) laurylether | Laureth-23 (INCI) | Brij^{®} 35 |
| Polyoxyethylen (2) cetylether | Ceteth-2 (INCI) | Brij^{®} 52 |
| Polyoxyethylen (10) cetylether | Ceteth-10 (INCI) | Brij^{®} 56 |
| Polyoxyethylen (20) cetylether | Ceteth-20 (INCI) | Brij^{®} 58 |
| Polyoxyethylen (6) cetylstearylether | Ceteareth-6 (INCI) | Cremophor A6 |
| Polyoxyethylen (20) cetylstearylether | Ceteareth-20 (INCI) | |
| Polyoxyethylen (25) cetylstearylether | Ceteareth-25 (INCI) | Cremophor A25, |
| Polyoxyethylen (2) stearylether | Steareth-2 (INCI) | Brij^{®} 72 |
| Polyoxyethylen (10) stearylether | Steareth-10 (INCI) | Brij^{®} 76 |
| Polyoxyethylen (20) stearylether | Steareth-20 (INCI) | Brij^{®} 78 |
| Polyoxyethylen (2) oleylether | Oleth-2 (INCI) | Brij^{®} 92 |
| Polyoxyethylen (10) oleylether | Oleth-10 (INCI) | Brij^{®} 96 |
| Polyoxyethylen (20) oleylether | Oleth-20 (INCI) | Brij^{®} 98 |

Geeignete Alkylglucoside sind beispielsweise ethoxylierte Sorbitanfettsäureester (Polysorbate), die beispielsweise unter dem Handelsname Tween^{®} von Croda International Plc (Snaith, UK) kommerziell erhältlich sind.

| Chemische Bezeichnung | INCI - Bezeichnung | Handelsname |
|---|---|---|
| Polyoxyethylen-(20)-sorbitanmonolaurat | Polysorbat 20 | Tween^{®} 20 |
| Polyoxyethylen-(4)-sorbitanmonolaurat | Polysorbat 21 | Tween^{®} 21 |
| Polyoxyethylen-(20)-sorbitanmonopalmitat | Polysorbat 40 | Tween^{®} 40 |
| Polyoxyethylen-(20)-sorbitanmonostearat | Polysorbat 60 | Tween^{®} 60 |
| Polyoxyethylen-(4)-sorbitanmonostearat | Polysorbat 61 | Tween^{®} 61 |
| Polyoxyethylen-(20)-sorbitantristearat | Polysorbat 65 | Tween^{®} 65 |
| Polyoxyethylen-(20)-sorbitanmonooleat | Polysorbat 80 | Tween^{®} 80 |
| Polyoxyethylen-(5)-sorbitanmonooleat | Polysorbat 81 | Tween^{®} 81 |
| Polyoxyethylen-(20)-sorbitantrioleat | Polysorbat 85 | Tween^{®} 85 |
| Polyoxyethylen-(20)-sorbitanmonoisostearat | Polysorbat 120 | |

Ein weiteres geeignetes Alkylglucosid ist beispielsweise das Tensid Kosteran SQ/O VH, das kommerziell von der Dr. W. Kolb AG (Hedingen, CH) erhältlich ist. Kosteran SQ/O VH ist ein Sorbitan-Ölsäureester mit durchschnittlich 1,5 Ölsäuremolekülen pro Molekül (Sorbitan Sesquioleat

Ein weiteres geeignetes Alkylglucosid ist beispielsweise PEG-80 Sorbitanlaurat, ein ethoxylierter Sorbitanmonoester der Laurinsäure mit einem mittleren Ethylenoxid-Gehalt von 80 Mol Ethylenoxid pro Molekül. PEG-80 Sorbitanlaurat ist beispielsweise unter dem Handelsnamen Tween^{®} 28 von Croda International Plc kommerziell erhältlich. Geeignete Alkylglycosidester sind Fettsäureester von Methyl- oder Ethylglykosiden, beispielsweise Methylglycosidester und Ethylglycosidester, oder Saccharoseester.

Vorzugsweise werden geeignete anionische Tenside aus der Gruppe, die aus Alkylcarboxylaten, Alkylsulfonaten, Alkylsulfaten, Alkylphoshaten, Alkylpolyglykolethersulfaten, Sulfonaten von Alkylcarbonsäureestern, N-Alkyl-Sarkosinaten, und Mischungen davon besteht, ausgewählt.

Geeignete Alkylcarboxylate weisen vorzugsweise die allgemeine Formel (II):

H₃C-(CH₃)ₐ-CH₂-COO⁻ M⁺, (II)

auf,
wobei a = 5 - 21, vorzugsweise 8 - 16, bedeutet und wobei M⁺ ein wasserlösliches Kation, vorzugsweise Kation eines Alkalimetalls oder Ammonium, vorzugsweise Li+, Na⁺, K⁺ oder NH₄⁺, bedeutet.

Geeignete Alkylsulfonate weisen vorzugsweise 3-30 C-Atome auf. Geeignete Alkylsulfonate sind vorzugsweise Monoalkylsulfonate mit 8-20 C-Atomen, sekundäre Alkylsulfonate der allgemeinen Formel (III): 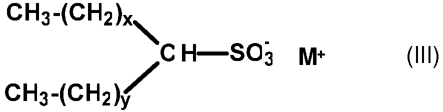 wobei x, y jeweils unabhängig von einander 0-17 bedeuten, wobei vorzugsweise x + y = 10 bis 20 bedeutet, und wobei M⁺ ein wasserlösliches Kation, vorzugsweise Kation eines Alkalimetalls oder Ammonium, vorzugsweise Li+, Na⁺, K⁺ oder NH₄⁺, bedeutet.

Geeignete Alkylsulfate weisen vorzugsweise mit der allgemeinen Formel (IV):

H₃C-(CH₃)_{d}-CH₂-O-SO₃⁻ M⁺, (IV)

auf,
wobei d = 6 - 20, vorzugsweise 8 - 18, bedeutet und wobei M⁺ ein wasserlösliches Kation, vorzugsweise Kation eines Alkalimetalls oder Ammonium, vorzugsweise Li+, Na⁺, K⁺ oder NH4⁺ bedeutet.

Ein geeignetes Alkylsulfat ist beispielsweise Natrium Dodecylsulfat (SDS).

Geeignete Alkylphosphate weisen vorzugsweise die allgemeine Formel (V):

H₃C-(CH₃)ₑ-CH₂-O-PO₃²⁻ 2 x M⁺, (V)

auf,
wobei e = 6 - 20, vorzugsweise 8-18 bedeutet und wobei M⁺ ein wasserlösliches Kation, vorzugsweise Kation eines Alkalimetalls oder Ammonium, vorzugsweise Li+, Na⁺, K⁺ oder NH₄⁺, bedeutet.

Geeignete Alkylpolyglykolethersulfate weisen vorzugsweise einen Alkylrest mit 6 bis 22 Kohlenstoffatomen, vorzugsweise 8 bis 20 Kohlenstoffatomen, auf und 1 bis 10 Ethylenoxideinheiten, vorzugsweise 2 bis 6 Ethylenoxideinheiten, im Etherteil auf.

Ein geeignetes N-Alkyl-Sarkosinat ist z.B. N-Lauroylsarcosinat.

Geeignete Sulfonate von Alkylcarbonsäureestern weisen vorzugsweise 6 bis 30 Kohlenstoffatome, vorzugsweise 8 bis 20 Kohlenstoffatome, auf.

Vorzugsweise umfassen geeignete Sulfonate von Alkylcarbonsäureestern wenigstens einen Alkylrest mit 6 bis 20 Kohlenstoffatomen, vorzugsweise 8 bis 18 Kohlenstoffatomen, und einen Alkylcarbonsäurerest mit 2 bis 10 Kohlenstoffatomen, vorzugsweise 2 bis 6 Kohlenstoffatomen, auf. Der Alkylrest kann Polyoxyethylen-(POE)-gruppen enthalten.

Geeignete Sulfonate von Alkylcarbonsäureestern sind beispielsweise Mono-Alkylestersulfosuccinate oder Di-Alkylestersulfosuccinate, z.B. Dioctylnatriumsulfosuccinat.

Vorzugsweise sind geeignete kationische Tenside quartäre Alkylammoniumsalze, Esterquats, acylierte Polyamine, Benzylammoniumsalze oder Mischungen davon.

Geeignete Alkylammoniumsalze weisen vorzugsweise die allgemeine Formel (VI) auf:

(R¹)(R²)(R³)(R⁴)N⁺ Z⁻ (VI)

wobei der organische Rest R¹ ein Alkylrest, der gradkettig oder verzweigt, vorzugsweise gradkettig, sein kann, mit 8 bis 20 C-Atomen, vorzugsweise mit 10 bis 18 C-Atomen, weiter bevorzugt mit 12 bis 16 C-Atomen, bedeutet, wobei die organischen Reste R², R³, und R⁴ jeweils unanhängig voneinander ein Alkylrest, der gradkettig oder verzweigt, vorzugsweise gradkettig, sein kann, mit 1 bis 20 C-Atomen, vorzugsweise mit 1 bis 16 C-Atomen, weiter bevorzugt mit 1 bis 12 C-Atomen, bedeutet, und wobei Z⁻ ein Anion bedeutet, das vorzugsweise aus der Gruppe, die aus Fluorid, Chlorid, Bromid, Iodid, Sulfat, Hydrogensulfat, Phosphat, Dihydrogenphosphat, Hydrogenphosphat, Tosylat, Mesylat, Formiat, Acetat, Propionat, Butanoat, Oxalat, Tartrat, Fumarat, Benzoat, Citrat und/oder Mischungen davon besteht, ausgewählt wird.

Vorzugsweise ist der organische Rest R¹ ein Alkylrest, der aus der Gruppe, die aus Octyl, Nonyl, Decyl, Undecyl, Dodecyl, Tridecyl, Tetradecyl, Pentadecyl, Hexadecyl, Heptadecyl, Octadecyl, Nonadecyl, Eicosadecyl und Kombinationen davon, vorzugsweise Dodecyl, Tridecyl, Tetradecyl, Pentadecyl, Hexadecyl und Kombinationen davon, besteht, ausgewählt wird.

Vorzugsweise sind die organischen Reste R², R³, und R⁴ jeweils unanhängig voneinander ein Alkylrest, der aus der Gruppe, die aus Methyl, Ethyl, Propyl, Butyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosadecyl und Kombinationen davon, vorzugsweise Methyl, Ethyl, Propyl, Butyl, Dodecyl, Tridecyl, Tetradecyl, Pentadecyl, Hexadecyl und Kombinationen davon, besteht, ausgewählt werden.

Beispielsweise ist ein geeignetes Alkylammoniumsalz der allgemeinen Formel (VI) ein Monoalkyltrimethylammoniumsalz eines Anions, das vorzugsweise aus der Gruppe, die aus Fluorid, Chlorid, Bromid, Iodid, Sulfat, Hydrogensulfat, Phosphat, Dihydrogenphosphat, Hydrogenphosphat, Tosylat, Mesylat, Formiat, Acetat, Propionat, Butanoat, Oxalat, Tartrat, Fumarat, Benzoat, Citrat und/oder Mischungen davon besteht, ausgewählt wird, wobei der organische Rest R¹ ein Alkylrest, der aus der Gruppe, die aus Octyl, Nonyl, Decyl, Undecyl, Dodecyl, Tridecyl, Tetradecyl, Pentadecyl, Hexadecyl, Heptadecyl, Octadecyl, Nonadecyl, Eicosadecyl und Kombinationen davon, vorzugsweise Dodecyl, Tridecyl, Tetradecyl, Pentadecyl, Hexadecyl und Kombinationen davon, besteht, ausgewählt wird, ist und wobei die organischen Reste R², R³ und R⁴ jeweils Methyl bedeuten.

Beispielsweise ist ein geeignetes Alkylammoniumsalz der allgemeinen Formel (VI) ein Dialkyltrimethylammoniumsalz eines Anions, das vorzugsweise aus der Gruppe, die aus Fluorid, Chlorid, Bromid, Iodid, Sulfat, Hydrogensulfat, Phosphat, Dihydrogenphosphat, Hydrogenphosphat, Tosylat, Mesylat, Formiat, Acetat, Propionat, Butanoat, Oxalat, Tartrat, Fumarat, Benzoat, Citrat und/oder Mischungen davon besteht, ausgewählt wird, wobei die organischen Rest R¹ und R² jeweils unabhängig voneinander ein Alkylrest, der aus der Gruppe, die aus Octyl, Nonyl, Decyl, Undecyl, Dodecyl, Tridecyl, Tetradecyl, Pentadecyl, Hexadecyl, Heptadecyl, Octadecyl, Nonadecyl, Eicosadecyl und Kombinationen davon, vorzugsweise Dodecyl, Tridecyl, Tetradecyl, Pentadecyl, Hexadecyl und Kombinationen davon, besteht, ausgewählt wird, bedeuten und wobei die organischen Reste R³ und R⁴ jeweils Methyl bedeuten.

Geeignete Alkylammoniumsalz der allgemeinen Formel (VI) sind vorzugsweise Dodecyltrimethylammoniumbromid (DTAB) und/oder Didodecyldimethylammoniumbromid (DDAB).

Geignete Esterquats umfassen beispielsweise Triethanolamin-diesterquats, Diethanolmethylamin-diesterquats oder Mischungen davon.

Geignete Esterquats können beispielsweise ausgehend von Triethanolamin oder Diethanolmethylamin hergestellt werden, indem man beispielsweise Diethanolmethylamin mit ein oder zwei Molekülen einer Fettsäuren oder im Falle des Triethanolamin mit ein, zwei oder drei Molekülen einer Fettsäure, vorzugsweise mit zwei Molekülen einer Fettsäure, verestert und anschließend mit Methylchlorid, Methylbromid oder mit Dimethylsulfat quaterniert. Als Fettsäuren setzt man zur Veresterung Fettsäuren mit 8 bis 24 Kohlenstoffatomen ein, die gesättigt oder ungesättigt sein können.

Vorzugsweise besitzen geeignete amphotere Tenside sowohl eine negativ als auch eine positiv geladene funktionelle Gruppe. Geeignete amphotere Tenside sind beispielsweise Alkylbetaine von Alkylresten mit 8-20 C-Atomen, Alkylsulfobetaine von Alkylresten mit 8 - 20 C-Atomen, Lecithine oder Kombinationen davon.

Geeignete amphotere Tenside sind beispielsweise CHAPS (3-[(3-Cholamidopropyl)dimethylammonio]-1-propansulfonat), CHAPSO (3-[(3-Cholamidopropyl)dimethylammonio]-2-hydroxy-1-propansulfonat), Cocamidopropylhydroxysultain, 1,2 Di-n-Octanoyl-sn-glycero-3-phosphocholin, 1,2-Di-O-hexadecyl-sn-glycero-3-phosphocholin oder Cocamidopropylbetain.

Eine erfindungsgemäße Dispersion umfasst vorzugsweise weiterhin wenigstens ein Alkanol mit 2 bis 12 Kohlenstoffatomen, und wenigstens 1 OH-Gruppe, vorzugsweise mit 1 bis 6 OH-Gruppen.

Vorzugsweise umfasst eine erfindungsgemäße Dispersion das wenigstens eine Alkanol in einem Anteil aus einem Bereich von 0 Gew.-% bis 50 Gew.-%, vorzugsweise aus einem Bereich von 0,1 Gew.-% bis 40 Gew.-%, weiter bevorzugt aus einem Bereich von 0,5 Gew.-% bis 35 Gew.-%, weiter bevorzugt aus einem Bereich von 1 Gew.-% bis 30 Gew.-%, weiter bevorzugt aus einem Bereich von 1,5 Gew.-% bis 25 Gew.-%, weiter bevorzugt aus einem Bereich von 5 Gew.-% bis 20 Gew.-%, weiter bevorzugt aus einem Bereich von 7 Gew.-% bis 19 Gew.-%, weiter bevorzugt aus einem Bereich von 10 Gew.-% bis 17 Gew.-%, jeweils bezogen auf das Gesamtgewicht der Dispersion.

Vorzugsweise wird das wenigstens eine Alkanol mit 2 bis 12 Kohlenstoffatomen bei Verwendung wenigstens eines anionischen Tensids, kationischen Tensids oder amphoteren Tensids als Cotensid verwendet.

Geeignete Alkanole sind verzweigte oder unverzweigte, vorzugsweise unverzweigte, Alkanole mit 2 bis 12 Kohlenstoffatomen und wenigstens 1 OH-Gruppe, vorzugsweise 1 bis 6 OH-Gruppen, vorzugsweise 1 bis 3 OH-Gruppen, oder Mischungen davon.

Vorzugsweise sind geeignete Alkanole verzweigt oder unverzweigt und weisen 2 bis 12 Kohlenstoffatome, weiter bevorzugt 4 bis 10 Kohlenstoffatome, auf.

Geeignet Alkanole mit 1 OH-Gruppe werden vorzugsweise aus der Gruppe, die aus Ethanol, 1-Propanol, 2-Propanol, 1-Buthanol, 2-Methyl-2-propanol, 1-Pentanol, 3-Methyl-1-butanol, 1-Hexanol, 1-Heptanol, 1-Octanol, 1-Nonanol, 1-Decanol, 1-Undecanol, 1-Dodecanol, und Mischungen davon besteht, ausgewählt.

Geeignet unverzweigte Alkanole mit 2 oder mehr OH-Gruppe, vorzugsweise 2 oder 3 OH-Gruppen, werden vorzugsweise aus der Gruppe, die aus Propan-1,2-diol (Propylenglycol) Propan-1,3-diol, Butan-1,2-diol, Butan-1,3-diol, Butan-1,4-diol, Butan-2,3-diol, Pentan-1,5-diol, Octan-1,8-diol, Propan-1,2,3-triol (Glycerin) oder Mischungen davon besteht, ausgewählt.

Vorzugsweise beträgt das Massenverhältnis von Tensiden zu Alkanol 4:1 bis 1:4, vorzugsweise von 3:1 bis 1:3, vorzugsweise von 2:1 bis 1:2, weiter bevorzugt 1:1.

Bei der erfindungsgemäßen Dispersion ist vorzugsweise ein Bestandteil der erfindungsgemäßen Dispersion fein verteilt (dispergierte Phase) in einem anderen kontinuierlichen Bestandteil der erfindungsgemäßen Dispersion (Dispersionsmedium, kohärente Phase).

Eine erfindungsgemäße Dispersion ist eine bei einer Temperatur aus einem Bereich von 2 bis 50°C und einem Druck aus einem Bereich von 800 bis 1200 mbar thermodynamisch stabile Dispersion, die wenigstens eine flüssige Phase umfasst und sich vorzugsweise kaum, weiter bevorzugt nicht, entmischt.

Eine erfindungsgemäße Dispersion umfasst oder ist eine Mikroemulsion, ein Gel, vorzugsweise ein Lyogel, oder eine Mischung davon, vorzugsweise eine Mikroemulsion und/oder ein Lyogel.

Die Erfinder haben festgestellt, dass sich eine erfindungsgemäße Dispersion, die eine Mikroemulsion, ein Gel, vorzugsweise ein Lyogel, oder eine Mischung davon umfasst oder ist, bei einer Temperatur aus einem Bereich von 2 bis 50°C und einem Druck aus einem Bereich von 800 bis 1200 mbar innerhalb eines Zeitraums von vorzugsweise 1 bis 5 Jahren kaum, weiter bevorzugt nicht, entmischt.

Bei einer alternativen Ausführungsform umfasst oder ist die erfindungsgemäße Dispersion bei einem Druck aus einem Bereich von 800 bis 1200 mbar und einer Temperatur aus einem Bereich von 2 bis 50°C eine Mikroemulsion.

Vorzugsweise ist bei einer Mikroemulsion die dispergierte Phase eine flüssige Phase, die in einer anderen flüssigen Phase (Dispersionsmedium) verteilt ist, wobei der wenigstens eine Photosensibilisator vorzugsweise in der dispergierten Phase, dem Dispersionsmedium, oder in beiden Phasen gelöst ist.

Eine erfindungsgemäße Mikroemulsion, umfasst vorzugsweise weiterhin wenigstens eine flüssige, unpolare Phase. Vorzugsweise liegt die wenigstens eine flüssige, unpolare Phase bei einer Temperatur aus einem Bereich von 0°C bis 100°C und einem Druck aus einem Bereich von 800 bis 1200 mbar im flüssigen Aggregatzustand vor.

Vorzugsweise umfasst die wenigstens eine flüssige, unpolare Phase wenigstens ein unpolares Lösungsmittel, vorzugsweise ein aprotisch-unpolares Lösungsmittel.

Vorzugsweise umfasst eine erfindungsgemäße Mikroemulsion das wenigstens eine unpolare Lösungsmittel in einem Anteil von wenigstens 0,1 Gew.-%, vorzugsweise von wenigstens 0,5 Gew.-%, weiter bevorzugt von wenigstens 1 Gew.-%, weiter bevorzugt von wenigstens 4 Gew.-%, weiter bevorzugt von wenigstens 10 Gew.-%, weiter bevorzugt von wenigstens 35 Gew.-%, weiter bevorzugt von wenigstens 50 Gew.-%, weiter bevorzugt von wenigstens 51 Gew.-%, jeweils bezogen auf das Gesamtgewicht der Mikroemulsion.

Vorzugsweise umfasst eine erfindungsgemäße Mikroemulsion das wenigstens eine unpolare Lösungsmittel in einem Anteil von aus einem Bereich von 0,1 Gew.-% bis 99,8 Gew.-%, vorzugsweise aus einem Bereich von 0,5 Gew.-% bis 99 Gew.-%, weiter bevorzugt aus einem Bereich von 1 Gew.-% bis 96 Gew.-%, weiter bevorzugt aus einem Bereich von 1,5 Gew.-% bis 90 Gew.-%, weiter bevorzugt aus einem Bereich von 3 Gew.-% bis 80 Gew.-%, weiter bevorzugt aus einem Bereich von 5 Gew.-% bis 75 Gew.-%, weiter bevorzugt aus einem Bereich von 10 Gew.-% bis 60 Gew.-%, weiter bevorzugt aus einem Bereich von 12 Gew.-% bis 49 Gew.-%, jeweils bezogen auf das Gesamtgewicht der Mikroemulsion.

Vorzugsweise wird das wenigstens eine unpolare Lösungsmittel aus der Gruppe, die aus Alkanen mit 6 bis 30 Kohlenstoffatomen, Monocarbonsäureester mit 4 bis 20 Kohlenstoffatomen, Polycarbonsäureester mit 6 bis 20 Kohlenstoffatomen und Mischungen davon besteht, ausgewählt.

Vorzugsweise weisen vorgenannte Alkane, Monocarbonsäureester und Polycarbonsäureester eine Löslichkeit in dem wenigstens einen polaren Lösungsmittel, vorzugsweise Wasser, bei einer Temperatur aus einem Bereich von 2 bis 50°C und einem Druck aus einem Bereich von 800 bis 1200 mbar von weniger als 1 g pro L polaren Lösungsmittel, vorzugsweise Wasser, auf. Weiter bevorzugt sind vorgenannte Alkane, Monocarbonsäureester und Polycarbonsäureester in dem polaren Lösungsmittel, vorzugsweise Wasser, bei einer Temperatur aus einem Bereich von 10 bis 25°C und einem Druck aus einem Bereich von 800 bis 1200 mbar nicht löslich.

Geeignete Alkane, Monocarbonsäureester und Polycarbonsäureester weisen vorzugsweise einen Siedepunkt (Sdp) von mehr als 80°C, vorzugsweise von mehr als 100°C auf. Vorzugsweise weisen Alkane, Monocarbonsäureester und Polycarbonsäureester einen Schmelzpunkt (Smp) von unterhalb 20°C, vorzugsweise von unterhalb 10°C, weiter bevorzugt von unterhalb 0°C, auf.

Geeignete Alkane sind vorzugsweise acyclische Alkane, die gradkettig oder verzweigt sein können, mit 5 bis 30 Kohlenstoffatomen, vorzugsweise mit 6 bis 25 Kohlenstoffatomen, weiter bevorzugt 8 bis 20 Kohlenstoffatomen, cyclische Alkane mit 5 bis 13 Kohlenstoffatomen, weiter bevorzugt 6 bis 12 Kohlenstoffatomen, oder Mischungen davon.

Geeignete Alkane können unsubstituiert oder mit Fluoratomen substituiert sein. Vorzugsweise sind geeignete Fluor-substituierte Alkane Perfluoralkane mit 5 bis 20 Kohlenstoffatomen, beispielsweise Perfluorheptan, Perfluoroctan, Perfluornonan, Perfluordecan, Perfluordecalin oder Mischungen davon.

Geeignete cyclische Alkane sind vorzugsweise Cyclohexan, Cycloheptan, Cyclooctan, Cyclononan, Cyclodecan, Cycloundecan oder Mischungen davon.

Geeignete cyclische Alkane können weiterhin mit acyclischen Alkylresten mit 1 bis 6 Kohlenstoffatomen, beispielsweise Methyl, Ethyl, n-Propyl, i-Propyl, n-Butyl, sec.-Butyl, tert.-Butyl, n-Pentyl oder Kombinationen davon, substituiert sein und werden beispielsweise aus der Gruppe, die aus Ethylcyclopentan, Propylcyclopentan, n-Butylcyclopentan, sec-Butylcyclopentan, tert-Butylcyclopentan, n-Pentylcyclopentan, Methylcyclohexan, Ethylcyclohexan, Propylcyclohexan, n-Butylcyclohexan, sec-Butylcyclohexan, tert-Butylcyclohexan, n-Pentylcyclohexan, und Mischungen davon besteht, ausgewählt.

Geeignete acyclische Alkane sind weiter bevorzugt Mischungen flüssiger acyclischer Alkane, die einen Schmelzpunkt (Smp) von nicht mehr als 20°C aufweisen.

Mischungen geeigneter Alkane sind vorzugsweise Parafinöle, weiter bevorzugt Weißöle. Geeignete Weißöle sind beispielsweise medizinische Weißöle.

Geeignete flüssige Paraffine sind beispielsweise im CAS-Verzeichnis unter CAS-8012-95-1 bzw. im EINECS-Verzeichnis unter EG 232-384-2 aufgeführt. Vorzugsweise beträgt ihre Dichte 0,81-0,89 g/cm³. Weiter bevorzugt liegt die Siedetemperatur geeigneter flüssiger Paraffine bei über 250 °C.

Geeignete Monocarbonsäureester sind vorzugsweise Ester von Alkanolen mit vorzugsweise 1 bis 10 Kohlenstoffatomen und Alkanmonocarbonsäuren mit vorzugsweise 2 bis 16 Kohlenstoffatomen, wobei der Monocarbonsäureester vorzugsweise 4 bis 20 Kohlenstoffatome aufweist.

Vorzugsweise weisen vorgenannte Polycarbonsäureester mit 6 bis 20 Kohlenstoffatomen 2 bis 4 Carboxy-Gruppen auf, die vorzugsweise vollständig verestert sind.

Geeignete Polycarbonsäureester sind vorzugsweise Diester von Alkandicarbonsäuren mit 4 bis 8 Kohlenstoffatomen, und Alkanolen mit 1 bis 12 Kohlenstoffatomen. Vorzugsweise können Alkandicarbonsäuren mit OH-Gruppen substituiert sein.

Geeignete Polycarbonsäureester sind beispielsweise Dimethylsuccinat, Diethylsuccinat, Dimethylsebacat, Diethylsebacat, Diethylhexyladipat, Diisononyladipat, Dimethyltartrat, Diethyltartrat, Diisopropyltartrat oder Mischungen davon.

Chiralitätszentren können, wenn nicht anders angegeben, in der R- oder in der S-Konfiguration vorliegen. Die Erfindung betrifft sowohl die Verwendung optisch reiner Verbindungen als auch Stereoisomerengemische, wie Enantiomerengemische und Diasteromerengemische, in jedem Verhältnis.

Beispielsweise kann Dieethyltartrat als (2S,3S)-Weinsäurediethylester, (2R,3R)-Weinsäurediethylester, (2R,3S)-Weinsäurediethylester oder als Mischung davon vorliegen.

Vorzugsweise ist eine Mikroemulsion eine Emulsion, die bei einer Temperatur aus einem Bereich von 2 bis 50°C und einem Druck aus einem Bereich von 800 bis 1200 mbar thermodynamisch stabil ist und deren dispergierte Phase derart kleine Domänen ("Tröpfchen") bildet, dass sichtbares Licht an ihnen nicht gestreut wird. Vorzugsweise ist eine erfindungsgemäße Mikroemulsion transparent.

Vorzugsweise umfasst eine erfindungsgemäße Mikroemulsion, die vorzugsweise eine Öl-in-Wasser (O/W) Mikroemulsion, eine Wasser-in-Öl (W/O) Mikroemulsion oder eine bikontinuierliche Mikroemulsion, vorzugsweise eine Öl-in-Wasser (O/W) Mikroemulsion oder eine Wasser-in-Öl (W/O) Mikroemulsion, ist,:
(a) wenigstens einen Photosensibilisator, der aus den oben genannten Phenalenonen, den oben genannten Flavinen und Mischungen davon, weiter bevorzugt aus den Verbindungen mit den Formeln (2) bis (28), (32) bis (49), (51) bis (64), und Mischungen davon, besteht, ausgewählt wird,
(b) wenigstens ein polares Lösungsmittel, vorzugsweise Wasser,
(c) wenigstens ein Tensid, das aus der Gruppe, die aus den oben genannten nichtionischen Tensiden, den oben genannten anionischen Tensiden, den oben genannten kationischen Tensiden, den oben genannten amphoteren Tensiden und Mischungen davon, vorzugsweise aus den oben genannten nichtionischen Tensiden, den oben genannten anionischen Tensiden und Mischungen davon, besteht, ausgewählt wird, und
(d) wenigstens ein unpolares Lösungsmittel, das weiter bevorzugt aus der Gruppe, die aus den oben genannten acyclischen Alkanen mit 5 bis 30 Kohlenstoffatomen, den oben genannten cyclischen Alkanen mit 5 bis 13 Kohlenstoffatomen, den oben genannten Perfluoralkanen mit 5 bis 20 Kohlenstoffatomen, den oben genannten Monocarbonsäureester mit 4 bis 20 Kohlenstoffatomen, den oben genannten Polycarbonsäureester mit 6 bis 20 Kohlenstoffatomen und Mischungen davon besteht, ausgewählt wird.

Vorzugsweise umfasst eine erfindungsgemäße Mikroemulsion weiterhin:
(e) wenigstens ein Alkanol, das aus der Gruppe, die aus den oben genannten Alkanolen mit 2 bis 12 Kohlenstoffatomen und vorzugsweise mit 1 bis 6 OH-Gruppen und Mischungen davon besteht, ausgewählt wird.

Vorzugsweise wird das wenigstens eine Tensid aus der Gruppe, die aus den oben genannten anionischen Tensiden und Mischungen davon besteht, ausgewählt und die erfindungsgemäße Mikroemulsion umfasst weiterhin wenigstens ein Alkanol, das aus der Gruppe, die aus den oben genannten Alkanolen mit 2 bis 12 Kohlenstoffatomen und vorzugsweise mit 1 bis 6 OH-Gruppen und Mischungen davon besteht, ausgewählt wird.

Vorzugsweise kann eine erfindungsgemäße Mikroemulsion eine Öl-in-Wasser (O/W) Mikroemulsion, eine Wasser-in-Öl (W/O) Mikroemulsion oder eine bikontinuierliche Mikroemulsion, vorzugsweise eine Öl-in-Wasser (O/W) Mikroemulsion oder eine Wasser-in-Öl (W/O) Mikroemulsion, umfassen oder daraus bestehen.

Eine bikontinuierliche Mikroemulsion umfasst vorzugsweise zwei Domänen, eine hydrophobe und eine hydrophile Domäne in Form von ausgedehnten nebeneinander liegenden und ineinander verschlungenen Domänen, an deren Grenzfläche stabilisierende grenzflächenaktive Tenside in einer monomolekularen Schicht angereichert sind.

Die erfindungsgemäße Mikroemulsion kann bei einer alternativen Ausführungsform eine Öl-in-Wasser (O/W) Mikroemulsion umfassen oder daraus bestehen, wobei die dispergierte Phase wenigstens eine flüssige, unpolare Phase umfasst, die weiter bevorzugt wenigstens ein unpolares Lösungsmittel umfasst, das aus der Gruppe, die aus den oben genannten Alkanen mit 6 bis 30 Kohlenstoffatomen, den oben genannten Monocarbonsäureester mit 4 bis 20 Kohlenstoffatomen, den oben genannten Polycarbonsäureester mit 6 bis 20 Kohlenstoffatomen und Mischungen davon besteht, ausgewählt wird. Vorzugsweise umfasst das Dispersionsmedium der Öl-in-Wasser (O/W) Mikroemulsion wenigstens ein polares Lösungsmittel, vorzugsweise Wasser.

Vorzugsweise umfasst eine erfindungsgemäße Öl-in-Wasser (O/W) Mikroemulsion das wenigstens eine unpolare Lösungsmittel in einem Anteil aus einem Bereich vom 0,1 Gew.-% bis 49,9 Gew.-%, vorzugsweise aus einem Bereich von 0,5 Gew.-% bis 48 Gew.-%, weiter bevorzugt aus einem Bereich von 1 Gew.-% bis 45 Gew.%, weiter bevorzugt aus einem Bereich von 3 Gew.-% bis 40 Gew.%, weiter bevorzugt aus einem Bereich von 5 Gew.-% bis 35 Gew.%, weiter bevorzugt aus einem Bereich von 7 Gew.-% bis 30 Gew.%, jeweils bezogen auf das Gesamtgewicht der Mikroemulsion.

Vorzugsweise umfasst eine erfindungsgemäße Öl-in-Wasser (O/W) Mikroemulsion weiterhin das wenigstens eine polare Lösungsmittel, vorzugsweise Wasser, in einem Anteil aus einem Bereich vom 50 Gew.-% bis 99,8 Gew.-%, vorzugsweise aus einem Bereich von 51 Gew.-% bis 99 Gew.-%, weiter bevorzugt aus einem Bereich von 52 Gew.-% bis 96 Gew.%, weiter bevorzugt aus einem Bereich von 53 Gew.-% bis 90 Gew.%, weiter bevorzugt aus einem Bereich von 54 Gew.-% bis 85 Gew.%, jeweils bezogen auf das Gesamtgewicht der Mikroemulsion.

Vorzugsweise umfasst eine erfindungsgemäße Öl-in-Wasser (O/W) Mikroemulsion weiterhin das wenigstens eine Tensid in einem Anteil aus einem Bereich von 0,1 Gew.-% bis 45 Gew.-%, vorzugsweise aus einem Bereich von 0,5 Gew.-% bis 40 Gew.-%, weiter bevorzugt aus einem Bereich von 1 Gew.-% bis 35 Gew.%, weiter bevorzugt aus einem Bereich von 3 Gew.-% bis 30 Gew.%, weiter bevorzugt aus einem Bereich von 5 Gew.-% bis 27 Gew.%, weiter bevorzugt aus einem Bereich von 7 Gew.-% bis 25 Gew.%, weiter bevorzugt aus einem Bereich von 10 Gew.-% bis 20 Gew.%, jeweils bezogen auf das Gesamtgewicht der Mikroemulsion.

Vorzugsweise umfasst eine erfindungsgemäße Öl-in-Wasser (O/W) Mikroemulsion weiterhin das wenigstens ein Alkanol in einem Anteil aus einem Bereich von 0 Gew.-% bis 50 Gew.-%, vorzugsweise aus einem Bereich von 0,1 Gew.-% bis 40 Gew.%, weiter bevorzugt aus einem Bereich von 0,5 Gew.-% bis 35 Gew.%, weiter bevorzugt aus einem Bereich von 1 Gew.-% bis 30 Gew.%, weiter bevorzugt aus einem Bereich von 1,5 Gew.-% bis 25 Gew.%, weiter bevorzugt aus einem Bereich von 5 Gew.-% bis 20 Gew.%, weiter bevorzugt aus einem Bereich von 7 Gew.-% bis 19 Gew.%, weiter bevorzugt aus einem Bereich von 10 Gew.-% bis 17 Gew.%, jeweils bezogen auf das Gesamtgewicht der Mikroemulsion.

Die erfindungsgemäße Mikroemulsion kann bei einer weiteren alternativen Ausführungsform eine Wasser-in-Öl (W/O) Mikroemulsion umfassen oder daraus bestehen, wobei die dispergierte Phase wenigstens ein polares Lösungsmittel, vorzugsweise Wasser, umfasst. Vorzugsweise umfasst das Dispersionsmedium der Wasser-in-Öl (W/O) Mikroemulsion wenigstens eine flüssige, unpolare Phase, die weiter bevorzugt wenigstens ein unpolares Lösungsmittel umfasst, das aus der Gruppe, die aus den oben genannten acyclischen Alkanen mit 5 bis 30 Kohlenstoffatomen, den oben genannten cyclischen Alkanen mit 5 bis 13 Kohlenstoffatomen, den oben genannten Perfluoralkanen mit 5 bis 20 Kohlenstoffatomen, den oben genannten Monocarbonsäureester mit 4 bis 20 Kohlenstoffatomen, den oben genannten Polycarbonsäureester mit 6 bis 20 Kohlenstoffatomen und Mischungen davon besteht, ausgewählt wird.

Vorzugsweise umfasst eine erfindungsgemäße Wasser-in-Öl (W/O) Mikroemulsion das wenigstens eine polare Lösungsmittel, vorzugsweise Wasser, in einem Anteil aus einem Bereich vom 0,1 Gew.-% bis 49,9 Gew.-%, vorzugsweise aus einem Bereich von 0,5 Gew.-% bis 48 Gew.-%, weiter bevorzugt aus einem Bereich von 1 Gew.-% bis 45 Gew.%, weiter bevorzugt aus einem Bereich von 3 Gew.-% bis 40 Gew.%, weiter bevorzugt aus einem Bereich von 5 Gew.-% bis 35 Gew.%, weiter bevorzugt aus einem Bereich von 7 Gew.-% bis 30 Gew.%, jeweils bezogen auf das Gesamtgewicht der Mikroemulsion.

Vorzugsweise umfasst eine erfindungsgemäße Wasser-in-Öl (W/O) Mikroemulsion weiterhin das wenigstens eine unpolare Lösungsmittel in einem Anteil aus einem Bereich vom 50 Gew.-% bis 99,8 Gew.-%, vorzugsweise aus einem Bereich von 51 Gew.-% bis 99 Gew.-%, weiter bevorzugt aus einem Bereich von 52 Gew.-% bis 96 Gew.%, weiter bevorzugt aus einem Bereich von 55 Gew.-% bis 90 Gew.%, weiter bevorzugt aus einem Bereich von 60 Gew.-% bis 80 Gew.%, jeweils bezogen auf das Gesamtgewicht der Mikroemulsion.

Vorzugsweise umfasst eine erfindungsgemäße Wasser-in-Öl (W/O) Mikroemulsion weiterhin das wenigstens eine Tensid und das wenigstens eine Alkanol in den oben genannten Gewichtsanteilen, jeweils bezogen auf das Gesamtgewicht der Mikroemulsion.

Vorzugsweise umfasst eine erfindungsgemäße Wasser-in-Öl (W/O) Mikroemulsion oder eine erfindungsgemäße Öl-in-Wasser (O/W) Mikroemulsion weiterhin wenigstens ein in dem wenigstens einen polaren Lösungsmittel, vorzugsweise Wasser, lösliches Salz eines Metalls, das aus der Gruppe, die aus Metallen der 1. bis 3 Hauptgruppe des Periodensystems der Elemente, vorzugsweise Alkalimetallen oder Erdalkalimetallen, besteht, ausgewählt wird, und wenigstens eines Anions, das aus der Gruppe, die aus Fluorid, Chlorid, Bromid, Iodid, Sulfat, Hydrogensulfat, Phosphat, Dihydrogenphosphat, Hydrogenphosphat, Tosylat, Mesylat, Formiat, Acetat, Propionat, Butanoat, Oxalat, Tartrat, Fumarat, Benzoat, Citrat und/oder Mischungen davon, weiter bevorzugt Chlorid, Sulfat, Hydrogensulfat, Formiat, Acetat, Benzoat, Citrat und/oder Mischungen davon, besteht, ausgewählt wird.

Vorzugsweise umfasst eine erfindungsgemäße Wasser-in-Öl (W/O) Mikroemulsion oder eine erfindungsgemäße Öl-in-Wasser (O/W) Mikroemulsion das wenigstens eine lösliche Salz in einem Anteil aus einem Bereich vom 0 Gew.-% bis 20 Gew.-%, vorzugsweise aus einem Bereich von 0,5 Gew.-% bis 15 Gew.-%, weiter bevorzugt aus einem Bereich von 0,7 Gew.-% bis 10 Gew.%, weiter bevorzugt aus einem Bereich von 1 Gew.-% bis 7 Gew.%, weiter bevorzugt aus einem Bereich von 1,5 Gew.-% bis 5 Gew.%, jeweils bezogen auf das Gesamtgewicht der Mikroemulsion.

Vorzugsweise ist die erfindungsgemäße Mikroemulsion eine, weiter bevorzugt bei einer Temperatur aus einem Bereich von 2 bis 50°C und einem Druck aus einem Bereich von 800 bis 1200 mbar thermodynamisch stabile, Monophase.

Weiter bevorzugt enthält die Mikroemulsion Tröpfchen mit einer Tröpfchengröße von kleiner als 350 nm, vorzugsweise von kleiner als 100 nm, weiter bevorzugt aus einem Bereich von einschließlich 1 nm bis einschließlich 95 nm, weiter bevorzugt von einschließlich 5 nm bis einschließlich 50 nm.

Die Erfinder haben überraschend festgestellt, dass die Bereitstellung wenigstens eines Photosensibilisators in einer Mikroemulsion, wobei der wenigstens eine Photosensibilisator vorzugsweise in der Mikroemulsion gelöst ist, die Anwendungseigenschaften des Photosensibilisators verbessert.

Beispielsweise kann der wenigstens eine Photosensibilisator in einem Konzentrat bereitgestellt werden, dass eine höhere Konzentration des Photosensibilisators enthält als beispielsweise für eine Anwendungslösung erforderlich ist.

Vorzugsweise liegt ein Konzentrat ebenfalls in Form einer Mikroemulsion vor. Die Erfinder haben überraschend festgestellt, dass eine erfindungsgemäße Mikroemulsion mit einer mehrfachen Menge Wasser, vorzugsweise 4-fachen bis 16 fachen Menge Wasser, jeweils bezogen auf das Volumen des zu verdünnenden Konzentrats, verdünnt werden kann, ohne dass die Benetzungsfähigkeit der erhaltenen Verdünnung sich im Vergleich zu der Benetzungsfähigkeit des Konzentrats signifikant verschlechtert.

Die einer alternativen Ausführungsform der erfindungsgemäßen Dispersion umfasst oder ist die Dispersion bei einem Druck aus einem Bereich von 800 bis 1200 mbar und einer Temperatur aus einem Bereich von 2 bis 50°C ein Gel, vorzugsweise ein Lyogel.

Vorzugsweise ist bei einem Gel, vorzugsweise Lyogel, die dispergierte Phase eine feste Komponente, die in einer flüssigen Phase (Dispersionsmedium) verteilt ist. Vorzugsweise ist der wenigstens eine Photosensibilisator in der flüssigen Phase gelöst.

Vorzugsweise bildet die feste Komponente dabei ein schwammartiges, dreidimensionales Netzwerk, dessen Poren durch eine Flüssigkeit (Lyogel) ausgefüllt sind. Die flüssige Komponente ist dadurch in der festen Komponente vorzugsweise immobilisiert. Beide Komponenten durchdringen sich vorzugsweise dabei vollständig (bikohärent).

Vorzugsweise umfasst ein erfindungsgemäßes Gel, vorzugsweise Lyogel:
(a) wenigstens einen Photosensibilisator, der aus der Gruppe, die aus den oben genannten Phenalenonen, den oben genannten Flavinen und Mischungen davon, weiter bevorzugt aus den Verbindungen mit den Formeln (2) bis (25), (32) bis (49), (51) bis (64), und Mischungen davon, besteht, ausgewählt wird,
(b) wenigstens ein polares Lösungsmittel, vorzugsweise Wasser,
(c) wenigstens ein Tensid, das aus der Gruppe, die aus den oben genannten nichtionischen Tensiden, den oben genannten anionischen Tensiden, den oben genannten kationischen Tensiden, den oben genannten amphoteren Tensiden und Mischungen davon, vorzugsweise aus den oben genannten nichtionischen Tensiden, den oben genannten anionischen Tensiden und Mischungen davon, besteht, ausgewählt wird, und
(d) wenigstens ein Gelbildner.

Geeignete Gelbildner werden vorzugsweise aus der Gruppe, die aus Polyacrylsäuren, Polyacrylamide, Alginaten, Celluloseether, und Mischungen besteht, ausgewählt.

Geeignete Celluloseether sind beispielsweise Carboxymethylcellulose (CMC), Methylcellulose (MC), Ethylcellulose (EC), Hydroxyethylcellulose (HEC), Hydroxyethylmethylcellulose (HEMC) oder Hydroxypropylmethylcellulose (HPMC), Hydroxyethyl-Methyl-Cellulosen, Hydroxypropyl-Methyl-Cellulosen, Ethylhydroxyethyl-Cellulosen, Carboxymethylhydroxyethyl-Cellulosen oder Mischungen davon.

Geeignete Carboxyyinylpolymere sind beispielsweise Polyacrylsäuren, Acrylat-Copolymere oder Mischungen davon.

Vorzugsweise umfasst ein erfindungsgemäßes Gel, vorzugsweise Lyogel, den wenigstens einen Gelbildner in einem Anteil aus einem Bereich vom 0,1 Gew.-% bis 49,9 Gew.-%, vorzugsweise aus einem Bereich von 0,5 Gew.-% bis 45 Gew.-%, weiter bevorzugt aus einem Bereich von 1 Gew.-% bis 41 Gew.%, weiter bevorzugt aus einem Bereich von 2 Gew.-% bis 37 Gew.%, weiter bevorzugt aus einem Bereich von 3 Gew.-% bis 25 Gew.%, weiter bevorzugt aus einem Bereich von 5 Gew.-% bis 15 Gew.%, jeweils bezogen auf das Gesamtgewicht des Gels, vorzugsweise Lyogels.

Vorzugsweise umfasst ein erfindungsgemäßes Gel, vorzugsweise Lyogel, weiterhin:
(e) wenigstens eine pH-regulierende Substanz., die vorzugsweise eine anorganische Säure, organische Säure, anorganische Base, organische Base, oder eine Mischung davon ist.

Vorzugsweise liegt der pH-Wert des Gels, vorzugsweise Lyogels, bei einer Temperatur aus einem Bereich von 2 bis 50°C und einem Druck aus einem Bereich von 800 bis 1200 mbar in einem Bereich von 4 bis 11, bevorzugt von 6 bis 10.

Geeignete anorganische Säuren sind beispielsweise Phosphorsäure, Schwefelsäure, Chlorwasserstoffsäure oder Mischungen davon.

Geeignete organische Säuren sind beispielsweise Essigsäure, Schwefelsäure, Toluensulfonsäure, Citronensäure, Barbitursäure, 4-(2-Hydroxyethyl)-1-piperazinethanesulfonsäure, 4-(2-Hydroxyethyl)-piperazin-1-propansulfonsäure, 2-(N-Morpholino)ethansulfonsäure oder Mischungen davon.

Geeignete anorganische Basen sind beispielsweise Phosphate, Hydrogenphosphate, Dihydrogenphosphate, Sulfate, Hydrogensulfate, Ammoniak, NaOH, KOH oder Mischungen davon.

Eine geeignete organische Base ist beispielsweise Tris(hydroxymethyl)-aminomethan, N-Methylmorpholin, Triethylamin, Pyridin oder Mischungen davon.

Vorzugsweise umfasst ein erfindungsgemäßes Gel, vorzugsweise Lyogel, weiterhin:
(f) wenigstens ein, in dem wenigstens einen polaren Lösungsmittel, vorzugsweise Wasser, lösliches Salz eines Metalls, das aus der Gruppe, die aus Metallen der 1. bis 3 Hauptgruppe des Periodensystems der Elemente, vorzugsweise Alkalimetallen oder Erdalkalimetallen, besteht, ausgewählt wird, und wenigstens eines Anions, das aus der Gruppe, die aus Fluorid, Chlorid, Bromid, Iodid, Sulfat, Hydrogensulfat, Phosphat, Dihydrogenphosphat, Hydrogenphosphat, Tosylat, Mesylat, Formiat, Acetat, Propionat, Butanoat, Oxalat, Tartrat, Fumarat, Benzoat, Citrat und/oder Mischungen davon, weiter bevorzugt Chlorid, Sulfat, Hydrogensulfat, Formiat, Acetat, Benzoat, Citrat und/oder Mischungen davon, besteht, ausgewählt wird.

Vorzugsweise umfasst ein erfindungsgemäßes Gel, vorzugsweise Lyogel, das wenigstens eine lösliche Salz in einem Anteil aus einem Bereich vom 0 Gew.-% bis 20 Gew.-%, vorzugsweise aus einem Bereich von 0,5 Gew.-% bis 15 Gew.-%, weiter bevorzugt aus einem Bereich von 0,7 Gew.-% bis 10 Gew.%, weiter bevorzugt aus einem Bereich von 1 Gew.-% bis 7 Gew.%, weiter bevorzugt aus einem Bereich von 1,5 Gew.-% bis 5 Gew.%, jeweils bezogen auf das Gesamtgewicht des erfindungsgemäßen Gels, vorzugsweise Lyogels.

Vorzugsweise weist das Gel, vorzugsweise Lyogel, eine dynamische Viskosität aus einem Bereich von 1000 Pas bis 5000 Pas auf.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele Erreger-bedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Einzellige oder mehrzellige Mikroorganismen können Auslöser von infektiösen Erkrankungen sein. Durch Applikation wenigstens eines Erreger-spezifischen Gegenmittels, beispielsweise Antibiotikum, Antimikotikum oder Virustatikum, kann die Anzahl der Erreger reduziert und/oder der Erreger inaktiviert werden. Die Applikation eines Erreger-spezifischen Gegenmittels kann systemisch und/oder topisch erfolgen.

Bei der systemischen Applikation wird das Erreger-spezifische Gegenmittel in das Blut- und/oder Lymphsystem des zu behandelnden Körpers übertragen und hierüber im gesamten Körper verteilt. Bei einer systemischen Aufnahme des Erreger-spezifischen Gegenmittels kann es zu einem Abbau des Gegenmittels und/oder zu Nebenwirkungen, beispielsweise durch eine biochemische Umwandlung (Metabolisierung) des Gegenmittels, kommen.

Bei der topischen Applikation des Erreger-spezifischen Gegenmittels erfolgt die Anwendung des Gegenmittels dort, wo es therapeutisch wirken soll, beispielsweise auf einer infizierten Hautpartie, während die gesunde Haut nicht belastet wird. Somit können systemische Nebenwirkungen weitgehend vermieden werden.

Oberflächliche Haut- oder Weichteilinfektionen müssen nicht notwendigerweise mit, einer systemischen Applikation eines Erreger-spezifischen Gegenmittels behandelt werden, da das Gegenmittel direkt auf die infizierte Hautpartien aufgetragen werden kann.

Die bisher bekannten Erreger-spezifischen Gegenmittel weisen sowohl bei systemischer als auch topischer Applikation teilweise starke Neben- und Wechselwirkungen auf. Darüber hinaus kann es auch bei topischer Applikation durch eine unzuverlässige Medikamenteneinnahme (Compliance) des Patienten, insbesondere bei Verwendung von Antibiotika, zur Resistenzbildung kommen.

Eine Alternative stellt hier die photodynamische Inaktivierung von Mikroorganismen dar, bei der Resistenzen gegenüber der photodynamischen Inaktivierung unbekannt sind. Unabhängig von der Art der zu bekämpfenden Mikroorganismen und der damit verbundenen infektiösen Erkrankungen wird die Anzahl der Erreger reduziert und/oder die Erreger werden abtötet. Beispielsweise können Mischungen aus verschieden Mikroorganismen, beispielsweise Pilze und Bakterien oder unterschiedliche Bakterienstämme, bekämpft werden.

Die Aufgabe der vorliegenden Erfindung wird ebenfalls gelöst durch die Bereitstellung einer Dispersion nach einem der Ansprüche 1 bis 14 zur Anwendung bei der photodynamischen Therapie zur Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden, wobei die Dispersion vorzugsweise bei der Behandlung und/oder Prophylaxe einer Erkrankung des Zahngewebes und/oder des Zahnhalteapparates verwendet wird.

Die Aufgabe der vorliegenden Erfindung wird ebenfalls durch die Bereitstellung eines nicht-medizinischen Verfahrens zur photodynamischen Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, gelöst, wobei das Verfahren folgende Schritte umfasst:
(A) in Kontakt bringen der Mikroorganismen mit wenigstens einer Dispersion nach einem der Ansprüche 1 bis 14, und
(B) Bestrahlen der Mikroorganismen und wenigstens eines, in der Dispersion enthaltenen Photosensibilisators mit elektromagnetischer Strahlung geeigneter Wellenlänge und Energiedichte.

Verzugsweise wird das erfindungsgemäße Verfahren zur Inaktivierung von Mikroorganismen bei der photodynamischen Entkeimung wenigstens einer Oberfläche eines Gegenstandes und/oder wenigstens einer Oberfläche eines Raumes durchgeführt.

Bei einer bevorzugten Ausführungsform des erfindungsgemäßen Verfahrens erfolgt die Bestrahlung der Mikroorganismen und des wenigstens einen Photosensibilisators mit elektromagnetischer Strahlung geeigneter Wellenlänge und Energiedichte in Gegenwart wenigstens einer Sauerstoff-abgebenden Verbindung, vorzugsweise Peroxid, und/oder wenigstens eines Sauerstoff-haltigen Gases, vorzugsweise Sauerstoff.

Die wenigstens eine Sauerstoff-abgebende Verbindung und/oder das wenigstens eine Sauerstoff-haltige Gas können vorzugsweise vor, oder während Schritt (B) des erfindungsgemäßen Verfahrens appliziert werden.

Durch eine zusätzliche Gabe von Sauerstoff in Form wenigstens einer Sauerstoff-haltigen Verbindung und/oder wenigstens eines Sauerstoff-haltigen Gases vor oder während der Bestrahlung der Mikroorganismen und des wenigstens einen Photosensibilisators mit elektromagnetischer Strahlung geeigneter Wellenlänge und Energiedichte wird die Ausbeute an entstandenen reaktiven Sauerstoffspezies (ROS), vorzugsweise Sauerstoffradikale und/oder Singulett-Sauerstoff erhöht.

Die Aufgabe der vorliegenden Erfindung wir ebenfalls gelöst durch die Verwendung wenigstens einer Dispersion nach einem der Ansprüche 1 bis 14 zur Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen.

Eine erfindungsgemäß zu verwendende Dispersion weist nach Bestrahlung mit elektromagnetischer Strahlung geeigneter Wellenlänge und Energiedichte eine hohe Ausbeute an Singulett-Sauerstoff auf.

Bei dem erfindungsgemäßen Verfahren und/oder der erfindungsgemäßen Verwendung liegt vorzugsweise die elektromagnetische Strahlung im sichtbaren Spektralbereich, ultravioletten und/oder infraroten Bereich. Weiter bevorzugt weist die elektromagnetische Strahlung eine Wellenlänge aus einem Bereich von 280 bis 1000 nm, weiter bevorzugt von 380 bis 1000 nm, auf.

Weiter bevorzugt weist die elektromagnetische Strahlung eine Energiedichte aus einem Bereich von 1 µW/cm² bis 1 kW/cm², weiter bevorzugt von 1 mW/cm² bis 100 W/cm², weiter bevorzugt von 2 mW/cm² bis 50 W/cm², weiter bevorzugt von 6 mW/cm² bis 30 W/cm², weiter bevorzugt von 7 mW/cm² bis 25 W/cm², auf.

Die Bestrahlungszeit kann in Abhängigkeit von der Art der Mikroorganismen und/oder der Schwere der Infektion variiert werden. Vorzugsweise liegt die Bestrahlungszeit in einem Bereich von 1 µs bis 1 h, weiter bevorzugt von 1 ms bis 1000 s.

Beispielsweise kann zur Bestrahlung eine in der WO 96/29943 A1, der EP 0 437 183 B1 oder der WO 2013/172977 A1 beschriebe Bestrahlungsvorrichtung verwendet werden.

Vorzugsweise umfasst die Bestrahlungsvorrichtung weiterhin eine Vorrichtung zur Abgabe der wenigstens einen Sauerstoff-haltigen Verbindung, vorzugsweise Peroxid, und/oder des wenigstens einen Sauerstoff-haltigen Gases, vorzugsweise von Sauerstoff
Vorzugsweise wird die elektromagnetische Strahlung durch eine Strahlungsquelle erzeugt, die aus der Gruppe, die aus künstlichen Strahlungsquellen, beispielsweise UV-Lampe, IR-Lampe, Leuchtstofflampen, Leuchtdioden, Laser oder chemisches Licht, besteht, ausgewählt wird.

Darüber hinaus haben die Erfinder überraschenderweise festgestellt, dass wenigstens ein, in der erfindungsgemäßen Dispersion enthaltener Photosensibilisator eine hohe Affinität zu Mikroorganismen aufweist.

Auf Grund der Affinität kann wenigstens ein, in der erfindungsgemäßen Dispersion enthaltener Photosensibilisator an Mikroorganismen effektiv binden und lokal ausreichend Singulett-Sauerstoff erzeugen, um die Mikroorganismen zu inaktivieren, vorzugsweise abzutöten.

Weiterhin wird durch die Bereitstellung des wenigstens einen Photosensibilisators in Form der erfindungsgemäßen Dispersion die Halbwertszeit des lokal gebildeten Singulett-Sauerstoffs nach Bestrahlung mit elektromagnetischer Strahlung geeigneter Wellenlänge und Energiedichte signifikant verlängert.

Nach Bestrahlung der erfindungsgemäßen Dispersion mit elektromagnetischer Strahlung geeigneter Wellenlänge und Energiedichte werden die Mikroorganismen durch die entstandenen reaktiven Sauerstoffspezies (ROS), vorzugsweise Sauerstoffradikale und/oder Singulett-Sauerstoff, inaktiviert, vorzugsweise abgetötet.

Vorzugsweise wird durch eine Verlängerung der Halbwertszeit des lokal gebildeten Singulett-Sauerstoffs nach Bestrahlung mit elektromagnetischer Strahlung geeigneter Wellenlänge und Energiedichte der Verlauf der Anaktivierung von Mikroorganismen oder der Dekolonisation beschleunigt.

Erfindungsgemäß wird unter dem Begriff "Dekolonisation" das Entfernen, vorzugsweise vollständige Entfernen, von Mikroorganismen verstanden.

Vorzugsweise können Körperoberflächen, beispielsweise Haut oder Schleimhaut, von Menschen und Tieren, vorzugsweise Säugetieren, behandelt werden. Bei dieser bevorzugten Ausführungsform wird wenigstens eine erfindungsgemäß zu verwendende Dispersion bei der Entkeimung und/oder Dekolonisierung von Haut- oder Weichteiloberflächen verwendet, wobei vorzugsweise die Hautintegrität erhalten bleibt.

Bei einer weiter bevorzugten Ausführungsform wird eine erfindungsgemäß zu verwendende Dispersion zur lokalen und/oder topischen, vorzugsweise nasalen, oralen, analen, vaginalen oder dermalen, Applikation eingesetzt.

Unter topischer Applikation wird auch die Anwendung am oder im Ohr, vorzugsweise Außenohr, verstanden. Das Außenohr umfasst den Ohrknorpel, die Ohrmuschel, das Ohrläppchen, den äußeren Gehörgang oder auch Ohrkanal und die Außenseite des Trommelfells.

Unter topischer Applikation wird auch die Anwendung an oder in der Nase und/oder den Nasennebenhöhlen, wie beispielsweise der Kieferhöhle, der Stirnhöhle und/oder Keilbeinhöhle, verstanden.

Unter topischer Applikation wird auch die Anwendung auf der Oberfläche des Auges, vorzugsweise der äußeren, apikalen Seite der Epithelschicht der Hornhaut, und/oder der äußeren Oberfläche der Anhangsorgane des Auges, vorzugsweise des Tränenapparates, der Bindehaut und/oder der Augenlider, verstanden.

Unter topischer Applikation wird auch die Anwendung auf die äußere, apikale Seite des Epithels von Hohlorganen, beispielsweise der Speiseröhre, den Magen-Darm-Trakt, der Gallenblase, der Gallengänge, des Kehlkopfs, der Luftröhre, der Bronchien, der Eileiter, der Gebärmutter, der Vagina, der Harnleiter, der Harnblase, oder der Harnröhre, verstanden.

Unter topischer Applikation wird auch die Anwendung an oder in Zähnen, beispielsweise in einem Wurzelkanal und/oder einer Wurzelkavität und/oder Zahnfissur, oder Zahnfleischtaschen und/oder Knochentaschen verstanden.

Bei einer weiteren bevorzugten Ausführungsform wird wenigstens eine erfindungsgemäß zu verwendende Dispersion zur Herstellung einer pharmazeutischen Zubereitung bei der Prophylaxe und/oder Behandlung einer infektiösen, vorzugsweise viralen, bakteriellen und/oder mykotischen, Hautkrankheit, die vorzugsweise ausgewählt wird aus der Gruppe, die aus Staphylococcal scalded skin syndrome, Impetigo, Hautabszess, Furunkel, Karbunkel, Phlegmone, Cellulitis, Akute Lymphadenitis, Pilonidalzyste, Pyodermie, Dermatitis purulenta, Dermatitis septica, Dermatitis suppurativa, Erythrasma, Erysipelas, Akne vulgaris oder Pilzinfektion besteht, verwendet.

Bei einer weiteren bevorzugten Ausführungsform wird wenigstens eine erfindungsgemäß zu verwendende Dispersion zur Herstellung einer pharmazeutischen Zubereitung bei der Wundheilung, beispielsweise bei Heilungsstörungen nach chirurgischen Interventionen, verwendet.

Vorzugsweise wird wenigstens eine erfindungsgemäß zu verwendende Dispersion bei der Entkeimung und/oder Reduktion der Keimzahl in infizierten Wunden verwendet.

Bei einer weiteren bevorzugten Ausführungsform wird wenigstens eine erfindungsgemäß zu verwendende Dispersion zur Herstellung einer pharmazeutischen Zubereitung bei der Prophylaxe und/oder Behandlung von infektiösen, vorzugsweise viralen, bakteriellen und/oder mykotischen, Erkrankungen des Ohrs, der oberen Luftwege, der Mundhöhle, des Rachens, des Kehlkopfes, der unteren Luftwege und/oder der Speiseröhre verwendet. Das Vorherrschen pathogener Mikroorganismen ist beispielsweise die Hauptursache für Infektionen in der Mundhöhle. Dabei tritt das Problem auf, dass die Mikroorganismen in äußerst komplex aufgebauten Biofilmen synergetisch organisiert sind. Diese Biofilme, beispielsweise Plaque oder Zahnbelag, bestehen aus mehreren, komplex aufgebauten Schichten und enthalten Eiweiße, Kohlenhydrate, Phosphate und Mikroorganismen. Zahnbelag entsteht besonders dort, wo Zahnflächen nicht durch natürliche oder künstliche Reinigung belagfrei gehalten werden können. Dieser Umstand macht es schwierig, einen Zugang zu den im Biofilm eingebundenen Mikroorganismen zu finden.

Konventionelle Therapien, wie beispielsweise Antibiotika und Spüllösungen oder mechanische Zahnreinigung, können nur begrenzt eingesetzt werden, da sie entweder die Bakterien nicht direkt beeinflussen, beispielsweise bei der Zahnreinigung, nur schwer dosiert und appliziert werden können, beispielsweise bei Antibiotika und Spüllösungen, oder eine generelle Anwendung aufgrund von negativen Begleiterscheinungen nicht zu rechtfertigen ist.

Beispielsweise werden in den Vereinigten Staaten jährlich 20 Millionen Wurzelkanäle behandelt, wobei etwa mehr als 2 Millionen endodontische Wiederbehandlungen durchgeführt werden, die sich durch eine verbesserte Entkeimung der Wurzelkanäle vermeiden ließen.

Vorzugsweise eignen sich das erfindungsgemäße Verfahren und die erfindungsgemäße Verwendung zu einer wirkungsvollen Eliminierung von Mikroorganismen im Wurzelkanalsystem eines humanen Zahnes, wobei die Wurzelkanäle und Dentinkanälchen verstanden werden.

Bei einer bevorzugten Ausführungsform wird wenigstens eine erfindungsgemäß zu verwendende Dispersion zur Herstellung einer pharmazeutischen Zubereitung bei der Behandlung und/oder Prophylaxe einer infektiösen, vorzugsweise viralen, bakteriellen und/oder mykotischen, Erkrankung des Zahngewebes, vorzugsweise Plaque, Karies oder Pulpitis, und/oder infektiösen, vorzugsweise viralen, bakteriellen und/oder mykotischen, Erkrankung des Zahnhalteapparates, vorzugsweise Gingivitis, Paradontitis, Endodontitis oder Periimplantitis, verwendet.

Bei einer weiteren bevorzugten Ausführungsform wird wenigstens eine erfindungsgemäß zu verwendende Dispersion bei der Reinigung von Zähnen, Zahnersatz und/oder
Zahnspangen verwendet oder bei der nasalen Dekolonisierung von Mikroorganismen verwendet.

Beispielsweise besitzen Methicillin-resistente Staphylococcus aureus (MRSA)-Stämme eine monatelange Persistenz bei nasaler Kolonisierung sowie eine hohe Umweltresistenz. Daher reduziert eine nasale Dekolonisierung, d.h. Entfernung der Mikroorganismen, in der Regel auch die Kolonisation an anderen Körperstellen.

Bei einer weiteren bevorzugten Ausführungsform wird wenigstens eine erfindungsgemäß zu verwendende Dispersion bei der Inaktivierung von Mikroorganismen in einer biologischen Flüssigkeit, vorzugsweise medizinischen Blutprodukt, verwendet.

Geeignete Vorrichtungen zur Bestrahlung einer biologischen Flüssigkeit sind dem Fachmann bekannt und werden beispielsweise in der WO 99/43790 A1, US 2009/0010806 A1 oder der WO 2010/141564 A2 beschrieben.

Geeignete biologische Flüssigkeiten umfassen beispielsweise Blut und Blutprodukte, einschließlich gefrorenem Frischplasma, Erythrozyten-Konzentrat, Thrombozyten-Konzentrat, Granulozyten-Konzentrat, thrombozytenreiches Plasma, Stammzellpräparate, Konzentrate von einzelnen Gerinnungsfaktoren, Humanalbumin, Immunglobuline, Fibrinkleber, Antithrombin, Protein C, Protein S, Fibrinolytika oder Kombinationen davon.

Bei einer bevorzugten Ausführungsform wird wenigstens eine erfindungsgemäß zu verwendende Dispersion zur photodynamischen Entkeimung von Oberflächen aller Art verwendet. Eine photodynamische Entkeimung von Oberflächen bewirkt eine photodynamische Inaktivierung von Mikroorganismen auf der behandelten Oberfläche.

Geeignete Oberflächen umfassen beispielsweise Oberflächen aus Kunststoff, Metall, Glas, Textilien, Holz, Stein oder Kombinationen davon.

Weiter bevorzugt wird wenigstens eine erfindungsgemäße Dispersion bei der photodynamische Entkeimung, Oberflächenreinigung und/oder -beschichtung, vorzugsweise von Medizinprodukten, elektronische Geräte, Hygieneartikeln, Lebensmittelverpackungen, Lebensmitteln, Möbeln, Baustoffen, oder Räumen, beispielsweise Böden, Wänden und/oder Fenstern, verwendet.

Weiter bevorzugt werden Gegenstände behandelt, die eine thermisch begrenzte Haltbarkeit aufweisen, beispielsweise Gegenstände aus thermoplastischen Kunststoffen, oder von Desinfektionsmitteln angegriffen werden.

Gegenstände, die eine thermisch begrenzte Haltbarkeit aufweisen, können beispielsweise nur unzureichend sterilisiert werden, da sie bei höheren Temperaturen ihre Form verlieren oder spröde werden.

Darüber hinaus kann es bei einer unsachgemäßen und/oder übermäßigen Anwendung von Desinfektionsmitteln zu Resistenzbildung durch Selektion robuster Mikroorganismen kommen, wenn beispielsweise die Wirkstoffkonzentration und Einwirkzeit und damit die keimreduzierende Wirkung zu gering ist.

Bei einer weiter bevorzugten Ausführungsform wird das erfindungsgemäße Verfahren zur Vermeidung einer bakteriellen Infektion verwendet, beispielsweise vor einer Implantation oder nach erfolgter Dekolonisierung beispielsweise zur Vorbeugung vor der Neubesiedlung mit krankheitserregenden Mikroorganismen, wie beispielsweise paradontalpathogene Mikroorganismen.

Zur Vermeidung von Infektionen durch Mikroorganismen kann das erfindungsgemäße Verfahren ebenfalls zur Dekolonisierung von Oberflächen verwendet werden.

Beispielsweise führt der Kontakt von immunsupprimierten Patienten mit kontaminierten Gegenständen häufig zur Ausbildung einer Infektion, da immunsupprimierte Patienten in der Regel anfällig sind gegenüber Infektionen, beispielsweise auch von geringen Keimzahlen. Insbesondere Oberflächen von Medizinprodukten, vorzugweise medizinischen Hilfsmitteln oder zahnmedizinischen Hilfsmitteln, weiter bevorzugt invasiven medizinischen Hilfsmitteln wie etwa Kathetern, Hohlsonden, Schläuchen oder Nabeln, müssen vor dem Eindringen in den menschlichen Körper desinfiziert werde.

Daher wird in einer weiteren bevorzugten Ausführungsform wenigstens eine erfindungsgemäße Dispersion zur Inaktivierung von Mikroorganismen auf Oberflächen von Medizinprodukten, vorzugsweise invasiven medizinischen Hilfsmitteln wie etwa Kontaktlinsen, chirurgische Instrumente, zahnmedizinische Bohrer, Mundspiegel, Küretten, zahnmedizinische Feilen, Kathetern, Hohlsonden, Schläuchen oder Nadeln, verwendet.

Vorzugsweise werden die Medizinprodukte aus Wundauflagen, Verbände, chirurgische Instrumenten, Kathetern, Hohlsonden, Schläuchen oder Nadeln ausgewählt.

Weiter bevorzugt werden unter Medizinprodukten auch zahnärztliche Abdrücke, Abdrucklöffel, Spangen, Aufbissschienen oder Zahnersatz, beispielsweise Prothesen, Kronen oder Implantate, sowie beispielsweise Hörgeräte oder Kontaktlinsen verstanden.

Vorzugsweise wird durch eine Behandlung der Oberfläche von Gegenständen aller Art mit wenigstens einer erfindungsgemäßen Dispersion auf der Oberfläche von Medizinprodukten und nachfolgender Bestrahlung mit elektromagnetische Strahlung geeigneter Wellenlänge und Energiedichte die Besiedelung von Mikroorganismen auf der behandelten Oberflächen reduziert, vorzugsweise verhindert.

Vorzugsweise erfolgt die Behandlung der Oberfläche durch Sprühen, Streichen, Spritzen, Sprayen, Tauchen oder Kombinationen davon.

Die Bestrahlung kann dabei direkt nach der Behandlung der Oberfläche mit wenigstens einer erfindungsgemäßen Dispersion erfolgen und/oder zu einem späteren Zeitpunkt, vor oder während der Verwendung des behandelten Gegenstandes, beispielsweise Medizinproduktes.

Bei einer weiteren bevorzugten Ausführungsform wird wenigstens eine erfindungsgemäße Dispersion zur Inaktivierung von Mikroorganismen auf Oberflächen von Lebensmittelverpackungen verwendet.

Geeignete Lebensmittelverpackungen schließen beispielsweise Behälter aus Glas, Metall, Kunststoff, Papier, Karton oder Kombinationen davon ein.

Geeignete Behälter können beispielsweise vor dem Befüllen mit einem Lebensmittel oder Getränk mit wenigstens einer erfindungsgemäßen Dispersion behandelt und nachfolgend mit einer geeigneten Strahlenquelle, die elektromagnetische Strahlung geeigneter Wellenlänge und Energiedichte erzeugt, bestrahlt werden. Anschließend kann das entsprechende Lebensmittel oder Getränk in den entkeimten Behälter gefüllt und der Behälter verschlossen werden.

Bei einer weiteren bevorzugten Ausführungsform wird wenigstens eine erfindungsgemäße Dispersion zur Inaktivierung von Mikroorganismen auf Oberflächen von Lebensmitteln verwendet.

Geeignete Lebensmittel umfassen beispielsweise Lebensmittel wie Fleisch, Fisch, Eier, Samen, Saaten, Nüssen, Beeren, Gewürzen, Obst oder Gemüse, die mit pathogenen Bakterien wie etwa Salmonellen, Clostridien, Escherichia coli - oder Camphylobacter - Spezies in Kontakt kommen können. Vorzugsweise können ebenfalls Bruteier photodynamisch entkeimt werden.

Als Magen-Darm-Infektionen wird eine Gruppe von Erkrankungen bezeichnet, die sich hauptsächlich durch Symptome im oberen Magen-Darm-Trakt wie Erbrechen, Durchfälle und Bauchschmerzen äußern. Magen-Darm-Infektionen werden durch Viren, Bakterien oder Parasiten verursacht. Die Erreger werden meist über kontaminiertes Wasser und/oder kontaminierte Lebensmittel aufgenommen.

Zu den bekanntesten Verursachern von Magen-Darm-Infektionen gehören beispielsweise Salmonellen, Campylobacter - Spezies oder Escherichia coli - Spezies wie z. B. enterohämorrhagische Escherichia coli (EHEC). Brechdurchfälle durch Lebensmittelvergiftungen werden vor allem durch Staphylokokken verursacht.

Am häufigsten gelangen Erreger von Magen-Darm-Infektionen, wie beispielsweise Salmonellen, über Lebensmittel in den Verdauungstrakt des Menschen. Die Erfinder haben festgestellt, dass durch das erfindungsgemäße Verfahren Mikroorganismen auf der Oberfläche von Lebensmitteln effizient beseitigen lassen.

Salmonellen sind beispielsweise Bakterien, die weltweit vorkommen. Eine Salmonellen-Erkrankung ist eine typische Lebensmittelinfektion, die Durchfall verursacht. Die Erreger vermehren sich im Magen-Darm-Trakt von Menschen und Tieren. Salmonellen können sich schnell auf ungekühlten Lebensmitteln vermehren. Die Bakterien gelangen unter Umständen auch durch schlechte Küchenhygiene ins Essen, zum Beispiel über verunreinigte Schneidebretter und/oder Messer.

Häufig mit Salmonellen belastete Lebensmittel sind beispielsweise rohe, bzw. nicht vollständig durchgegarte Eier und Eiprodukte, wie beispielsweise Mayonnaise, Cremes oder Salate auf Eierbasis oder roher Kuchenteig. Häufig mit Salmonellen belastete Lebensmittel sind ebenfalls Speiseeis, rohes Fleisch, zum Beispiel rohes Hack oder Tatar, Rohwurstsorten, zum Beispiel Mett oder Salami. Auch pflanzliche Lebensmittel können mit Salmonellen besiedelt sein.

Campylobacter sind beispielsweise weltweit auftretende Bakterien, die ansteckende Durchfall-Erkrankungen auslösen. Campylobacter-Spezies leben vor allem im Verdauungstrakt von Tieren, die meist nicht selbst erkranken. Campylobacter sind in Deutschland die häufigsten bakteriellen Erreger von Durchfall-Erkrankungen.

Die Hauptansteckungsquelle für Campylobacter ist der Verzehr von Lebensmitteln, die mit den Bakterien belastet sind. Häufig erfolgt die Übertragung über Geflügelfleisch. Zwar können sich Campylobacter in Lebensmitteln nicht vermehren, allerdings können Campylobacter einige Zeit in der Umwelt überleben. Auch mangelnde Küchenhygiene kann zu einer Ansteckung führen, beispielsweise über Schneidebretter und/oder Messer, die nach der Zubereitung von rohem Fleisch nicht ausreichend gereinigt werden.

Häufige mit Campylobacter belastet Lebensmittel sind beispielsweise nicht ausreichend erhitztes Geflügelfleisch und Geflügelprodukte, Rohmilch oder Rohmilchprodukte, nicht durchgegartes Hackfleisch oder frische Rohwurstsorten, wie beispielsweise Mettwurst, und verunreinigtes Trinkwasser, zum Beispiel aus einer Brunnenanlage.

Enterohämorrhagische Escherichia coli (EHEC) finden sich im Darm von Wiederkäuern wie Rindern, Schafen, Ziegen, Rehen oder Hirschen. Die Bakterien werden mit dem Kot infizierter Tiere ausgeschieden. Da EHEC relativ unempfindlich sind, können sie in der Umwelt wochenlang überleben. Sie sind hoch ansteckend und schon eine geringe Menge an Keimen reicht für eine Übertragung aus. Das Fell von Rindern und anderen Wiederkäuern kann mit Kotspuren verunreinigt sein. Durch Berühren und Streicheln der Tiere können die Bakterien an die Hände und von dort in den Mund gelangen. Auch das Spielen auf Wiesen, auf denen Wiederkäuer gehalten werden, stellt beispielsweise ein Ansteckungsrisiko für Kinder dar.

Durch Anwendung des erfindungsgemäßen Verfahrens können ebenfalls Oberflächen von Schuhen, beispielsweise Sohlen, auf einfache Weise photodynamisch entkeimt werden.

Weiterhin haben die Erfinder festgestellt, dass das erfindungsgemäße Verfahren auch zur photodynamischen Entkeimung der Oberfläche von Tierprodukten, beispielsweise Fellen, Leder, Haaren, Fasern, oder Wolle, geeignet ist.

Die EHEC-Bakterien können beispielsweise bei mangelnder Händehygiene auch auf berührten Gegenständen haften bleiben und von dort indirekt weiterverbreitet werden.

Eine Übertragung auf den Menschen kann auch durch roh verzehrte oder unzureichend erhitzte Lebensmittel erfolgen. Häufig mit EHEC belastet Lebensmittel sind beispielsweise Rohmilch und Rohmilchprodukte, rohe oder nicht ausreichend durchgegarte Fleischerzeugnisse, wie beispielsweise Rinderhackfleisch (z.B. Hamburger) und streichfähige Rohwurstsorten, beispielsweise Teewurst. Häufig mit EHEC belastet Lebensmittel sindebenfalls pflanzliche Lebensmittel wie Gemüse, das durch Düngen oder über verunreinigtes Wasser mit den Erregern belastet wurden, unpasteurisierte Fruchtsäfte, die aus verunreinigtem Obst hergestellt wurden, Samen, die für das Ziehen von Sprossenkeimlingen verwendet werden, und alle Speisen, auf die der Erreger von verunreinigten Lebensmitteln direkt oder indirekt durch verunreinigte Hände oder Küchenutensilien übertragen wurde.

Clostridium difficile ist beispielsweise ein Bakterium, das weltweit vorkommt. Bei gesunden Menschen ist Clostridium difficile ein harmloses Darmbakterium. Werden konkurrierende Arten der normalen Darmflora durch Antibiotika zurückgedrängt, kann sich Clostridium difficile vermehren und Toxine produzieren, die zu einer unter Umständen lebensbedrohenden Durchfallserkrankung, beispielsweise Antibiotikaassoziierte Kolitis, führen können, insbesondere wenn bereits vorher eine Antibiotika-assoziierte Diarrhoe eingetreten ist.

Clostridium difficile ist einer der häufigsten Krankenhauskeime (nosokomialen Erreger). Darüber hinaus kann Clostridium difficile widerstandsfähige Dauerformen, sogenannte Sporen, bilden, durch die die Bakterien auch außerhalb des Magen-Darm-Traktes ggf. jahrelang überleben können. Eine Übertragung kann deshalb auch über Gegenstände und Flächen erfolgen, an denen die Erreger haften, wie zum Beispiel Toiletten, Türklinken, Griffe und/oder Handläufe.

Die oben beschriebenen Probleme können bei Anwendung des erfindungsgemäßen Verfahrens vermieden werden, da krankheitsauslösende Erreger auf kontaminierten Flächen nach Anwendung des erfindungsgemäßen Verfahrens wirksam beseitigt werden.

Bei einer weiteren bevorzugten Ausführungsform wird wenigstens eine erfindungsgemäße Dispersion zur Inaktivierung von Mikroorganismen in einem Raum, beispielsweise einem Reinraum oder einem Operationssaal verwendet. Nach Einbringen in den Raum, beispielsweise durch Vernebeln, Versprühen, Verspritzen oder Verdampfen, kann der Raum mit einer geeigneten Strahlenquelle, die elektromagnetische Strahlung geeigneter Wellenlänge und Energiedichte erzeugt, bestrahlt werden, wodurch vorhandene Mikroorganismen inaktiviert werden.

Bei einer weiteren bevorzugten Ausführungsform wird wenigstens eine erfindungsgemäße Dispersion zur Inaktivierung von Mikroorganismen in einer Flüssigkeit oder flüssige Zubereitungen verwendet. Geeignete Flüssigkeit oder flüssige Zubereitungen sind beispielsweise Dispersionsfarben, Kühlstoffe, Kühlschmierstoffe, Schmierstoffe, Bremsflüssigkeiten, Lacke, Klebstoffe, oder Ölen. Vorzugsweise ist die flüssige Zubereitung eine wässrige Zubereitung.

Vorzugsweise handelt es sich bei der Flüssigkeit um Wasser.

Dabei kann wenigstens eine erfindungsgemäße Dispersion zur Aufbereitung von Wasser für die Getränke- und Lebensmittelindustrie, die Pharma-, Chemie- und Kosmetikindustrie, die Elektroindustrie verwendet werden. Ferner kann wenigstens eine erfindungsgemäße Dispersion bei der Trinkwasser- und Regenwasseraufbereitung, der Behandlung von Abwasser oder bei der Aufbereitung von Wasser für den Einsatz in der Klimatechnik eingesetzt werden.

Geeignete Gegenstände sind beispielsweise Medizinprodukte, Lebensmittelverpackungen, Hygieneartikel, Textilien, Handgriffe, Handläufe, Kontaktlinsen, Baustoffe, Geldscheine, Münzen, Spielchips, Karten, Sportgeräte, Textilien, Geschirr, Besteck, oder elektronische Geräte. Geeignete Gegenstände sind ebenfalls Geräte oder Anlagen mit wasserführenden Leitungen und/oder wasserführenden Behältern, bei denen beispielsweise während des Betriebs des Gerätes oder der Anlage Kondenswasserbildung auftritt.

Geeignete Gegenstände sind beispielsweise Dichtungen, Membranen, Siebe, Filter, Behälter und/oder Rohre von Warmwassererzeugungsanlagen, Warmwasserverteilungsanlagen, Wärmeaustauschern, Klimaanlagen, Luftbefeuchtern, Kühlanlagen, Kühlschränken, Getränkeautomaten, Waschmaschinen oder Trocknern.

Beispielsweise können geringe Mengen von Mikroorganismen trotz Filterung der von Außen zugeführten Luft in eine Klimaanlage gelangen und dort zumindest kurzzeitig existieren. Die Stoffwechselprodukte dieser Mikroorganismen können zu modrigen und muffigen Gerüchen führen.

Weiterhin wird beispielsweise beim Betrieb einer Klimaanlage der Luft Feuchtigkeit entzogen und aufgefangen. Ein Großteil des Kondenswassers wird entfernt und läuft beispielsweise durch einen Kondenswasserablauf ab. Dennoch bleibt eine Restfeuchte auf der Oberfläche des Verdampfers der Klimaanlage zurück, insbesondere dann, wenn die Klimaanlage beispielsweise in einem PKW erst mit dem Abschalten des Motors ausgeschaltet wird und sich die Temperatur nicht mehr ausgleichen kann.

Durch die Luft auf den Verdampfer gelangte Mikroorganismen, beispielsweise Pilzsporen und/oder Bakterien, finden nun ein ideales, feucht-warmes Klima vor und können sich ungehindert ausbreiten.

Da beispielsweise Schimmelpilze ein Gesundheitsrisiko darstellen, sollte die Klimaanlage regelmäßig entkeimt und vorhandene Mikroorganismen durch Anwendung des erfindungsgemäßen Verfahrens abgetötet werden.

Bei einem Wechsel der Filter der Klimaanlage, beispielsweise Staub- und/oder Pollenfilter, kann weiterhin das Filtergehäuse und die angrenzenden Luftkanäle der Klimaanlage durch Anwendung des erfindungsgemäßen Verfahrens gereinigt werden. Mittels der Reinigung des Verdampfers der Klimaanlage durch Anwendung des erfindungsgemäßen Verfahrens können weiterhin Geruchsbelästigungen, die durch die Klimaanlage entstehen, beseitigt werden.

Legionellen sind beispielsweise Bakterien, die beim Menschen unterschiedliche Krankheitsbilder verursachen, beispielsweise grippeartige Beschwerden oder schwere Lungenentzündungen. Legionellen vermehren sich vorzugsweise bei Temperaturen zwischen 25 °C und 45 °C. Besonders in künstlichen Wassersystemen wie Wasserleitungen in Gebäuden finden die Erreger aufgrund der vorherrschenden Temperaturen gute Wachstumsbedingungen. In Ablagerungen und/oder Belägen eines Rohrsystems können sich Legionellen ebenfalls gut vermehren. Daher kann das erfindungsgemäße verfahren beispielsweise in Kombination mit einem Verfahren zur Beseitigung der Ablagerungen und/oder Belägen verwendet werden.

Legionellen werden durch zerstäubtes, vernebeltes Wasser übertragen. Die erregerhaltigen Tröpfchen können sich in der Luft verbreiten und eingeatmet werden. Mögliche Ansteckungsquellen sind beispielsweise Warmwasserversorgungen, insbesondere Duschen, Luftbefeuchter oder Wasserhähne, ebenso Kühltürme oder Klimaanlagen oder sonstige Anlagen, die Wasser zu Wassertröpfchen zerstäuben, beispielsweise Nebelerzeuger, Nebelbrunnen, Zierspringbrunnen oder Ähnliches. Auch in Schwimmbädern ist über Wasserfälle, Rutschen, Warmsprudelbecken (Whirlpools) und/oder Fontänen eine Übertragung möglich. Eine Ansteckung mit Legionellen wird durch Anwendung des erfindungsgemäßen Verfahrens auf Oberflächen kontaminierter Gegenstände verhindert.

Das erfindungsgemäße Verfahren kann beispielsweise in Geräten oder Anlagen mit wasserführenden Leitungen und/oder wasserführenden Behältern, beispielsweise Geräte oder Anlagen, die in der Fischzucht verwendet werden, angewendet werden.

Epidemieartige Fischkrankheiten sind beispielsweise eine große wirtschaftliche Bedrohung für alle intensiv bewirtschafteten Fischfarmen, wo Nutzfische auf engem Raum gehalten werden. Zur Bekämpfung von Fischkrankheiten werden beispielsweise Antibiotika und/oder chemische Additive eingesetzt. Als chemische Additive werden beispielsweise Löschkalk (Calciumhydroxid), Wasserstoffperoxid, Peressigsäurepräparate, Kupfersulfat, Chloramine, Natriumcarbonat, Natriumchlorid oder Formaldehyd verwendet.

Um den Einsatz von Antibiotika und/oder oben genannte chemische Additive zu verringern, kann wenigstens eine erfindungsgemäß zu verwendende Dispersion zur photodynamischen Entkeimung von Geräten oder Anlagen der Fischzucht, beispielsweise Fischbecken, Teichen, Pumpen, Filtern, Rohren, Netzen, Haken oder Fußmatten, verwendet werden. Ebenso können beispielsweise Fische und/oder Fischeier photodynamisch entkeimt werden. Weiterhin können Terrarien, Aquarium-Behälter, Sand, Kies, und/oder Grünpflanzen vor und/oder während ihrer Verwendung photodynamisch entkeimt werden.

Geeignete elektronische Geräte umfassen beispielsweise Herdplatten, Fernbedienungen, Kopfhöhrer, Freisprecheinrichtungen, Headsets, Mobiltelefone, Bedienelemente, wie Knöpfe, Schalter, Touch-Screens, oder Tastaturen. Geeignete Baustoffe umfassen beispielsweise Beton, Glas, Sand, Kies, Wandverkleidungen, Putz, Estrich oder Ähnliches.

Geeignete Wandverkleidung umfassen beispielsweise Holzvertäfelungen, Fliesen, Massivholzplatten, mitteldichte Holzfaserplatten, Sperrholzplatten, Multiplex-Platten, Faserbetonplatten, Gipskartonplatten, Gipsfaserplatten, Tapeten aus Kunststoff, Schaumstoff und/oder Zellulose

Beispielsweise kann wenigstens eine erfindungsgemäß zu verwendende Dispersion zur Beseitigung von Schimmelpilzen verwendet werden.

Vorzugsweise wird eine mit Schimmelpilz behaftete Oberfläche mit wenigstens einer erfindungsgemäß zu verwendenden Dispersion behandelt und nachfolgend mit einer geeigneten Strahlenquelle, die elektromagnetische Strahlung geeigneter Wellenlänge und Energiedichte erzeugt, bestrahlt werden, wodurch es nachfolgend zu einer Reduzierung, vorzugsweise Inaktivierung, von Schimmelpilz auf der behandelten Oberfläche kommt.

Bei den vorgenannte bevorzugten Ausführungsform der erfindungsgemäßen Verwendung oder des erfindungsgemäßen Verfahrens erfolgt die Bestrahlung der Mikroorganismen und der wenigstens einen erfindungsgemäß zu verwendenden Dispersion mit elektromagnetischer Strahlung geeigneter Wellenlänge und Energiedichte in Gegenwart wenigstens einer Sauerstoff-abgebenden Verbindung, vorzugsweise Peroxid, und/oder wenigstens eines Sauerstoff-haltigen Gases, vorzugsweise Sauerstoff.

Die wenigstens eine Sauerstoff-abgebende Verbindung und/oder das wenigstens eine Sauerstoff-haltige Gas können vorzugsweise vor, oder während der Bestrahlung mit elektromagnetischer Strahlung geeigneter Wellenlänge und Energiedichte appliziert werden.

Durch eine zusätzliche Gabe von Sauerstoff in Form wenigstens einer Sauerstoff-haltigen Verbindung und/oder wenigstens eines Sauerstoff-haltigen Gases vor oder während der Bestrahlung der Mikroorganismen und des wenigstens einen Photosensibilisators mit elektromagnetischer Strahlung geeigneter Wellenlänge und Energiedichte wird die Ausbeute an entstandenen reaktiven Sauerstoffspezies (ROS), vorzugsweise Sauerstoffradikale und/oder Singulett-Sauerstoff erhöht..

Gemäß einem Aspekt 1 betrifft die vorliegende Erfindung eine Photosensibilisator-Dispersion umfassend:
(a) wenigstens einen Photosensibilisator, der aus der Gruppe, die aus Phenalenonen, Flavinen und Mischungen davon, besteht, ausgewählt wird,
(b) wenigstens eine flüssige, polare Phase, und
(c) wenigstens ein Tensid,
wobei die Dispersion bei einem Druck aus einem Bereich von 800 bis 1200 mbar und einer Temperatur aus einem Bereich von 2 bis 50°C eine Mikroemulsion, vorzugsweise eine Öl-in-Wasser (O/W) Mikroemulsion, ein Gel oder eine Mischung davon umfasst oder daraus besteht.

Gemäß einem Aspekt 2 betrifft die vorliegende Erfindung eine Photosensibilisator-Dispersion gemäß Aspekt 1, wobei der wenigstens eine Photosensibilisator positiv geladen, negativ geladen oder ungeladen ist, wobei der wenigstens eine Photosensibilisator weiter bevorzugt wenigstens einen organischen Rest mit a) wenigstens einem neutralen, protonierbaren Stickstoffatom und/oder b) wenigstens einem positiv geladenen Stickstoffatom aufweist.

Gemäß einem Aspekt 3 betrifft die vorliegende Erfindung eine Photosensibilisator-Dispersion gemäß einem der Aspekte 1 bis 2, wobei der wenigstens eine Photosensibilisator, wobei der wenigstens eine Photosensibilisator ein Phenalenon-Derivat ist, das aus der Gruppe, die aus den Verbindungen mit den Formeln (2) bis (28) und Mischungen davon besteht, ausgewählt wird:

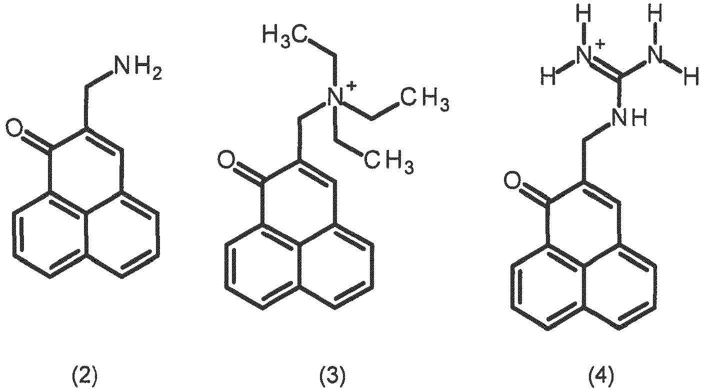

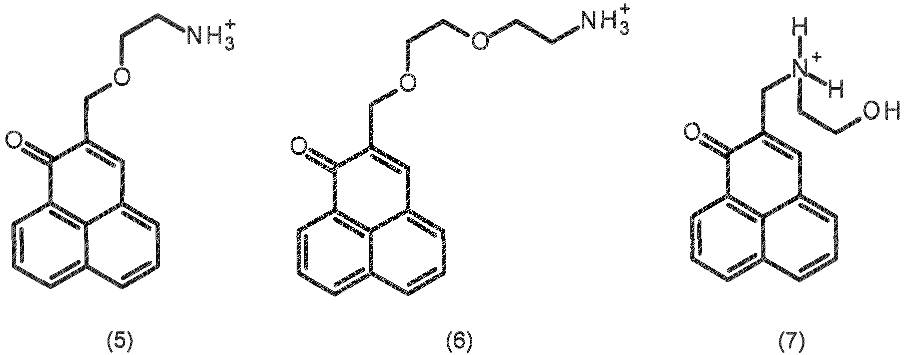

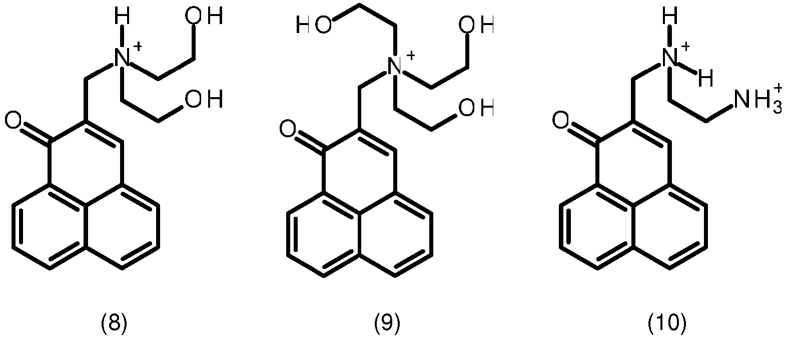

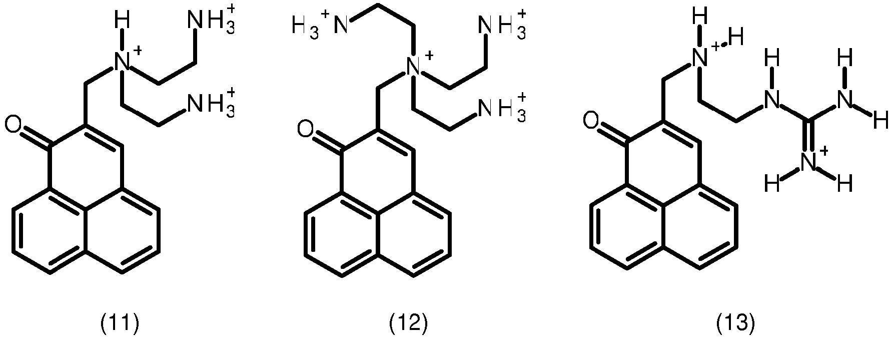

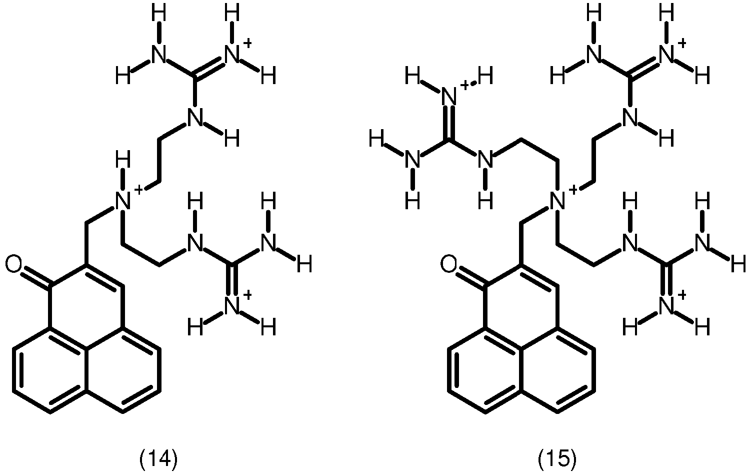

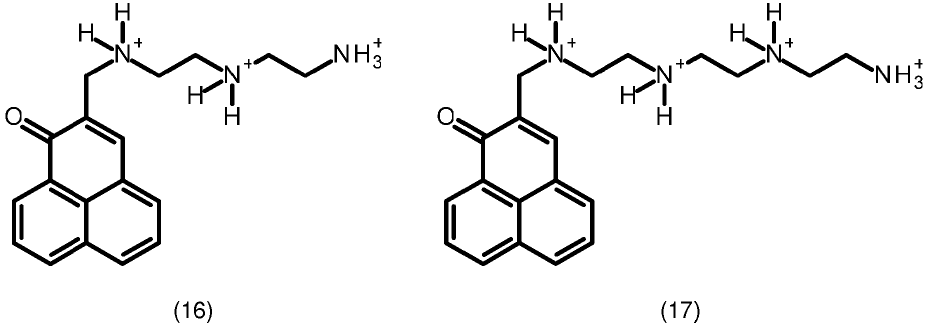

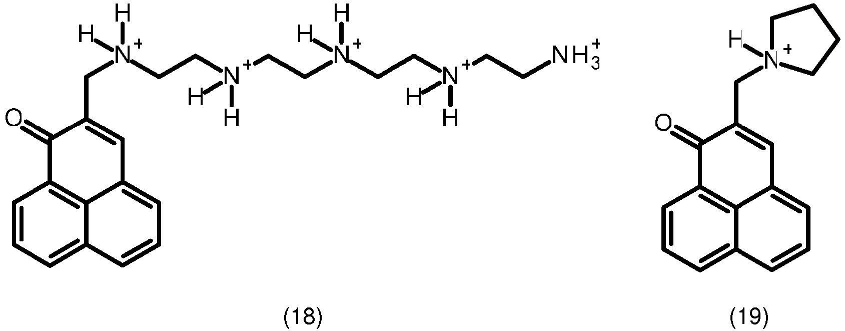

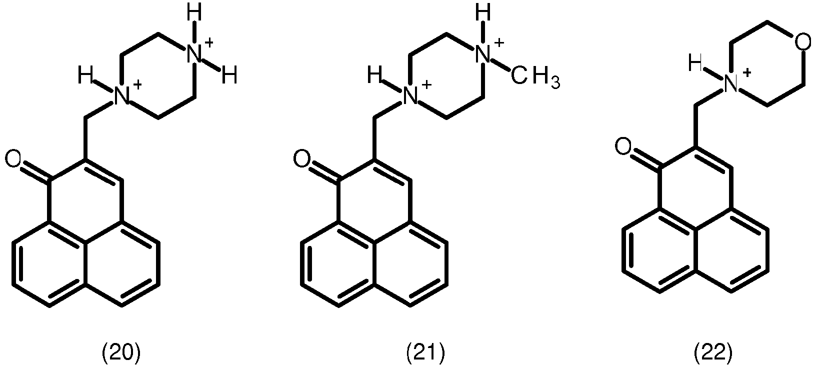

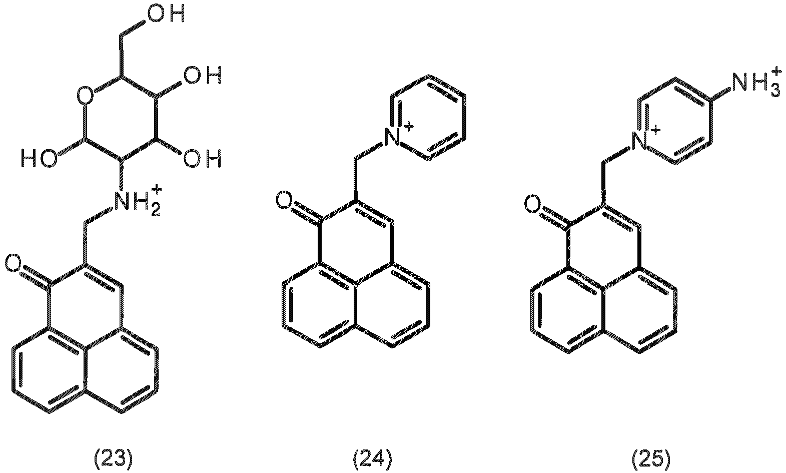

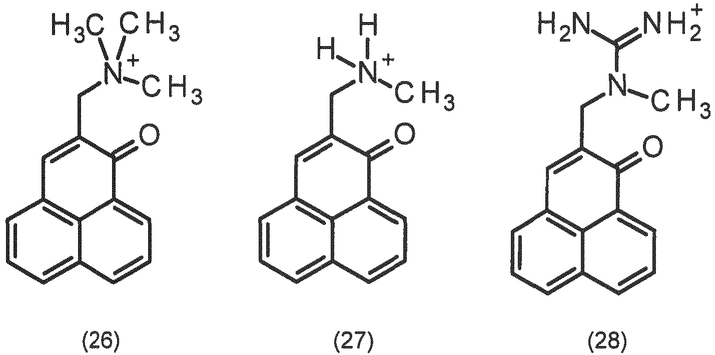

Gemäß einem Aspekt 4 betrifft die vorliegende Erfindung eine Photosensibilisator-Dispersion gemäß einem der Aspekte 1 bis 3, wobei der wenigstens eine Photosensibilisator ein Flavin-Derivat aus der Gruppe, die aus den Verbindung mit den Formeln (32) bis (49), (51) bis (64) und Mischungen davon besteht, ausgewählt wird:

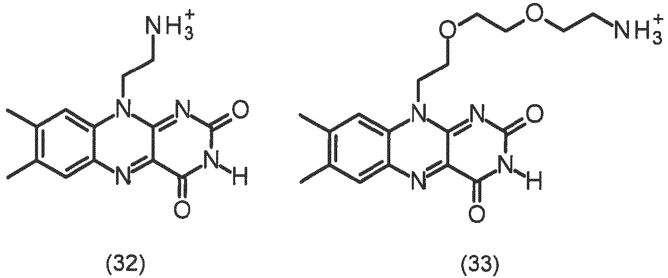

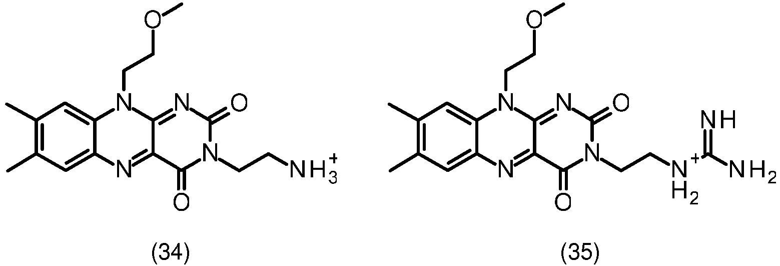

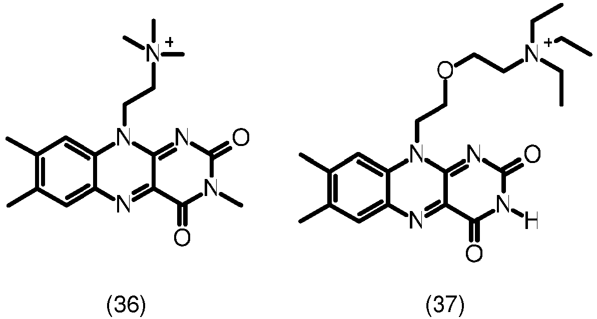

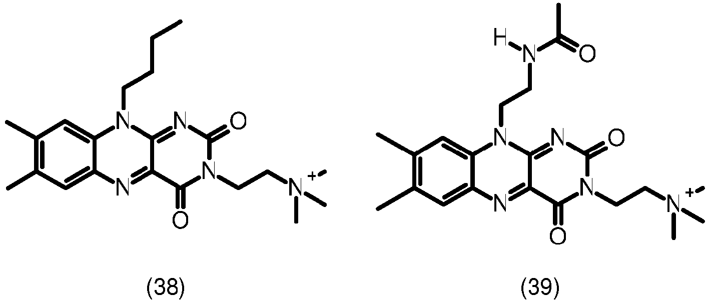

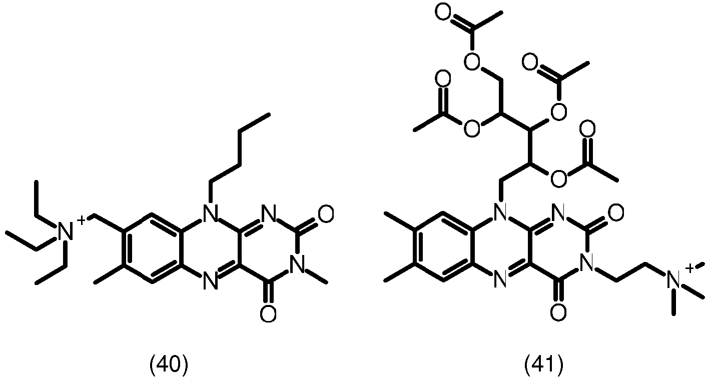

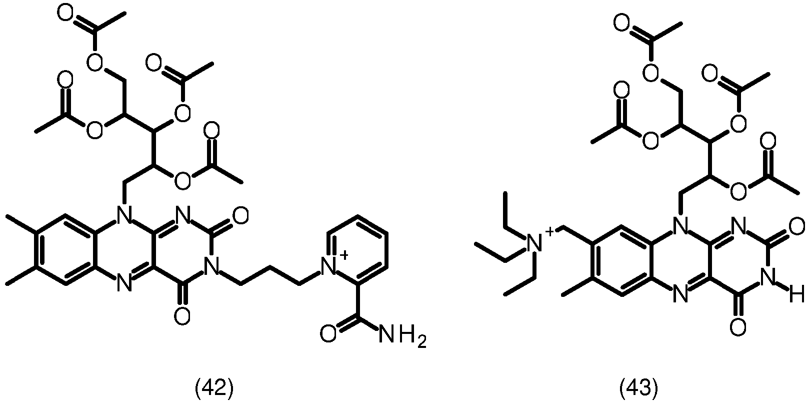

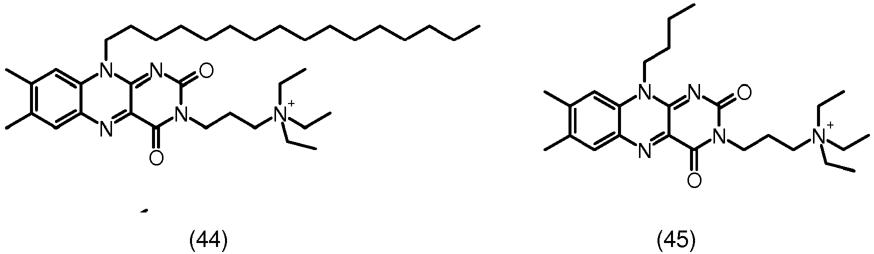

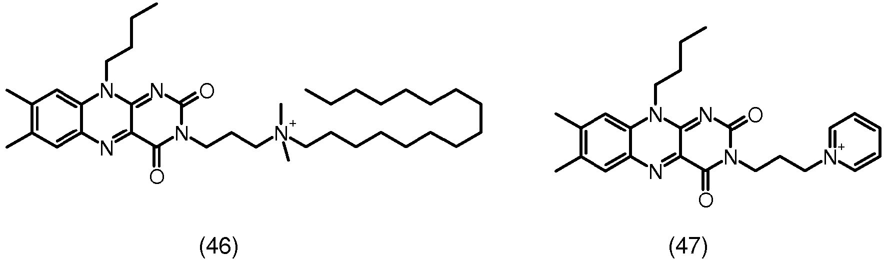

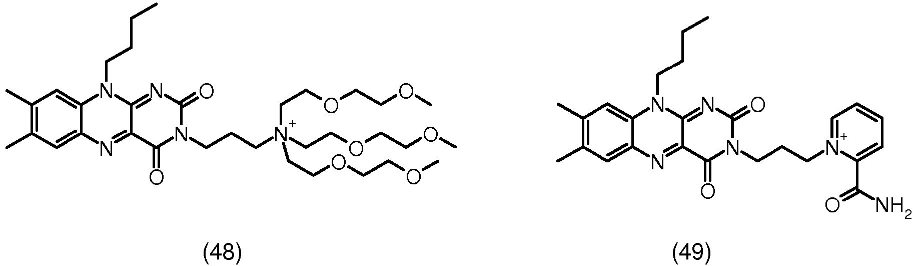

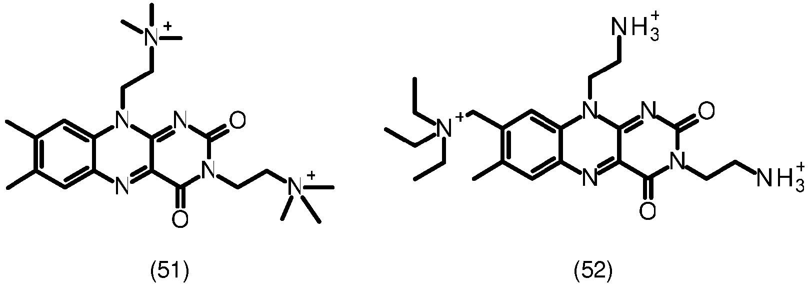

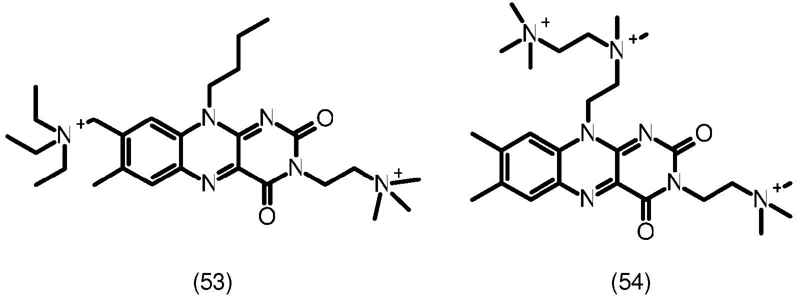

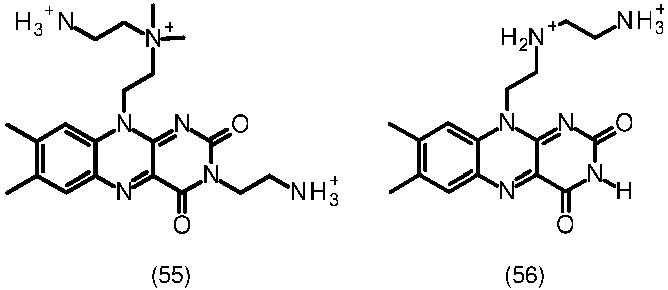

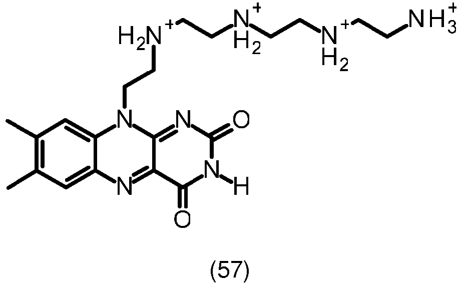

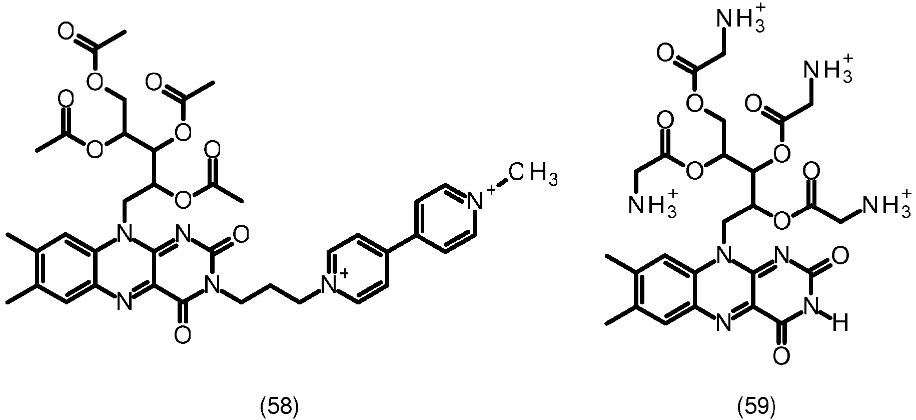

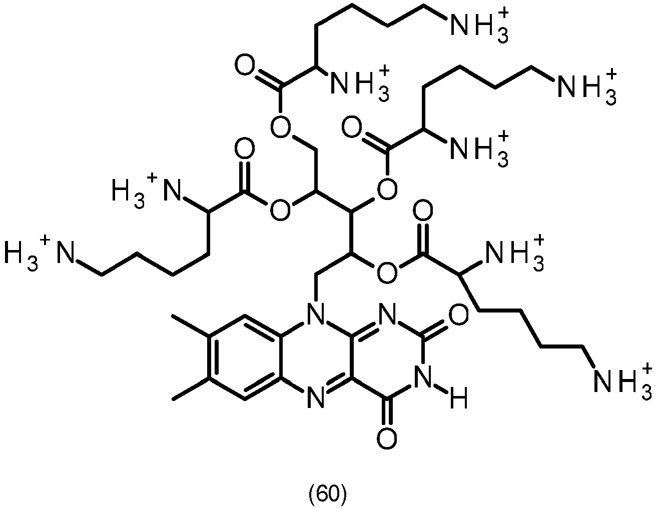

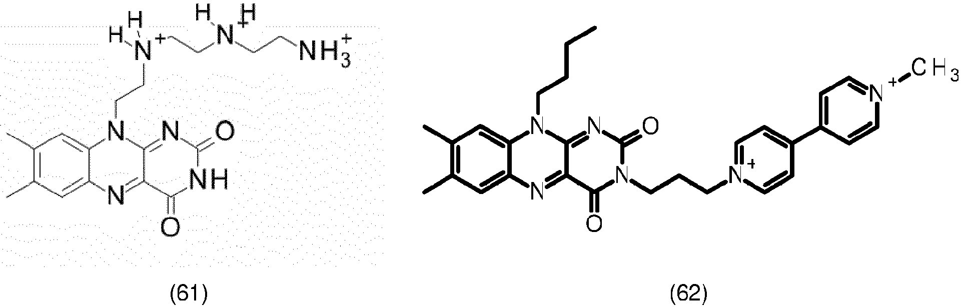

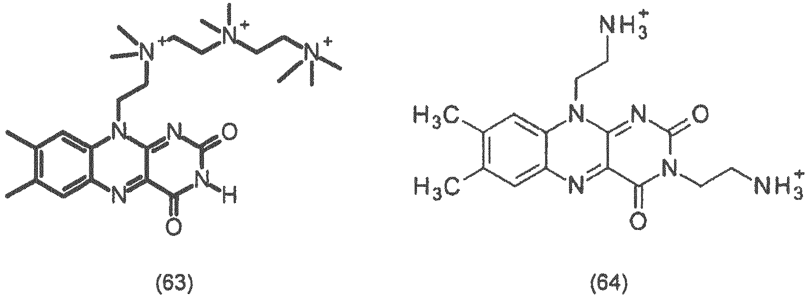

Gemäß einem Aspekt 6 betrifft die vorliegende Erfindung eine Photosensibilisator-Dispersion gemäß einem der Aspekte 1 bis 5, wobei der wenigstens eine Photosensibilisator aus der Gruppe, die aus den Verbindungen mit den Formeln (2) bis (28), (32) bis (49), (51) bis (64), und Mischungen davon besteht, ausgewählt wird.

Gemäß einem Aspekt 7 betrifft die vorliegende Erfindung eine Photosensibilisator-Dispersion gemäß einem der Aspekte 1 bis 6, wobei als Gegenion zum positiv geladenen Stickstoffatom wenigstens ein Anion ausgewählt wird, das aus der Gruppe, die aus Fluorid, Chlorid, Bromid, Iodid, Sulfat, Hydrogensulfat, Phosphat, Dihydrogenphosphat, Hydrogenphosphat, Tosylat, Mesylat, Formiat, Acetat, Propionat, Butanoat, Oxalat, Tartrat, Fumarat, Benzoat, Citrat und Mischungen davon besteht.

Gemäß einem Aspekt 8 betrifft die vorliegende Erfindung eine Photosensibilisator-Dispersion gemäß einem der Aspekte 1 bis 7, wobei die Dispersion den wenigstens einen Photosensibilisator in einer Konzentration aus einem Bereich von 0,1 µM bis 1000 µM, vorzugsweise aus einem Bereich von 1 µM bis 750 µM, weiter bevorzugt aus einem Bereich von 2 µM bis 500 µM umfasst.

Gemäß einem Aspekt 9 betrifft die vorliegende Erfindung eine Photosensibilisator-Dispersion gemäß einem der Aspekte 1 bis 8, wobei die wenigstens eine flüssige, polare Phase wenigstens ein polares Lösungsmittel, vorzugsweise Wasser, umfasst.

Gemäß einem Aspekt 10 betrifft die vorliegende Erfindung eine Photosensibilisator-Dispersion gemäß einem der Aspekte 1 bis 9, wobei die Dispersion das wenigstens eine polare Lösungsmittel, vorzugsweise Wasser, in einem Anteil von wenigstens 0,1 Gew.-%, vorzugsweise von wenigstens 0,5 Gew.-%, weiter bevorzugt von wenigstens 1 Gew.-%, weiter bevorzugt von wenigstens 4 Gew.-%, weiter bevorzugt von wenigstens 10 Gew.-%, weiter bevorzugt von wenigstens 35 Gew.-%, weiter bevorzugt von wenigstens 50 Gew.-%, weiter bevorzugt von wenigstens 51 Gew.-%, jeweils bezogen auf das Gesamtgewicht der Dispersion, umfasst.

Gemäß einem Aspekt 11 betrifft die vorliegende Erfindung eine Photosensibilisator-Dispersion gemäß einem der Aspekte 1 bis 10, wobei die Dispersion das wenigstens eine polare Lösungsmittel, vorzugsweise Wasser, in einem Anteil von aus einem Bereich von 0,1 Gew.-% bis 99,8 Gew.-%, vorzugsweise aus einem Bereich von 0,5 Gew.-% bis 99 Gew.-%, weiter bevorzugt aus einem Bereich von 4 Gew.-% bis 98 Gew.-%, weiter bevorzugt aus einem Bereich von 10 Gew.-% bis 97 Gew.-%. weiter bevorzugt aus einem Bereich von 35 Gew.-% bis 96 Gew.-%, weiter bevorzugt aus einem Bereich von 50 Gew.-% bis 95 Gew.-%, weiter bevorzugt aus einem Bereich von 51 Gew.-% bis 94 Gew.-%, weiter bevorzugt aus einem Bereich von 53 Gew.-% bis 93 Gew.-%, weiter bevorzugt aus einem Bereich von 70 Gew.-% bis 92 Gew.-%, jeweils bezogen auf das Gesamtgewicht der Dispersion, umfasst.

Gemäß einem Aspekt 12 betrifft die vorliegende Erfindung eine Photosensibilisator-Dispersion gemäß einem der Aspekte 1 bis 11, wobei das wenigstens eine Tensid aus der Gruppe, die aus den oben genannten nichtionischen Tensiden, den oben genannten anionischen Tensiden, den oben genannten kationischen Tensiden, den oben genannten amphoteren Tensiden und Mischungen davon, vorzugsweise den oben genannten nichtionischen Tensiden, den oben genannten anionischen Tensiden und Mischungen davon, besteht, ausgewählt wird.

Gemäß einem Aspekt 13 betrifft die vorliegende Erfindung eine Photosensibilisator-Dispersion gemäß einem der Aspekte 1 bis 12, wobei die Dispersion das wenigstens eine Tensid in einem Anteil aus einem Bereich von 0,1 Gew.-% bis 65 Gew.-%, vorzugsweise aus einem Bereich von 1 Gew.-% bis 55 Gew.-%, weiter bevorzugt aus einem Bereich von 3 Gew.-% bis 50 Gew.-%, weiter bevorzugt aus einem Bereich von 5 Gew.-% bis 41 Gew.-%, weiter bevorzugt aus einem Bereich von 7 Gew.-% bis 37 Gew.-%, weiter bevorzugt aus einem Bereich von 9 Gew.-% bis 30 Gew.-%, weiter bevorzugt aus einem Bereich von 10 Gew.-% bis 27 Gew.-%, jeweils bezogen auf das Gesamtgewicht der Dispersion, umfasst.

Gemäß einem Aspekt 14 betrifft die vorliegende Erfindung eine Photosensibilisator-Dispersion gemäß einem der Aspekte 1 bis 13, wobei die nichtionischen Tenside aus der Gruppe, die aus den oben genannten Polyalkylenglycolethern, den oben genannten Alkylglucosiden, den oben genannten Alkylpolyglycosiden, den oben genannten Alkylglycosidestern und Mischungen davon besteht, ausgewählt werden.

Gemäß einem Aspekt 15 betrifft die vorliegende Erfindung eine Photosensibilisator-Dispersion gemäß einem der Aspekte 1 bis 14, wobei die anionischen Tenside aus der Gruppe, die aus den oben genannten Alkylcarboxylaten, den oben genannten Alkylsulfonaten, den oben genannten Alkylsulfaten, den oben genannten Alkylphoshaten, den oben genannten Alkylpolyglykolethersulfaten, den oben genannten Sulfonaten von Alkylcarbonsäureestern, den oben genannten N-Alkyl-Sarkosinaten und Mischungen davon besteht, ausgewählt werden.

Gemäß einem Aspekt 16 betrifft die vorliegende Erfindung eine Photosensibilisator-Dispersion gemäß einem der Aspekte 1 bis 15, wobei die kationische Tenside aus der Gruppe, die aus den oben genannten quartäre Alkylammoniumsalze, aus den oben genannten Esterquats, aus den oben genannten acylierte Polyamine, aus den oben genannten Benzylammoniumsalze und Mischungen davon besteht, ausgewählt werden. Gemäß einem Aspekt 17 betrifft die vorliegende Erfindung eine Photosensibilisator-Dispersion gemäß einem der Aspekte 1 bis 16, wobei die Dispersion weiterhin wenigstens eine flüssige, unpolare Phase aufweist, die wenigstens ein unpolares Lösungsmittel, das aus der Gruppe, die aus den oben genannten acyclischen Alkanen mit 5 bis 30 Kohlenstoffatomen, den oben genannten cyclischen Alkanen mit 5 bis 13 Kohlenstoffatomen, den oben genannten Perfluoralkanen mit 5 bis 20 Kohlenstoffatomen, den oben genannten Monocarbonsäureestern mit vorzugsweise 4 bis 20 Kohlenstoffatomen, den oben genannten Polycarbonsäureestern mit vorzugsweise 6 bis 20 Kohlenstoffatomen, und Mischungen davon ausgewählt wird, umfasst.

Gemäß einem Aspekt 18 betrifft die vorliegende Erfindung eine Photosensibilisator-Dispersion gemäß einem der Aspekte 1 bis 17, wobei die Dispersion das wenigstens eine unpolare Lösungsmittel in einem Anteil von wenigstens 0,1 Gew.-%, vorzugsweise von wenigstens 0,5 Gew.-%, weiter bevorzugt von wenigstens 1 Gew.-%, weiter bevorzugt von wenigstens 4 Gew.-%, weiter bevorzugt von wenigstens 10 Gew.-%, weiter bevorzugt von wenigstens 35 Gew.-%, weiter bevorzugt von wenigstens 50 Gew.-%, weiter bevorzugt von wenigstens 51 Gew.-%, jeweils bezogen auf das Gesamtgewicht der Dispersion, umfasst.

Gemäß einem Aspekt 19 betrifft die vorliegende Erfindung eine Photosensibilisator-Dispersion gemäß einem der Aspekte 1 bis 18, wobei die Dispersion das wenigstens eine unpolare Lösungsmittel in einem Anteil von aus einem Bereich von 0,1 Gew.-% bis 99,8 Gew.-%, vorzugsweise aus einem Bereich von 0,5 Gew.-% bis 99 Gew.-%, weiter bevorzugt aus einem Bereich von 1 Gew.-% bis 96 Gew.-%, weiter bevorzugt aus einem Bereich von 1,5 Gew.-% bis 90 Gew.-%, weiter bevorzugt aus einem Bereich von 3 Gew.-% bis 80 Gew.-%, weiter bevorzugt aus einem Bereich von 5 Gew.-% bis 75 Gew.-%, weiter bevorzugt aus einem Bereich von 10 Gew.-% bis 60 Gew.-%, weiter bevorzugt aus einem Bereich von 12 Gew.-% bis 49 Gew.-%, jeweils bezogen auf das Gesamtgewicht der Dispersion, umfasst.

Gemäß einem Aspekt 20 betrifft die vorliegende Erfindung eine Photosensibilisator-Dispersion gemäß einem der Aspekte 1 bis 19, wobei die Dispersion weiterhin wenigstens ein Alkanol mit 2 bis 12 Kohlenstoffatomen, und vorzugsweise mit 1 bis 6 OH-Gruppen, enthält.

Gemäß einem Aspekt 21 betrifft die vorliegende Erfindung eine Photosensibilisator-Dispersion gemäß einem der Aspekte 1 bis 20, wobei die Dispersion das wenigstens ein Alkanol in einem Anteil aus einem Bereich von 0 Gew.-% bis 50 Gew.-%, vorzugsweise aus einem Bereich von 0,1 Gew.-% bis 40 Gew.-%, weiter bevorzugt aus einem Bereich von 0,5 Gew.-% bis 35 Gew.-%, weiter bevorzugt aus einem Bereich von 1 Gew.-% bis 30 Gew.-%, weiter bevorzugt aus einem Bereich von 1,5 Gew.-% bis 25 Gew.-%, weiter bevorzugt aus einem Bereich von 5 Gew.-% bis 20 Gew.-%, weiter bevorzugt aus einem Bereich von 7 Gew.-% bis 19 Gew.-%, weiter bevorzugt aus einem Bereich von 10 Gew.-% bis 17 Gew.-%, jeweils bezogen auf das Gesamtgewicht der Dispersion, umfasst.

Gemäß einem Aspekt 22 betrifft die vorliegende Erfindung eine Photosensibilisator-Dispersion gemäß einem der Aspekte 1 bis 21, wobei die Dispersion bei einem Druck aus einem Bereich von 800 bis 1200 mbar und einer Temperatur aus einem Bereich von 2 bis 50°C eine Öl-in-Wasser (O/W) Mikroemulsion, eine Wasser-in-Öl (W/O) Mikroemulsion oder eine bikontinuierliche Mikroemulsion, vorzugsweise eine Öl-in-Wasser (O/W) Mikroemulsion oder eine Wasser-in-Öl (W/O) Mikroemulsion, umfasst oder daraus besteht.

Gemäß einem Aspekt 23 betrifft die vorliegende Erfindung eine Photosensibilisator-Dispersion gemäß einem der Aspekte 1 bis 22, wobei die Dispersion eine Mikroemulsion, vorzugsweise eine Öl-in-Wasser (O/W) Mikroemulsion, umfasst oder ist, die:
(a) wenigstens einen Photosensibilisator, der aus der Gruppe, die aus den oben genannten Phenalenonen, den oben genannten Flavinen und Mischungen davon, weiter bevorzugt aus den Verbindungen mit den Formeln (2) bis (25), (32) bis (49), (51) bis (64), und Mischungen davon, besteht, ausgewählt wird,
(b) wenigstens ein polares Lösungsmittel, vorzugsweise Wasser,
(c) wenigstens ein Tensid, das aus der Gruppe, die aus den oben genannten nichtionischen Tensiden, den oben genannten anionischen Tensiden, den oben genannten kationischen Tensiden, den oben genannten amphoteren Tensiden und Mischungen davon, vorzugsweise den oben genannten nichtionischen Tensiden, den oben genannten anionischen Tensiden und Mischungen davon, besteht, ausgewählt wird, und
(d) wenigstens ein unpolares Lösungsmittel, das weiter bevorzugt aus der Gruppe, die aus den oben genannten acyclischen Alkanen mit 5 bis 30 Kohlenstoffatomen, den oben genannten cyclischen Alkanen mit 5 bis 13 Kohlenstoffatomen, den oben genannten Perfluoralkanen mit 5 bis 20 Kohlenstoffatomen, den oben genannten Monocarbonsäureester mit 4 bis 20 Kohlenstoffatomen, den oben genannten Polycarbonsäureester mit 6 bis 20 Kohlenstoffatomen und Mischungen davon besteht, ausgewählt wird, und
(e) optional, wenigstens ein Alkanol, das aus der Gruppe, die aus den oben genannten Alkanolen mit 2 bis 12 Kohlenstoffatomen und vorzugsweise mit 1 bis 6 OH-Gruppen und Mischungen davon besteht, ausgewählt wird,
umfasst.

Gemäß einem Aspekt 24 betrifft die vorliegende Erfindung eine Photosensibilisator-Dispersion gemäß einem der Aspekte 1 bis 23, wobei die Dispersion eine Öl-in-Wasser (O/W) Mikroemulsion ist, die vorzugsweise das wenigstens eine unpolare Lösungsmittel in einem Anteil aus einem Bereich vom 0,1 Gew.-% bis 49,9 Gew.-%, vorzugsweise aus einem Bereich von 0,5 Gew.-% bis 48 Gew.-%, weiter bevorzugt aus einem Bereich von 1 Gew.-% bis 45 Gew.%, weiter bevorzugt aus einem Bereich von 3 Gew.-% bis 40 Gew.%, weiter bevorzugt aus einem Bereich von 5 Gew.-% bis 35 Gew.%, weiter bevorzugt aus einem Bereich von 7 Gew.-% bis 30 Gew.%, jeweils bezogen auf das Gesamtgewicht der Mikroemulsion, und vorzugsweise das wenigstens eine polare Lösungsmittel, vorzugsweise Wasser, in einem Anteil aus einem Bereich vom 50 Gew.-% bis 99,8 Gew.-%, vorzugsweise aus einem Bereich von 51 Gew.-% bis 99 Gew.-%, weiter bevorzugt aus einem Bereich von 52 Gew.-% bis 96 Gew.%, weiter bevorzugt aus einem Bereich von 53 Gew.-% bis 90 Gew.%, weiter bevorzugt aus einem Bereich von 54 Gew.-% bis 85 Gew.%, jeweils bezogen auf das Gesamtgewicht der Mikroemulsion, und vorzugsweise das wenigstens eine Tensid in einem Anteil aus einem Bereich von 0,1 Gew.-% bis 45 Gew.-%, vorzugsweise aus einem Bereich von 0,5 Gew.-% bis 40 Gew.-%, weiter bevorzugt aus einem Bereich von 1 Gew.-% bis 35 Gew.%, weiter bevorzugt aus einem Bereich von 3 Gew.-% bis 30 Gew.%, weiter bevorzugt aus einem Bereich von 5 Gew.-% bis 27 Gew.%, weiter bevorzugt aus einem Bereich von 7 Gew.-% bis 25 Gew.%, weiter bevorzugt aus einem Bereich von 10 Gew.-% bis 20 Gew.%, jeweils bezogen auf das Gesamtgewicht der Mikroemulsion, und optional weiterhin das wenigstens ein Alkanol in einem Anteil aus einem Bereich von 0 Gew.-% bis 50 Gew.-%, vorzugsweise aus einem Bereich von 0,1 Gew.-% bis 40 Gew.%, weiter bevorzugt aus einem Bereich von 0,5 Gew.-% bis 35 Gew.%, weiter bevorzugt aus einem Bereich von 1 Gew.-% bis 30 Gew.%, weiter bevorzugt aus einem Bereich von 1,5 Gew.-% bis 25 Gew.%, weiter bevorzugt aus einem Bereich von 5 Gew.-% bis 20 Gew.%, weiter bevorzugt aus einem Bereich von 7 Gew.-% bis 19 Gew.%, weiter bevorzugt aus einem Bereich von 10 Gew.-% bis 17 Gew.%, jeweils bezogen auf das Gesamtgewicht der Mikroemulsion.

Gemäß einem Aspekt 25 betrifft die vorliegende Erfindung eine Photosensibilisator-Dispersion gemäß einem der Aspekte 1 bis 24, wobei die Dispersion weiterhin wenigstens eine pH-regulierende Substanz enthält, die vorzugsweise eine anorganische Säure, organische Säure, anorganische Base, organische Base, ein Salz davon oder eine Mischung davon ist.

Gemäß einem Aspekt 26 betrifft die vorliegende Erfindung eine Photosensibilisator-Dispersion gemäß einem der Aspekte 1 bis 25, wobei die Dispersion weiterhin wenigstens einen Gelbildner, der aus der Gruppe, die aus den oben genannten Carboxyvinylpolymeren, den oben genannten Polyacrylamiden, den oben genannten Alginaten, den oben genannten Celluloseethern und Mischungen davon ausgewählt wird, umfasst.

Gemäß einem Aspekt 27 betrifft die vorliegende Erfindung eine Photosensibilisator-Dispersion gemäß einem der Aspekte 1 bis 26, wobei die Dispersion bei einem Druck aus einem Bereich von 800 bis 1200 mbar und einer Temperatur aus einem Bereich von 2 bis 50°C ein Lyogel, umfasst oder ist.

Gemäß einem Aspekt 28 betrifft die vorliegende Erfindung eine nicht-medizinische Verwendung einer Dispersion nach einem der Aspekte 1 bis 27 zur photodynamischen Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden.

Gemäß einem Aspekt 29 betrifft die vorliegende Erfindung eine Verwendung gemäß Aspekt 28 zur Oberflächenreinigung und/oder Oberflächenbeschichtung eines Gegenstandes.

Gemäß einem Aspekt 30 betrifft die vorliegende Erfindung eine Verwendung nach einem der Aspekte 28 bis 29, zur Oberflächenreinigung und/oder Oberflächenbeschichtung von Medizinprodukten, Lebensmittelverpackungen, Textilien, Baustoffen, elektronische Geräten, Möbeln oder Hygieneartikeln.

Gemäß einem Aspekt 31 betrifft die vorliegende Erfindung eine Verwendung nach einem der Aspekte 28 bis 30, zur Entkeimung von Flüssigkeiten.

Gemäß einem Aspekt 32 betrifft die vorliegende Erfindung eine Verwendung nach einem der Aspekte 28 bis 31, zur Entkeimung von Lebensmitteln.

Gemäß einem Aspekt 33 betrifft die vorliegende Erfindung ein nicht-medizinisches Verfahren zur photodynamischen Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, wobei das Verfahren folgende Schritte umfasst:
(A) in Kontakt bringen der Mikroorganismen mit wenigstens einer Dispersion nach einem der Aspekte 1 bis 27, und
(B) Bestrahlen der Mikroorganismen und wenigstens eines, in der Dispersion enthaltenen Photosensibilisators mit elektromagnetischer Strahlung geeigneter Wellenlänge und Energiedichte.

Die Erfindung wird nachfolgend durch Figuren und Beispiele erläutert, ohne hierauf beschränkt zu sein.

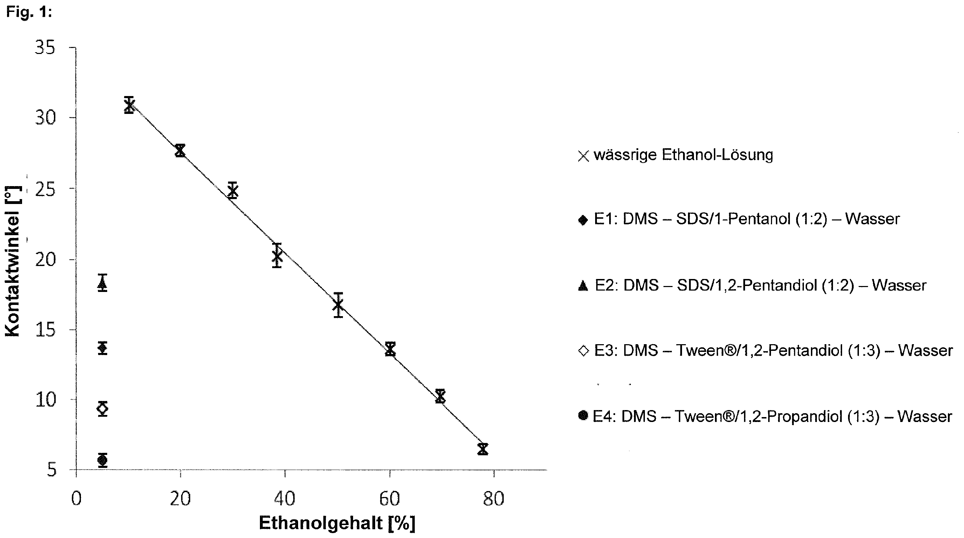

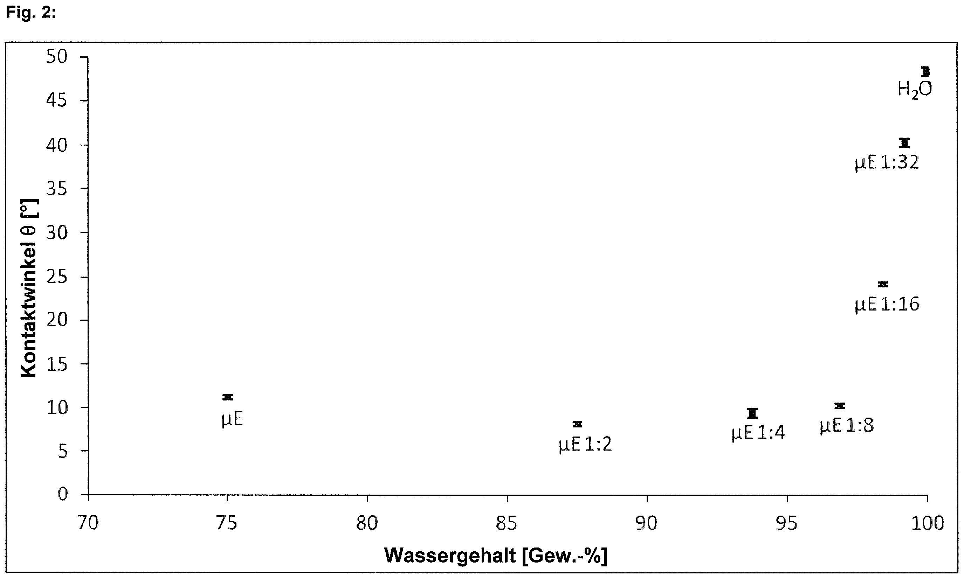

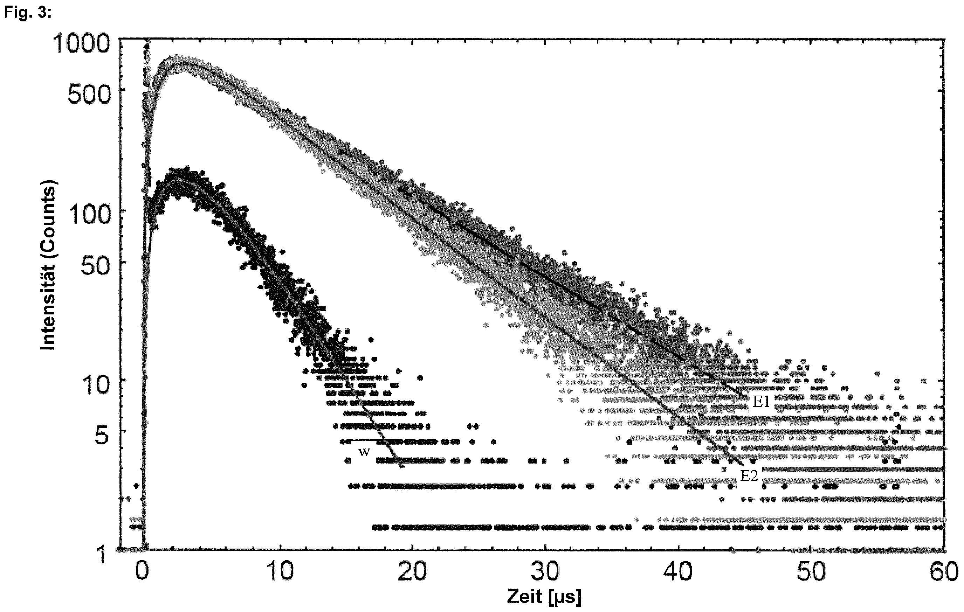

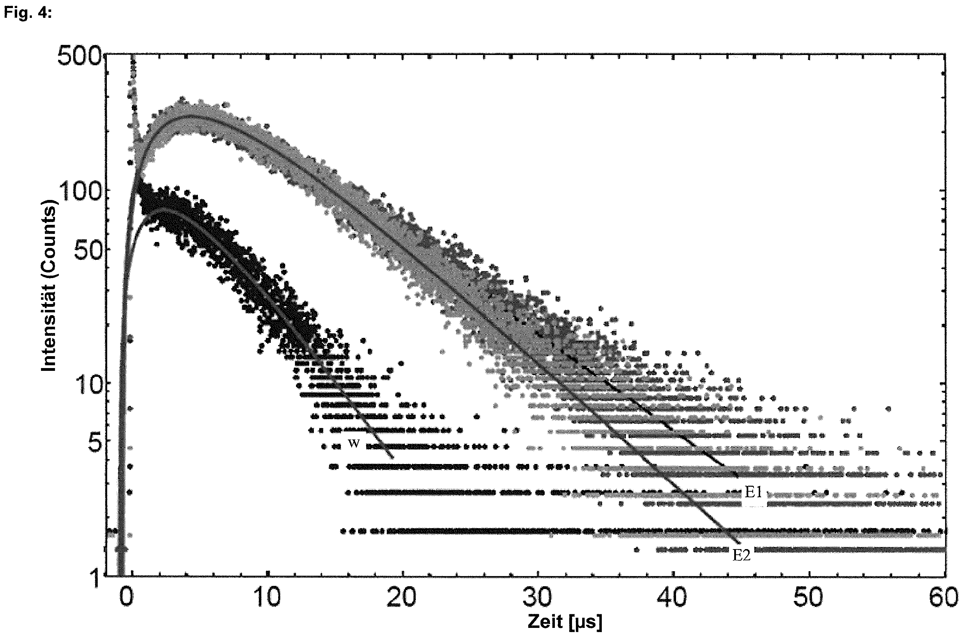

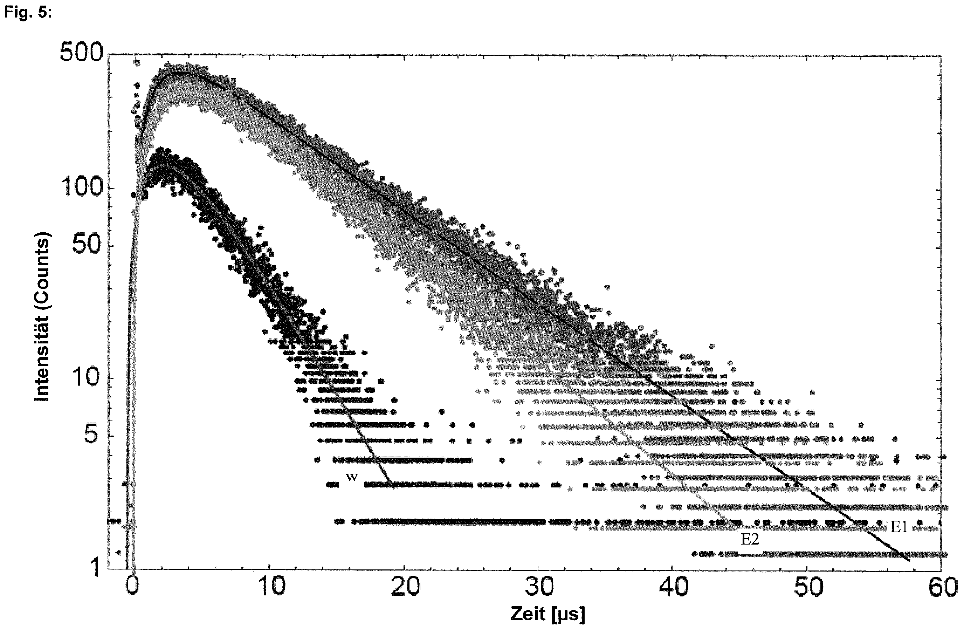

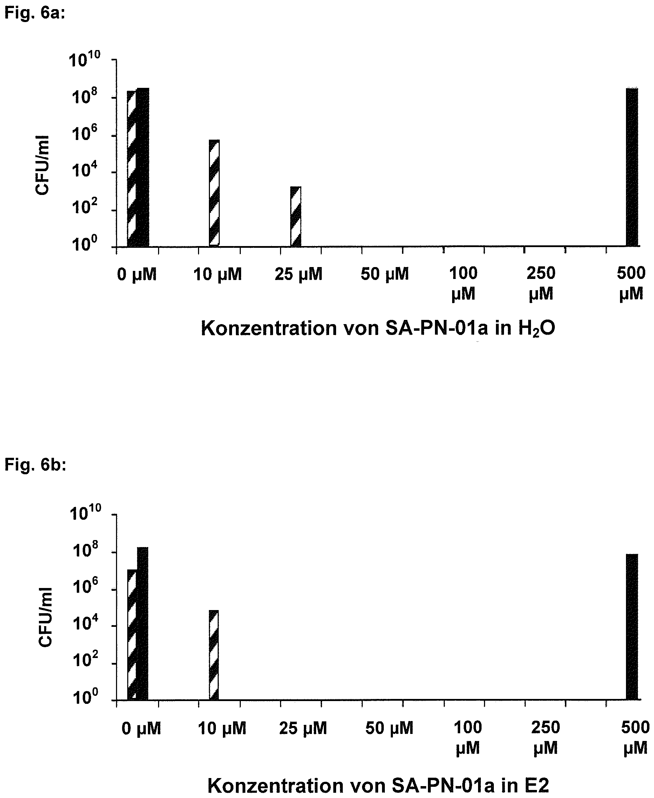

Figur 1 zeigt die Mittelwerte der in Beispiel 1 gemessenen Kontaktwinkel der Photosensibilisator-freien Mikroemulsionen E1 bis E4 sowie wässriger Ethanollösungen mit den angegebenen Konzentrationen. Figur 2 zeigt die Mittelwerte der in Beispiel 1 gemessenen gemessenen Kontaktwinkel der Verdünnung einer Mikroemulsion E3 (DMS; TWEEN^{®} 20/1,2-Pentandiol (1:3); Wasser) mit Wasser. Figur 3 zeigt die in Beispiel 1 gemessenen Zeit-aufgelösten Singulett-Sauerstoff Spektren für den Photosensibilisator TMPyP in Wasser (w), Mikroemulsion E1 (E1) bzw. Mikroemulsion E2 (E2). Figur 4 zeigt die in Beispiel 1 gemessenen Zeit-aufgelösten Singulett-Sauerstoff Spektren für den Photosensibilisator SA-PN-01a in Wasser (w), Mikroemulsion E1 (E1) bzw. Mikroemulsion E2 (E2). Figur 5 zeigt die in Beispiel 1 gemessenen Zeit-aufgelösten Singulett-Sauerstoff Spektren für den Photosensibilisator FL-AS-H-1a in Wasser (w), Mikroemulsion E1 (E1) bzw. Mikroemulsion E2 (E2). Figur 6a zeigt die in Beispiel 1 gemessenen Ergebnisse der Phototoxizitätstests für den Photosensibilisator SA-PN-01a in Wasser in den angegebenen Konzentrationen.

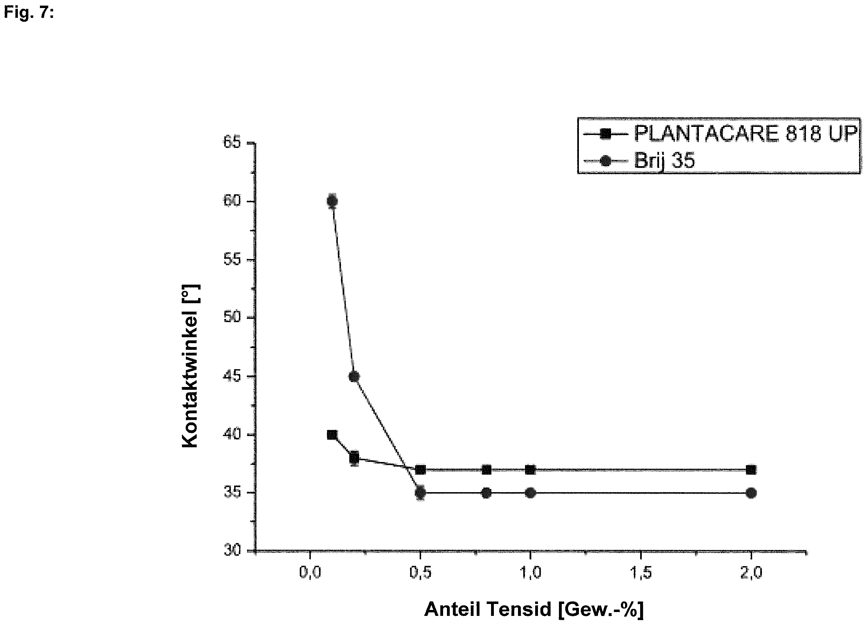

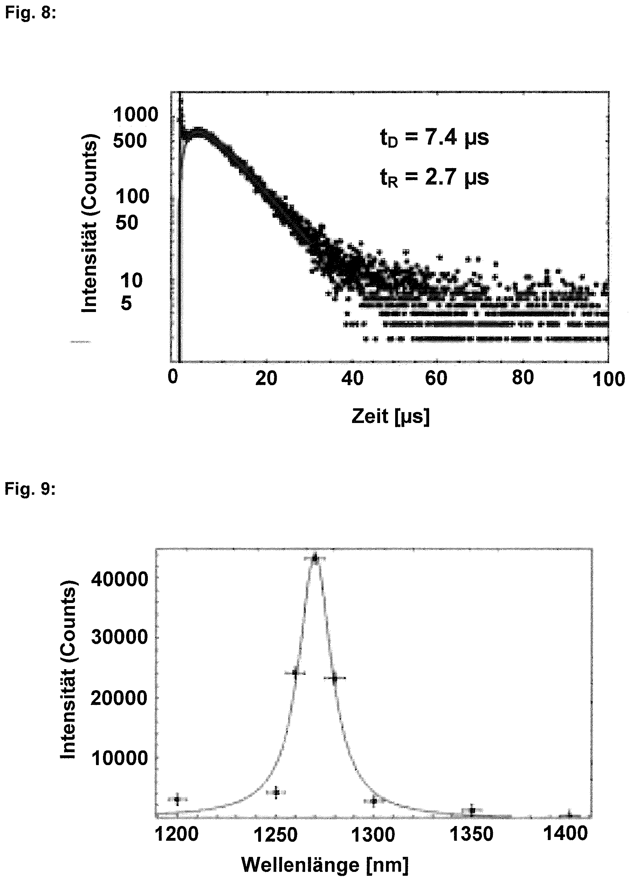

Figur 6b zeigt die in Beispiel 1 gemessenen Ergebnisse der Phototoxizitätstests für den Photosensibilisator SA-PN-01a in Mikroemulsion E2 (E2) in den angegebenen Konzentrationen. Figur 7 zeigt die Mittelwerte der in Beispiel 3 gemessenen Kontaktwinkel der Photosensibilisator-freien Gele G2 und G3, bei denen die entsprechende Menge des angegebenen Tensids hinzugegeben wurde. Figur 8 zeigt das in Beispiel 3 gemessenen Zeit-aufgelöste Singulett-Sauerstoff Spektrum für den Photosensibilisator TMPyP in Gel G3. Figur 9 zeigt das in Beispiel 3 gemessenen Wellenlängen-aufgelöste Singulett-Sauerstoff Spektrum für den Photosensibilisator TMPyP in Gel G3.

### Beispiele

Alle verwendeten Chemikalien wurden von gängigen Anbietern käuflich erworben (TCI, ABCR, Acros, Merck und Fluka) und ohne weitere Reinigung eingesetzt. Lösemittel wurden vor Gebrauch destilliert und bei Bedarf auf übliche Art und Weise getrocknet. Trockenes DMF wurde bei Fluka (Taufkirchen, DE) käuflich erworben. Dünnschichtchromatographien wurden auf Dünnschicht-Aluminiumfolien, beschichtet mit Kieselgel 60 F254, der Firma Merck (Darmstadt, DE) durchgeführt. Präparative Dünnschichtchromatographien wurden auf kommerziell erhältlichen, mit Kieselgel 60 beschichteten Glasplatten (20cm x 20 cm, Carl Roth GmbH & Co. KG, Karlsruhe, DE)) durchgeführt. Die Verbindungen wurden durch UV-Licht (λ = 254 nm, 333 nm) und teilweise mit bloßem Auge detektiert oder mit Ninhydrin angefärbt. Chromatographien wurden mit Kieselgel (0.060 - 0.200) der Firma Acros (Waltham, US) durchgeführt. NMR-Spektren wurden auf einem Bruker-Spektrometer Avance 300 (300 MHz [¹H-NMR], 75 MHz [¹³C-NMR]) (Bruker Corporation, Billerica, US) gemessen. Alle chemischen Verschiebungen sind in δ [ppm] relativ zum externen Standard (Tetramethylsilan, TMS) angegeben. Die Kopplungskonstanten sind jeweils in Hz angegeben; Charakterisierung der Signale: s = Singulett, d = Dublett, t = Triplett, m = Multiplett, dd = Dublett vom Dublett, br = breit. Die Integration bestimmt die relative Anzahl an Atomen. Die eindeutige Bestimmung der Signale in den Kohlenstoffspektren erfolgte mittels der DEPT-Methode (Pulswinkel: 135°). Fehlergrenzen: 0.01 ppm für 1 H-NMR, 0.1 ppm für 13C-NMR und 0.1 Hz für Kopplungskonstanten. Das verwendete Lösemittel ist jeweils für jedes Spektrum aufgeführt. Die IR-Spektren wurden an einem Biorad Spektrometer Excalibur FTS 3000 (Bio-Rad Laboratories GmbH, München, DE) aufgenommen. ES-MS wurden mit einem ThermoQuest Finnigan TSQ 7000 Spektrometer gemessen, sämtliche HR-MS auf einem ThermoQuest Finnigan MAT 95 (jeweils Thermo Fisher Scientific Inc, Waltham, US) Spektrometer ermittelt, Argon diente als Ionisierungsgas für FAB-Ionisierung (Fast Atom Bombardement). Schmelzpunkte wurden mit Hilfe des Schmelzpunktmessgerätes Büchi SMP-20 (Büchi Labortechnik GmbH, Essen, DE) unter Verwendung einer Glaskapillare bestimmt. Sämtliche UV/Vis-Spektren wurden mit einem Varian Cary 50 Bio UV/VIS Spektrometer, Fluoreszenzspektren mit einem Varian Cary Eclipse Spektrometer aufgenommen. Die Lösungsmittel für Absorbtions- und Emissionsmessungen wurden in spezieller spektroskopischer Reinheit von Acros oder Baker bzw. Uvasol von Merck gekauft. Milliporewasser (18 MΩ, Milli QPlus) wurde für alle Messungen verwendet.

In den nachfolgenden Beispielen wurden folgende Photosensibilisatoren verwendet:
1.) 5,10,15,20-tetrakis(1-Methyl-4-pyridyl)-porphyrin-tetra-(p-Toluensulfonat) (TMPyP, M = 1363.65 g/mol) 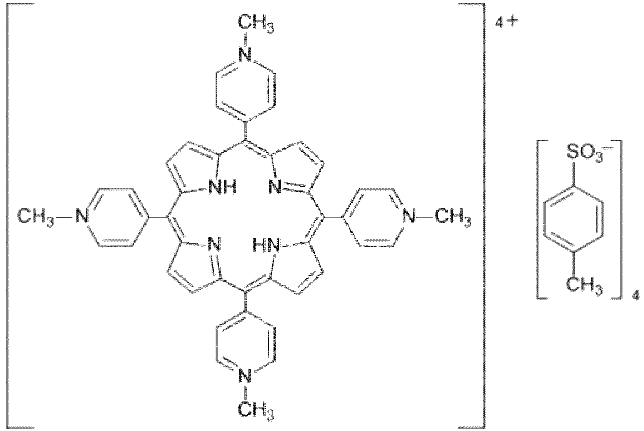 TMPyP wurde kommerziell von der TCI Deutschland GmbH (Eschborn, DE) bezogen.
2.) 2-(4-Pyridinyl)methyl)-1 H-phenalen-1 -on-chlorid (SA-PN-01a, M = 307.78 g/mol), Chlorid der Verbindung mit der Formel (24) 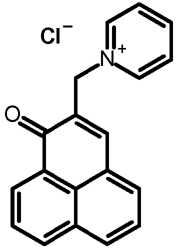 SA-PN-01 a wurde gemäß der in EP 2 678 035 A2, Beispiel 7) beschriebenen Synthese hergestellt. Das ¹H-NMR-Spektrum in DMSO-d6 war identisch zu dem aus der Literatur bekannten Spektrum.
3a.) 10-[2-({[(tert-Butyl)oxy]carbonyl}amino)eth-1-yl]-7,8-dimethyl-[3H,10H]-benzo[g]pteridine-2,4-dion (Flavin **32a**) 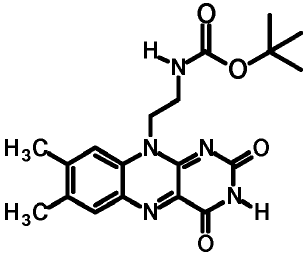 Die Synthese erfolgte gemäß der in Butenandt, J. et al. (2002) publizierten Vorschrift aus kommerziell erhältlichen Vorstufen. Das ¹H-NMR-Spektrum in DMSO-d6 war identisch zu dem aus der Literatur bekannten Spektrum.
3b.) 10-(2-Aminoeth-1-yl)-7,8-dimethyl-[3H,10H]-benzo[g]pteridine-2,4-dion Hydrochlorid (FL-AS-H-1 a; M = 321.77 g/mol),
   Chlorid der Verbindung mit der Formel (32) 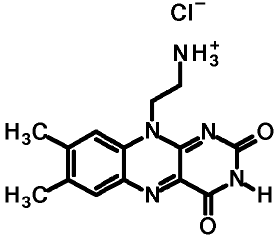 Flavin 32a (2.0 mmol) wurde in Dichlormethan (100 mL) gelöst, HCl in Diethylether (10 mL) wurde zugetropft und die Reaktionsmischung über Nacht im Dunklen unter Feuchtigkeitsausschluss gerührt. Der Niederschlag wurde abgesaugt, mit Diethylether gewaschen und getrocknet. Das ¹H-NMR-Spektrum in DMSO-d6 war identisch zu dem aus der Literatur bekannten Spektrum
4a) 3,10-Bis[2'-(tert-butyloxycarbonylamino)eth-1'-yl]7,8-dimethylbenzo[g]-pteridine-2,4-dion (Flavin **64a**) 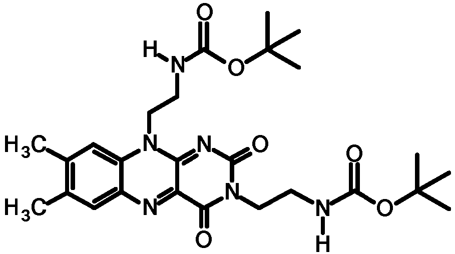 Die Synthese von Flavin **64a** erfolgte gemäß der in Svoboda J. et al. (2008) publizierten Vorschrift aus Flavin 32a. Das ¹H-NMR-Spektrum in DMSO-d6 war identisch zu dem aus der Literatur bekannten Spektrum.
4b) 3,10-Bis(2'-aminoeth-1'-yl)-7,8-dimethylbenzo[g]pteridine-2,4-dion-dihydrochlorid (FL-AS-H-2; M = 401.29 g/mol),
   Dichlorid der Verbindung (64) 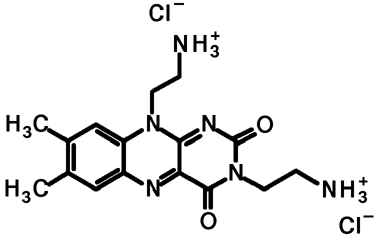 Flavin 64a (2.0 mmol) wurde in Dichlormethan (100 mL) gelöst, HCl in Diethylether (10 mL) wurde zugetropft und die Reaktionsmischung über Nacht im Dunklen unter Feuchtigkeitsausschluss gerührt. Der Niederschlag wurde abgesaugt, mit Diethylether gewaschen und getrocknet. Das ¹H-NMR-Spektrum in DMSO-d6 war identisch zu dem aus der Literatur bekannten Spektrum.
5 Synthese der Verbindungen mit der Formel (26), (27), (28a) und (28): 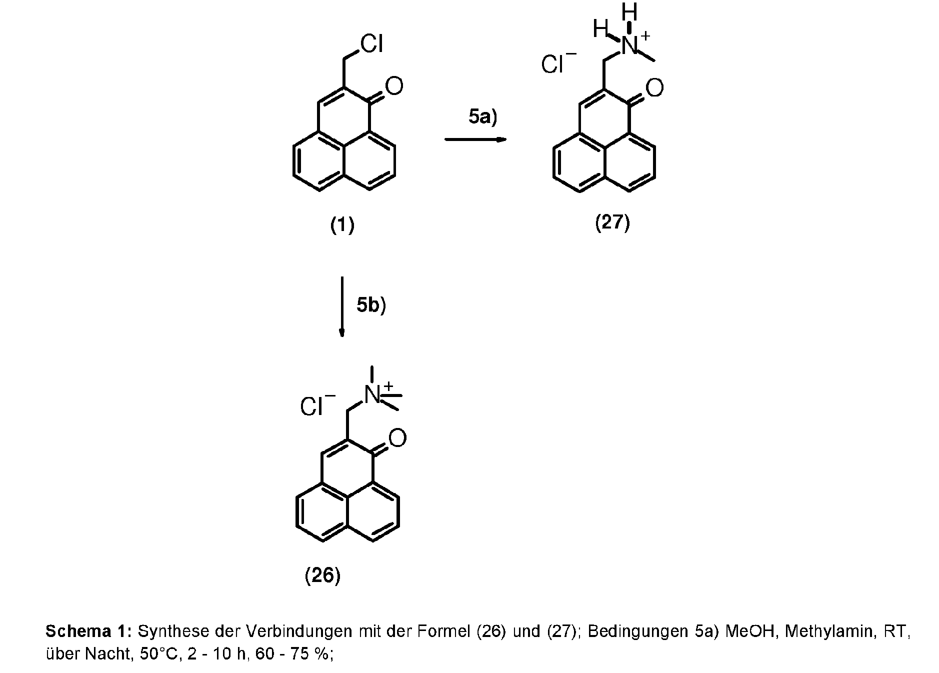
5b) Trimethylamin, Ethanol, RT, über Nacht, 50°C, 5 h, 83 %;

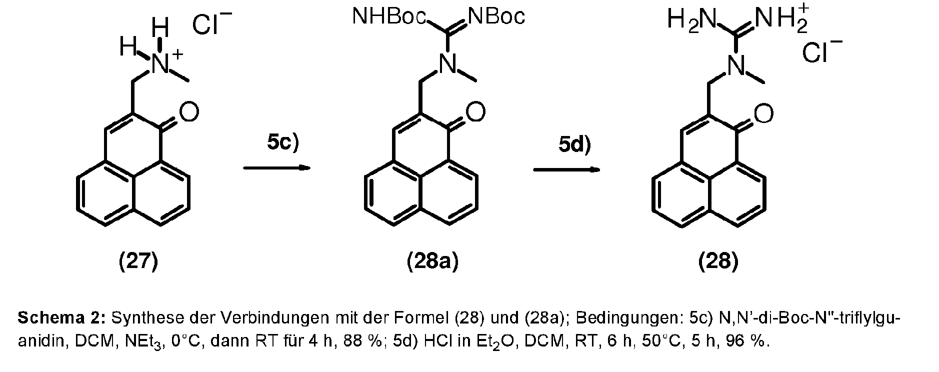

### 5a) N-Methyl-N-(1-oxo-1 H-phenalen-2-yl)methanaminium-chlorid

### Chlorid der Verbindung mit der Formel (27)

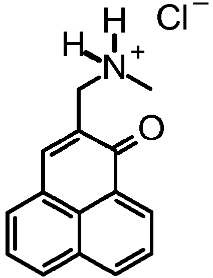

Zu 2-Chloromethyl-1H-phenalen-1-one (1) (113 mg, 0,5 mmol) in Methanol (10 mL) wurde eine eiskalte Lösung von Methylamine in Methanol (40 mL, 10 %) über 1h zugetropft. Nach 30h Rühren bei Raumtemperatur wurden das überschüssige Amin und das Lösungsmittel im Stickstoffstrom abgetrieben. Der Rückstand wurde in Dichlormethan (DCM) / Ethanol 4:1 gelöst und durch Zugabe von Diethylether gefällt. Das Produkt wurde abzentrifugiert (60 min, 4400 rpm, 0°C) und der Überstand verworfen. Dieser Schritt wurde einmal wiederholt. Der Rückstand wurde in Diethylether suspendiert. Nach Absetzen des gelben Feststoffes wurde der Überstand abdekantiert und verworfen. Dieser Schritt wurde zweimal wiederholt. Das Produkt (101 mg, 0,39 mmol).war ein gelblich-braunes Pulve.
***¹H-NMR** (300 MHz, CDCl₃): δ[ppm] = 8.66 (d, J = 7.4 Hz, 1H), 8.28 - 8.20 (m, 2H), 8.08 (d, J= 8.3 Hz, 1H), 7.94 (d, J = 7.0 Hz, 1H), 7.80 (t, J = 7.7 Hz, 1H), 7.67 - 7.59 (m, 1H), 4.20 (s, 2H), 2.79 (s, 3H).* - ***MS** (ESI-MS, CH₂Cl₂*/*MeOH* + *10 mmol NH₄OAc): e*/*z* (%) = *224.1 (MH⁺, 100%);- Molekulargewicht (**MW**) = 224.28 + 35.45 g*/*mol;- Summenformel*
*(**MF**) = C₁₅H₁₄NOCl.*

### 5b) N,N,N-trimethyl-1-(1-oxo-1H-phenalen-2-yl)methanaminium-chlorid (SA-PN-02a)

### Chlorid der Verbindung mit der Formel (26)

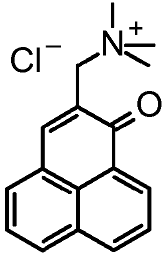

In einem Schlenck-Kolben wurde 2-(chloromethyl)-1 H-phenalen-1-on (1) (230 mg, 1 mmol) in Ethanol (60 mL) vorgelegt. Trimethylamin in Ethanol (5 mL, 5,6 M, 23 mmol) wurde mittels Spritze über das Septum zugegeben. Die Lösung wurde über Nacht im Dunklen gerührt. Anschließend wurde bei 50°C für 30 h weitergerührt. Das Lösungsmittelvolumen wurde auf 3 mL eingeengt. Diethylether (50 mL) wurde zugegeben um das Produkt vollständig zu fällen. Das Produkt wurde abzentrifugiert (60 min, 4400 rpm, 0°C) und der Überstand verworfen. Der Rückstand wurde in Diethylether suspendiert. Nach Absetzen des gelben Feststoffes wurde der Überstand abdekantiert und verworfen. Dieser Schritt wurde zweimal wiederholt. Der Feststoff wurde bei vermindertem Druck getrocknet und ein gelbes Pulver erhalten (210 mg, 0,73 mmol).
***¹H-NMR** (600 MHz, D₂O): δ[ppm] = 8.02 (d,J= 8 Hz, 1H), 7.97 (d,J= 6.3 Hz, 1H), 7.92 (d, J = 8.2 Hz, 1H), 7.77 (s, 1H), 7.62 (d, J = 7 Hz, 1H), 7.50 (t,J= 7.8 Hz, 1H) 7.45 (t, J = 7.8 Hz, 1H), 4.12 (s, 2H), 2.98 (s, 9H).- **MS** (ESI-MS, CH₂Cl₂*/*MeOH* + *10 mmol NH₄OAc): e*/*z (%) = 252.1 (100, M+); - **MW** = 287.79g*/*mol; - **MF =** G₁₇H18NOGl;*

### 5c) 1-((1-oxo-1H-phenalen-2-yl)methyl)-1-methyl-2,3-di(tert-butoxycarbonyl)guanidin Verbindung mit der Formel (28a)

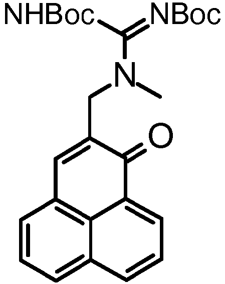

In einem trockenen 25 mL Rundkolben wurde N,N'-di-Boc-N"-triflylguanidine (0,41 g, 1,05 mmol) in Dichloromethan (10 mL) vorgelegt. Triethylamin (0,3 g, 0,39 mL, 3 mmol) wurde langsam bei 2-5°C unter Feuchtigkeitsausschluss zugegeben. Verbindung 3 (130 mg, 0.5 mmol) wurde in einer Portion zugegeben. Nach 5 h Rühren bei Raumtemperaturwurde mit Dichlormethan (30 mL) verdünnt und die Lösung in einen Scheidetrichter überführt. Die organische Phase wurde mit wässrigem Kaliumhydrogensulfat (10 mL, 5%), gesättigter Natriumbicarbonat Lösung (10 mL) und gesättigter Kochsalzlösung (20 mL) gewaschen, über MgSO₄ getrocknet, gefiltert und einrotiert. Das Rohprodukt wurde durch Säulenchromatographie mit Acetone/Petrolether 1:2 gereinigt und lieferte das Produkt als gelben Feststoff (0.21 g). Für weitere Reinigung wurde das Material in Aceton (1 mL) gelöst und mit Petrolether (14 mL) gefällt. Der Niederschlag wurde abgesaugt und mit Petrolether gewaschen.
***¹H-NMR** (300 MHz, CDCl₃): δ[ppm] = 8.63 (d, J =* 7.3 *Hz, 1H), 8.21 (d, J =* 7.9 *Hz, 1H), 8.03 (d, J = 8.2 Hz, 1H), 7.85 - 7.70 (m, 3H),* 7.67 - 7.54 *(m, 1H),* 4.59 *(s, 2H), 3.01 (s, 3H), 1.50 (s, 9H), 1.48 (s, 9H).* - **MS** *(ESI-MS, CH₂Cl₂*/*MeOH* + *10 mmol NH₄OAc): e*/*z* (%) = 466.1 *(MH⁺, 100%); - **MW** =* 465.53 *g*/*mo*/*; -* ***MF* =** *C₂₆H₃₁N₃O₅*

### 5d) 1-((1-oxo-1H-phenalen-2-yl)methyl)-1-methyl-guanidinium-chlorid (SA-PN-24d)

### Chlorid der Verbindung mit der Formel (28)

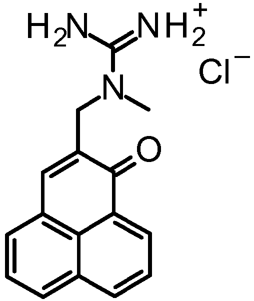

Herstellung und Aufreinigung der Verbindung wurden unter Lichtschutz ausgeführt. Verbindung 5 (200 mg, 0,45 mmol) wurde in Dichloromethan (20 mL, getrocknet über CaCl₂) vorgelegt. Eine gesättigte Lösung von HCl in Diethylether (2 mL) wurde zugetropft. Nach Rühren für 4 h bei Raumtemperatur unter Feuchtigkeitsausschluss wurde die Lösung auf zwei Blue Caps aufgeteilt und mit Diethylether auf jeweils 15 mL aufgefüllt. Das Produkt wurde abzentrifugiert (60 min, 4400 rpm, 0°C) und der Überstand verworfen. Der Rückstand wurde in Diethylether suspendiert. Nach Absetzen des gelben Feststoffes wurde der Überstand abdekantiert und verworfen. Dieser Schritt wurde zweimal wiederholt. Anschließend wurde das Produkt bei vermindertem Druck getrocknet um 130 mg eines gelben Pulvers zu erhalten.
**¹H-NMR** (300 MHz, DMSO-d6): δ[ppm] = 8.60 - 8.47 (m, 4H), 8.33 - 8.24 (m, 2H), 8.16 - 8.09 (m, 2H), 7.98 - 7.89 (m, 2H), 7.84 - 7.73 (m, 4H), 7.57 - 7.48 (m, 7H), 4.55 - 4.42 (m, 4H), 3.05 (s, 6H). - **MS** (ESI-MS, CH₂Cl₂/MeOH + 10 mmol *NH₄OAc):* e/z (%) = 266.1 (MH⁺, 100%); - **MW =** 266.3 + 35.45 *=* 301.75 g/mol; - **MF** = C₁₆H₁₆N₃OCl

### Beispiel 1:

### A) Herstellung verschiedener Wasser-haltiger Mikroemulsionen

Die nachfolgend angegebenen Gew.-% der Bestandteile der Mikroemulsionen E1 bis E4 beziehen sich jeweils auf das Gesamtgewicht der entsprechenden Mikroemulsion ohne Photosensibilisator.

Mikroemulsion E1: Mikroemulsion bestehend aus DMS, SDS und 1-Pentanol mit einem konstanten Massenverhältnis SDS zu 1-Pentanol von 1:2 sowie Wasser.
20,0 Gew.-% Dimethylsuccinat (DMS)
8,33 Gew.-% Natriumdodecylsulfat (SDS)
16,67 Gew.-% 1-Pentanol
55,0 Gew.-% Wasser

Mikroemulsion E2: Mikroemulsion bestehend aus DMS, SDS und 1,2-Pentandiol mit einem konstanten Massenverhältnis von 1:2 SDS zu 1,2-Pentandiol sowie Wasser.
20,0 Gew.-% Dimethylsuccinat (DMS)
8,33 Gew.-% Natriumdodecylsulfat (SDS)
16,67 Gew.-% 1,2-Pentandiol
55,0 Gew.-% Wasser

Mikroemulsion E3: Mikroemulsion bestehend aus DMS, TWEEN^{®}20 und 1,2-Pentandiolmit einem konstanten Massenverhältnis TWEEN^{®} 20 zu 1,2-Pentandiol von 1:3 sowie Wasser.
10,0 Gew.-% Dimethylsuccinat (DMS)
3,75 Gew.-% TWEEN^{®} 20
11,25 Gew.-% 1,2-Pentandiol
75,0 Gew.-% Wasser

Mikroemulsion E4: Mikroemulsion bestehend aus DMS, TWEEN^{®}20 und 1,2-Propandiol mit einem konstanten Massenverhältnis TWEEN^{®} 20 zu 1,2-Propandiol von 1:3 sowie Wasser
10,0 Gew.-% Dimethylsuccinat (DMS)
3,75 Gew.-% TWEEN^{®} 20
11,25 Gew.-% 1,2-Propandiol
75,0 Gew.-% Wasser

Die entsprechenden Mikroemulsionen E1 bis E4 wurden zunächst ohne Photosensibilisator hergestellt wobei alle Komponenten außer Wasser eingewogen wurden und nacheinander vermischt wurden. Nachdem eine homogene Mischung erhalten wurde, erfolgte die Zugabe der entsprechenden Menge Wasser unter ständigem Rühren.

Beispielsweise erfolgt die Herstellung von 100 g Mikroemulsion E4 durch Einwaage von 3.75g TWEEN^{®} 20, 11.25 g 1,2-Propandiol und 10 g DMS. Die resultierende Lösung wurde bis zum Erhalt einer homogenen Mischung gerührt. Anschließend wurden 75 g Wasser unter Rühren hinzugegeben.

Für die weiteren Experimente wurden die Photosensibilisatoren in der entsprechenden Konzentration in der jeweiligen Mikroemulsion gelöst und solange gerührt, bis der Photosensibilisator vollständig gelöst war.

### B) Kontaktwinkel-Prüfung

Die Benetzung von Oberflächen durch die verwendeten Mikroemulsionen wurde mit Hilfe der Kontaktwinkel-Prüfung bestimmt.

Für die Kontaktwinkel-Prüfung wurden die oben angegebenen Emulsionen ohne Photosensibilisator sowie Photosensibilisator-haltige Emulsionen, die die Photosensibilisatoren TMPyP, SA-PN-01a, SA-PN-02a, SA-PN-24d, FL-AS-H-1a oder FL-AS-H-2 enthielten, verwendet.

Um die neuen Dispersionen mit konventionellen, Alkohol-haltigen Desinfektionslösungen zu vergleichen, wurden weiterhin wässrige Ethanol-Lösungen mit verschiedenen Ethanol-Konzentrationen aus einem Bereich von 10 Gew.-% Ethanol bis 90 Gew.-% Ethanol als Vergleichslösungen verwendet.

Darüber hinaus wurden Verdünnungen der oben angegebenen Emulsionen ohne Photosensibilisator sowie Photosensibilisator-haltigen Emulsionen verwendet, bei denen die entsprechende Mikroemulsion in 5 Schritten bis zu einem Wassergehalt von 99 Gew.-% verdünnt wurde.

Der Kontaktwinkel wurde mit Hilfe des Kontaktwinkelmessgerätes DataPhysics OCA 35 der Firma DataPhysics Instruments GmbH (Filderstadt, DE) gemäß Herstellerangaben bestimmt.

Für die Messung wurde jeweils 2.5 µL jeder zu testenden Lösung bei Raumtemperatur unter Vollklimatisierung (Temperatur: 25°C, Druck: 1013 mbar, relative Luftfeuchtigkeit: 50 %) mit einer automatischen Hamilton-Spritze auf einen Glasobjektträger als Prüfoberfläche in Form eines Tropfens aufgebracht und im zeitlichen Abstand von einer Sekunde fotografiert.

Anschließend wurde für jede Aufnahme sowohl der linke als auch der rechte Kontaktwinkel zwischen Tropfen und Prüfoberfläche mit Hilfe der Software SCA 20 der Firma DataPhysics Instruments GmbH ermittelt sowie der Mittelwert zwischen den gemessenen Kontaktwinkeln berechnet. Jede Messung wurde jeweils 4-mal wiederholt.

Figur 1 zeigt die Mittelwerte der gemessenen Kontaktwinkel für wässrige Ethanol-Lösungen mit verschiedenen Ethanol-Konzentrationen aus einem Bereich von 10 Gew.-% Ethanol bis 80 Gew.-% Ethanol.

Exemplarisch sind in Figur 1 weiterhin die Mittelwerte der gemessenen Kontaktwinkel der Photosensibilisator-freien Mikroemulsionen E1 bis E4 eingezeichnet.

Die Mittelwerte der gemessenen Kontaktwinkel von Mikroemulsionen E1 bis E4, die jeweils 100 µm eines der verwendeten Photosensibilisatoren enthielten, wichen nur unwesentlich von den gemessenen Kontaktwinkeln der Photosensibilisator-freien Mikroemulsionen E1 bis E4 ab.

Die verschiedenenMikroemulsionen mit SDS und TWEEN^{®} 20 zeigten einen signifikant reduzierten Kontaktwinkel im Vergleich zu reinem Wasser. Mehr als 40 Gew.-% Ethanol müssten verwendet werden, um eine vergleichbare Benetzung der verwendeten Glasoberfäche zu erreichen.

Der Effekt der Verdünnung einer Mikroemulsion mit Wasser ist exemplarisch in Figur 2 an der verwendeten Photosensibilisator-freien Mikroemulsion E3 (DMS; TWEEN^{®} 20/1,2-Pentandiol (1:3); Wasser) dargestellt.

Wie in Figur 2 zu sehen ist, kann Mikroemulsion E3 mit etwa der 8-fachen Menge Wasser verdünnt werden, ohne dass der Kontaktwinkel der erhaltenen Verdünnung im oben beschriebenen Test signifikant ansteigt. Selbst eine 16-fache Verdünnung zeigt noch eine ausreichende Benetzung der im Test verwendeten Glasplatte.

Ähnliche Ergebnisse wurden für die Mikroemulsionen E1, E2 und E4 sowie für Mikroemulsionen E1 bis E4, die jeweils 5 µM eines der verwendeten Photosensibilisatoren TMPyP, SA-PN-01a, SA-PN-02a, SA-PN-24d, FL-AS-H-1a oder FL-AS-H-2 aufwiesen, erhalten.

### C) UV/VIS-Messungen

Die Absorption der verwendeten Photosensibilisatoren TMPyP, SA-PN-01a und FL-AS-H-1a in den jeweiligen Mikroemulsionen E1 bis E4 wurde durch Aufnahme eines Absorptions-Spektrums für einen Wellenlängebereich von 250nm bis 600 nm bestimmt.

Dazu wurden die Photosensibilisatoren SA-PN-01a und FL-AS-H-1a in einer Konzentration von 20 µM in Wasser und in der jeweiligen Mikroemulsion E1 bis E4 gelöst.

Wegen der höheren Absorption von TMPyP in Lösung wurde der Photosensibilisator TMPyP jeweils in einer Konzentration von 5 µM verwendet.

Absorptionsspektren wurden mit einem Varian Cary BIO UV/VIS/IR Spektrometer (Agilent Technologies Inc., Santa Clara, CA, USA) gemessen wobei eine 10 mm Hellma Quartz-Küvette (SUPRASIL, Type 101-QS, Hellma GmbH & Co. KG, Mühlheim, DE) verwendet wurde.

Die jeweils gemessenen Absorptionsspektren von TMPyP, SA-PN-01 a und FL-AS-H-1 a in den Mikroemulsionen E1 bis E4 sind innerhalb der Fehlergrenzen nahezu identisch zum entsprechenden Absorptionsspektren von TMPyP, SA-PN-01a und FL-AS-H-1a in Wasser.

Es gibt keine Unterschiede hinsichtlich der Intensität der Signale oder Veränderungen des Spektrums.

### D) Bestimmung von nach Bestrahlung gebildetem Singulett-Sauerstoff

Die Bildung von Singulett-Sauerstoff nach Bestrahlung einer Photosensibilisator-haltigen Mikroemulsion wurde durch Zeit-aufgelöste Singulett-Sauerstoff Lumineszenzenzmessungen bestimmt.

Für die entsprechenden Messungen wurden jeweils 5 µM der verwendeten Photosensibilisatoren in Wasser oder den Emulsionen E1 bis E4 gelöst.

Die Zeit-aufgelöste Singulett-Sauerstoff Lumineszenzenzmessungen wurde gemäß der in S. Y. Egorov et al. 1999 beschriebenen Methode durchgeführt
Zur Erzeugung von Singulett-Sauerstoff wurde ein stimmbares Lasersystem (Model: NT242-SH/SFG, Seriennummer: PGD048) der Firma EKSPLA (Vilnius, Lettland) verwendet. Ein Teil des erzeugten monochromatischen Laserstrahls wurde auf eine Photodiode gerichtet, die als Trigger-Signal für die Zeit-korrelierte Einzel-Photon-Messung diente.

Der andere Teil des Laserstrahls wurde auf eine 1 cm dicke Quartz-Küvette (SUPRASIL, Type 101-QS, Hellma GmbH & Co. KG, Mühlheim, DE), in der die zu untersuchende Lösung angeordnet war.

Die Bildung von Singulett-Sauerstoff wurde durch eine direkte Detektion der Zeit- und spektral-aufgelösten Singulett-Sauerstoff Lumineszenz nachgewiesen
Eine Singulett-Sauerstoff Lumineszenz wurde mittels eines Stickstoff-gekühlten Photomultipliers (Model R5509-42, Hamamatsu Photonics, Hamamatsu, Japan) und eines Multiscalers (7886S, FAST Com Tec GmbH, Oberhaching, Deutschland) durchgeführt.

Die Singulett-Sauerstoff Lumineszenz wurde bei einer Wellenlänge aus einem Bereich von 1200 nm bis 1400 nm unter Verwendung von Interferenz-Filtern, die vor dem Photomultiplier angeordnet waren, nachgewiesen.

Die Zeit-aufgelösten Singulett-Sauerstoff Spektren sind exemplarisch für die jeweiligen Photosensibilisatoren TMPyP, SA-PN-01a und FL-AS-H-1a in den Figuren 3 bis 5 dargestellt.

Eine Zusammenfassung des Singulett-Sauerstoff Nachweises ist in Tabelle 1 dargestellt.

Jeder der verwendeten Photosensibilisatoren TMPyP, SA-PN-01 a und FL-AS-H-1 a erzeugt nach Bestrahlung mit elektromagnetischer Strahlung Singulett-Sauerstoff.

Der gebildete Singulett-Sauerstoff weist in der jeweiligen Mikroemulsion eine signifikant längere Halbwertszeit auf im Vergleich zu Wasser. Die Mikroemulsion verlängert die Halbwertszeit des gebildeten Singulett-Sauerstoffs etwa um das Doppelte im Vergleich zur Halbwertszeit des gebildeten Singulett-Sauerstoffs in Wasser, die etwa 3,5 µs beträgt.

Die Quantenausbeute an Singulett-Sauerstoff ist in den Mikroemulsionen wenigstens 2-mal so hoch im Vergleich zu Wasser.

Die Bildung von Singulett-Sauerstoff in den verwendeten Mikroemulsionen mit FL-AS-H-1a ist 5-mal und mit SA-PN-01a sogar 7-mal höher als in Wasser.

**Tabelle 1: Ergebnisse der Singulett-Sauerstoff-Messungen für die Photosensibilisatoren TMPyP, SA-PN-01 a und TMPyP in Wasser, Mikroemulsion E1 (DMS; SDS/1-Pentanol (1:2); Wasser) und Mikroemulsion E2 (DMS; SDS/1,2-Pentandiol (1:2); Wasser).**

| **Photosensi bi lisator** | **Lösungsmittel** | **Bildungszeit (µs)** | **Abklingzeit (µs)** | **Integral** |
|---|---|---|---|---|
| TMPyP | H₂O | 1.9 | 3.8 | 985 |
| TMPyP | E1 | 1.7 | 8.9 | 2874 |
| TMPyP | E2 | 2.3 | 7.2 | 1955 |
| | | | | |
| SA-PN-01a | H₂O | 2.5 | 3.2 | 571 |
| SA-PN-01a | E1 | 1.0 | 9.2 | 4413 |
| SA-PN-01a | E2 | 1.6 | 7.4 | 3933 |
| | | | | |
| FL-AS-H-1a | H₂O | 3.6 | 3.6 | 366 |
| FL-AS-H-1a | E1 | 3.0 | 8.6 | 1752 |
| FL-AS-H-1a | E2 | 4.0 | 6.7 | 1695 |

Zusammenfassend lässt sich feststellen, dass die Verwendung einer Mikroemulsion die Photophysik des verwendeten Photosensibilisators positiv beeinflusst.

Signifikant größere Mengen an Singulett-Sauerstoff wurden in einer der verwendeten Mikroemulsionen gebildet und die Lichtabsorption des jeweils verwendeten Photosensibilisators in der Mikroemulsion blieb im Wesentlichen unverändert.

### E) Phototoxizitätsmessungen

Um die Phototoxizität der erfindungsgemäßen Mikroemulsionen zu untersuchen, wurde ein MTT-Test verwendet. Der Nachweis der Zellvitalität mittels MTT-Test beruht auf der Reduktion des gelben, wasserlöslichen Farbstoffs 3-(4,5-Dimethylthiazol-2-yl)-2,5-diphenyltetrazoliumbromid (MTT, Sigma-Aldrich Chemie GmbH, München, DE) in ein blauviolettes, wasserunlösliches 2,3,5-Triphenyltetrazoliumchlorid (Formazan). MTT ist ein membrangängiger Farbstoff, der durch mitochondriale Dehydrogenasen lebender Zellen metabolisiert wird, was schließlich zur Bildung von Formazan-Kristallen führt.

Die Formazan-Kristalle sind nicht mehr membrangängig und akkumulieren in proliferierenden, ungeschädigten Zellen. Nach Lyse der Zellen und Solubilisierung der Kristalle wurde der Farbstoff anschließend durch colorimetrische Messung bei 550 nm in einem Multiwell-Spektrophotometer (ELISA-Reader) quantifiziert. Die gebildete Formazanmenge wurde als optische Dichte (OD-Wert) erfasst. Die gemessene Menge Formazan ist direkt proportional zu der Zahl der proliferierenden Zellen, so dass dieser Test zur Messung der Phototoxizität der verwendeten Mikroemulsionen geeignet ist. Anhand einer vorher erstellten Eichkurve wurde dem gemessenen OD-Wert eine Zellzahl zugeordnet.

Die Konzentration des jeweiligen Photosensibilisators TMPyP, SA-PN-01 a, SA-PN-02a, SA-PN-24d, FL-AS-H-1a oder FL-AS-H-2 in den Mikroemulsionen E1 bis E4 betrug 0 µM, 10 µM, 25 µM, 50 µM, 100 µM, 250 µM und 500 µM.

Darüber hinaus wurden die jeweiligen Mikroemulsionen E1 bis E4 ohne Photosensibilisator als Kontrolle mitgeführt.

Die Phototoxizitätsmessungen wurden an *Escherichia coli* (*E. coli;* ATCC-Nummer: 25922) und *Staphylococcus aureus* (*S. aureus;* ATCC-Nummer: 25923) wie von Mosmann (1983) beschrieben durchgeführt. (Mosmann T.: Rapid colorimetric assay for cellular growth and survival: application to proliferation and cytotoxicity assays. in: J. Immunol. Methods. 1983 (65); Seiten 55 - 63).

25 µL einer über Nacht gewachsenen Suspension der verwendeten Bakterien in Müller-Hinton-Flüssigmedium (Merck KGaA, Darmstadt, Deutschland) mit einer optischen Dichte von 0,6 bei 600 nm wurden mit 25 µL der zu testenden Lösung bei Raumtemperatur für 10 Sekunden im Dunklen in einer 96-well Mikrotiterplatte (Cellstar, Greiner Bio-One, Frickenhausen, Deutschland) inkubiert.

Anschließend wurde die Mikrotiterplatte für 40 s bestrahlt. Zur Bestrahlung wurde die Lichtquelle Blue V der Firma Waldmann (Villingen-Schwenningen, Deutschland) genutzt, welche Licht von 380 bis 480 nm emittiert (Emissionsmaximum bei ca. 420 nm). Die applizierte Leistung betrug 20 mW/cm².

Bei jedem Experiment wurden drei Kontrollen mitgeführt, um Nebeneffekte der Bestrahlung / des Photosensibilisators (PS) auf das Überleben der Bakterien auszuschließen: (i) kein PS, nur Licht (= Lichtkontrolle), (ii) kein Licht, nur PS (= Dunkelkontrolle) und (iii) weder Licht, noch PS (= Referenzkontrolle).

Nach Abschluss der Bestrahlung wurde in jede Vertiefung der Mikrotiterplatte 75 µl einer 25 Gew.-% igen SDS-Lösung hinzugegeben und die Bakterienzellen bei 37° C über Nacht im Brutschrank lysiert.

Schließlich wurde die optische Dichte (OD) bei 550 nm mit Hilfe eines Mikrotiterplattenphotometer (Model EAR 400 AT, SLT Laborinstruments Austria, Salzburg, AT) bestimmt.

Nach Lyse der Zellen und Solubilisierung der Kristalle kann der Farbstoff anschließend durch colorimetrische Messung bei 550 nm in einem Multiwell-Spektrophotometer (ELISA-Reader) quantifiziert werden.

Die Bestimmung der koloniebildenden Einheiten (CFU) wurde gemäß der von Miles und Misra publizierten Methode durchgeführt (Miles, AA; Misra, SS, Irwin, JO (1938 Nov). "The estimation of the bactericidal power of the blood" The Journal of hygiene 38 (6): 732-49). Dazu wurden serielle Verdünnungen von 10⁻² bis 10⁻⁹ der entsprechenden Bakteriensuspension hergestellt. Je 3 x 20 µl der entsprechenden Bakterienverdünnungen wurden dann auf Müller-Hinton Platten aufgetropft und bei 37° C für 24 h inkubiert. Danach wurde die Anzahl der überlebenden koloniebildenden Einheiten (KBE bzw. CFU) bestimmt. Alle Versuche wurden jeweils dreimal wiederholt.

In einem Konzentrationsbereich von 10 µM bis 100 µM des Photosensibilisators TMPyP sowohl in Wasser als auch in einer der verwendeten Mikroemulsionen E1 bis E4 wurde E. coli und S. aureus durch den bei der Bestrahlung gebildeten Singulett-Sauerstoff zerstört.

Bei einer Konzentration von mehr als 100 µM des Photosensibilisators TMPyP in Wasser tritt eine Abschirmwirkung auf. TMPyP kann 25- bis 30-mal mehr Licht absorbieren. Daher ist die Bildung Singulett-Sauerstoff bei hohen Konzentrationen von mehr als 100 µM geringer und entsprechend konzentrierte wässrige Lösungen konnten die Menge an E. coli und S. aureus nur um 2 log-Sufen reduzieren.

Im Unterschied dazu tritt bei Verwendung von TMPyP in einer der Mikroemulsionen E1 bis E4 eine signifikant geringere Abschirmung auf. Daher ist die Menge an gebildeten Singulett-Sauerstoff bei hohen Konzentrationen von TMPyP (mehr als 100 µM bis 500 µM) höher im Vergleich zu wässrigen Lösungen.

Entsprechend konzentrierte Mikroemulsionen mit TMPyP in einer Konzentration von mehr als 100 µM bis 500 µM konnten die Menge an E. coli und S. aureus nur um 5 log₁₀-Sufen reduzieren.

Die Photosensibilisatoren SA-PN-01a, SA-PN-02a und SA-PN-24d waren gegenüber E. coli und S. aureus bei Verwendung in einer Mikroemulsion wirksamer als bei Verwendung in Wasser.

S. aureus wurde in Wasser komplett zerstört (Reduzierung der Menge nach Bestrahlung von mehr als 6 log₁₀-Sufen) bei Verwendung von SA-PN-01a, SA-PN-02a und SA-PN-24d in einer Konzentration aus einem Bereich von 50 bis 500 µM.

Bei Verwendung von SA-PN-01a in einer der Mikroemulsionen E1 bis E4 konnte bereits ab einer Konzentration von 25 µM SA-PN-01a eine Reduzierung der Menge an E. coli und S. aureus nach Bestrahlung von mehr als 6 log₁₀-Sufen erreicht werden.

Darüber hinaus ist eine Konzentration von 10 µM SA-PN-01 a in einer der Mikroemulsionen E1 bis E4 ausreichend, um nach Bestrahlung eine Reduzierung der Menge an E. coli und S. aureus von 3 log10-Sufen zu erreichen.

In den Figuren 6a und 6b ist die Wirkung von SA-PN-01a in Wasser bzw. SA-PN-01a in Mikroemulsion E2 (E2) auf Staphylococcus aureus exemplarisch dargestellt.

Figur 6a zeigt die Wirkung einer wässrigen Lösung des Photosensibilisators SA-PN-01 a in den angegebenen Konzentrationen auf Staphylococcus aureus nach einer Bestrahlung (gestrichelten Balken) mit der Lichtquelle Blue V (Bestrahlungsdauer: 40 s). Die applizierte Leistung betrug jeweils 20 mW/cm.

Als Kontrolle wurden zwei unbestrahlte Proben (schwarze Balken) mitgeführt, bei denen jeweils Staphylococcus aureus mit reinem Wasser ohne SA-PN-01 a (Konzentration: 0 µM) bzw. SA-PN-01 a in Wasser in einer Konzentration von 500 µM behandelt wurden.

Figur 6b zeigt exemplarisch die Wirkung des Photosensibilisators SA-PN-01 a in Mikroemulsion E2 in den angegebenen Konzentrationen auf Staphylococcus aureus nach einer Bestrahlung (gestrichelten Balken) mit der Lichtquelle Blue V (Bestrahlungsdauer: 40 s). Die applizierte Leistung betrug jeweils 20 mW/cm.

Als Kontrolle wurden zwei unbestrahlte Proben (schwarze Balken) mitgeführt, bei denen jeweils Staphylococcus aureus mit Mikroemulsion E2 ohne SA-PN-01 a (Konzentration: 0 µM)bzw. SA-PN-01 a in Mikroemulsion E2 in einer Konzentration von 500 µM behandelt wurden.

Dargestellt sind jeweils die nach dem Test gemäß der von Miles und Misra publizierten Methode gemessenen koloniebildenden Einheiten der überlebenden Bakterien, die als koloniebildenden Einheiten pro Milliliter (CFU/ml) angegeben sind.

Für den Photosensibilisator FL-AS-H-1 a wurden bei eine Konzentration von 10 µM FL-AS-H-1a in einer der Mikroemulsionen E1 bis E4 eine Reduzierung der Menge an E. coli und S. aureus von etwa 2 log₁₀-Sufen gemessen.

### Beispiel 2:

### A) Herstellung verschiedener Öl-haltiger Mikroemulsionen

Weiterhin wurden die ölhaltigen Mikroemulsionen E5 und E6 hergestellt.

Die nachfolgend angegebenen Gew.-% der Bestandteile der Mikroemulsionen E5 bis E6 beziehen sich jeweils auf das Gesamtgewicht der entsprechenden Mikroemulsion ohne Photosensibilisator
Mikroemulsion E5:
   66 Gew.-% Dodecan
   29 Gew.-% Lutensol AO7
   5 Gew.-% Wasser
Mikroemulsion E6:
   66 Gew.-% Paraffinöl
   4 Gew.-% Wasser
   10 Gew.-% Lutensol AO 7
   20 Gew.-% Kosteran SQ/O VH

Das Tensid Lutensol AO7 ist kommerziell erhältlich von BASF SE (Ludwigshafen, DE). Lutensol AO7 ist ein ethoxyliertes Gemisch aus Fettalkoholen mit 13 bis 15 Kohlenstoffatomen mit durchschnittlich 7 Ehyloxideinheiten (PEG 7).

Das Tensid Kosteran SQ/O VH ist kommerziell erhältlich von der Dr. W. Kolb AG (Hedingen, CH). Kosteran SQ/O VH ist ein Sorbitan-Ölsäureester mit durchschnittlich 1,5 Ölsäuremolekülen pro Molekül (Sorbitan Sesquioleat).

### B) UV/VIS-Messungen

Die Absorption des verwendeten Photosensibilisators FL-AS-H-1 a in den jeweiligen Mikroemulsionen E5 und E6 wurde durch Aufnahme eines Absorptions-Spektrums für einen Wellenlängebereich von 250nm bis 600 nm wie unter Beispiel 1 beschreiben bestimmt. Der Photosensibilisators FL-AS-H-2 wurde dabei in einer Konzentration von 10 µM in den Mikroemulsionen E5 und E6 sowie in Wasser gelöst.

Das Absorptionsspektrum von FL-AS-H-2 in Mikroemulsion E6 zeigt keine Verschiebung des Spektrums im Vergleich zu dem im Wasser gemessenen Spektrum. Lediglich die Intensität des Absorptionssignals ist höher als in Wasser oder in Mikroemulsion E5. Weiterhin wurde eine Absorptionsspektrum von FL-AS-H-2 in Mikroemulsion E5 und E6 sowie in Wasser nach Bestrahlung mit unterschiedlichen Lichtdosen gemessen.

Zur Bestrahlung wurde die Lichtquelle Blue V der Firma Waldmann genutzt, welche Licht von 380 bis 480 nm emittiert (Emissionsmaximum bei ca. 420 nm). Die applizierte Lichtdosis betrug von 5,5 J bis 990 J.

Es zeigte sich, dass der Photosensibilisators FL-AS-H-2 sowohl in Wasser als auch in den Mikroemulsionen E5 und E6 abgebaut wird. Der Abbau in Wasser erfolgt dabei signifikant schneller als in der jeweiligen Mikroemulsion E5 oder E6.

### Beispiel 3

### A) Herstellung Photosensibilisator-haltiger Gele

Die nachfolgend angegebenen Gew.-% der Bestandteile der Gele G1 bis G3 beziehen sich jeweils auf das Gesamtgewicht der verwendeten wässrigen Stammlösung.

**Gel G1: (Vergleichsbeispiel - kein Tensid)**

| Komponente | Menge [mL] |
|---|---|
| Carbopol SF-1 (4 Gew.-% wässrige Lösung) | 6.25 |
| Natriumhydroxid (2 Gew.-% wässrige Lösung) | 2 |
| Natriumchlorid (10 Gew.-% wässrige Lösung) | 4 |

Als Gelbildner wurde Carbopol Aqua SF-1 Polymer, ein Acrylat-Copolymer, das von der Lubrizol Corporation (Wickliffe, OH, USA) erworben wurde, verwendet.

**Gel G2:**

| Komponente | Menge [mL] |
|---|---|
| Carbopol SF-1 (4 Gew.-% wässrige Lösung) | 6.25 |
| Natriumhydroxid (2 Gew.-% wässrige Lösung) | 2 |
| Natriumchlorid (20 Gew.-% wässrige Lösung) | 2 |
| Brij 35 (6 Gew.-% wässrige Lösung) | 2 |

Als Tensid wurde Brij 35, ein Polyoxyethylen (23) laurylether, der von der Firma Merck KGaA (Darmstadt, DE) bezogen wurde, verwendet.

**Gel G3:**

| Komponente | Menge [mL] |
|---|---|
| Carbopol SF-1 (4 Gew.-% wässrige Lösung) | 6.25 |
| Natriumhydroxid (2 Gew.-% wässrige Lösung) | 2 |
| Natriumchlorid (20 Gew.-% wässrige Lösung) | 2 |
| PLANTACARE 818 UP (6 Gew.-% wässrige Lösung) | 2 |

Als Tensid wurde PLANTACARE 818 UP, ein C8 bis C16 Fettalkoholglukoside der D-Glucopyranose, das von der BASF SE (Ludwigshafen, DE) bezogen wurde, verwendet.

Gemäß Herstellangaben weist das Tensid eine Verteilung der Länge des Fettalkoholanteils von:

| | |
|---|---|
| C6 | max. 0.5 % |
| C8 | 24 - 30 % |
| C10 | 15 - 22 % |
| C12 | 37 - 42 % |
| C14 | 12 - 18 % |
| C16 | max. 4 % |

auf.

Zu einer entsprechenden Menge einer 4 Gew.-% wässrigen Lösung von Carbopol Aqua SF-1 wurde in einem Messbecher zunächst die oben angegebenen Menge einer 2 Gew.-% wässrige Lösung NaOH unter Rühren portionsweise hinzugegeben. Nachdem sich ein klares Gel gebildet hatte, wurde die oben angegebene Menge einer Natriumchlorid Lösung zur Einstellung der Viskosität hinzugegeben.

Anschließend wurde die jeweils oben angegebene Menge einer 6 Gew.-% wässrige Lösung eines der oben angegebenen Tenside unter Rühren hinzugetropft.

Der verwendete Photosensibilisator TMPyP wurde in einer Endkonzentration von 100 µM zu dem jeweiligen Gel hinzugegeben.

Die Gele G1, G2 und G3 jeweils mit und ohne Photosensibilisator TMPyP waren transparent und zeigten pseudoelastisches Verhalten.

Weiterhin veränderten die Gele G2 und G3 bei 24 stündiger Lagerung bei 50°C sowie bei 0°C nicht ihre Konsistenz.

### B) Kontaktwinkel-Prüfung

Die Benetzung von Oberflächen durch die hergestellten Gele wurde mit Hilfe der Kontaktwinkelprüfung bestimmt.

Für die Kontaktwinkelprüfung wurden die oben angegebenen Gele ohne Photosensibilisator sowie Photosensibilisator-haltige Gele verwendet.

Die Kontaktwinkel-Prüfung wurde wie in Beispiel 1 beschrieben durchgeführt, wobei als Prüfoberfläche eine Polyethylen-Testplatte verwendet wurde.

Exemplarisch sind in Figur 7 die gemessenen Kontaktwinkel der Photosensibilisator-freien Gele G2 und G3 gezeigt, bei denen die entsprechende Menge des angegebenen Tensids hinzugegeben wurde. Die gemessenen Kontaktwinkel der jeweils Photosensibilisator-haltigen Gele G2 und G3 sind identisch.

Die Messungen zeigten, dass bei einem Anteil von 0,5 Gew.-%, bezogen auf das Gesamtgewicht des Gels, sich ein minimaler Kontaktwinkel und somit eine maximale Benetzung erzielen ließ.

Um ggf. vorhandenen Bakterienaggregate aufzulösen, wurden im Folgenden der Anteil der Tenside auf 1,0 Gew.-%, bezogen auf das Gesamtgewicht des Gels, erhöht.

### C) UV/VIS-Messungen

Die Absorption des verwendeten Photosensibilisators TMPyP in den jeweiligen Gelen G1 bis G3 sowie in Wasser wurde durch Aufnahme eines Absorptions-Spektrums für einen Wellenlängebereich von 250nm bis 600 nm wie unter Beispiel 1 beschreiben bestimmt.

Der Photosensibilisators TMPyP wurde dabei in einer Konzentration von 10 µM in den Gelen G1 bis G3 sowie in Wasser gelöst.

Das Absorptionsspektrum von TMPyP in den Gelen G1 bis G3 zeigt keine Verschiebung des Spektrums im Vergleich zu dem im Wasser gemessenen Spektrum.

### D) Bestimmung von nach Bestrahlung gebildetem Singulett-Sauerstoff

Die Bildung von Singulett-Sauerstoff nach Bestrahlung einer Photosensibilisator-haltigen Mikroemulsion wurde durch Zeit-aufgelöste Singulett-Sauerstoff Lumineszenzenzmessungen wie unter Beispiel 1 beschrieben bestimmt.

In den Gelen G1, G2 und G3 konnte in Gegenwart von TMPyP (10 µM Endkonzentration) nach Bestrahlung die Bildung von Singulett-Sauerstoff nachgewiesen werden.

In Figur 8 ist exemplarisch das Zeit-aufgelöste Singulett-Sauerstoff Spektrum für den Photosensibilisator TMPyP in Gel G3 dargestellt. Die gemessene Anstiegszeit des Signals (t_{R}) betrug 2,7 µs.

Die gemessene Abklingzeit des Signals (t_{D}) betrug 7,4 µs.

In Figur 9 ist exemplarisch das Wellenlängen-aufgelöste Singulett-Sauerstoff Spektrum für den Photosensibilisator TMPyP in Gel G3 dargestellt.

Der eindeutige Peak im Wellenlängen-aufgelösten Spektrum bei 1270 nm, zeigt eindeutig, dass nach Bestrahlung Singulett-Sauerstoff durch TMPyP im Gel gebildet wird.

Die gemessene Abklingzeit des Singulett-Sauerstoff-Signals im Gel (7,4 µs) ist im Vergleich zu der gemessenen Abklingzeit des Singulett-Sauerstoff-Signals in Wasser (~ 3.5 µs) signifikant verlängert, so dass der gebildete Singulett-Sauerstoff in einem der getesteten Gele G1 bis G3 länger wirksam ist.

### Literatur:

Butenandt J., Epple R., Wallenborn E.-U., Eker A.P.M., Gramlich V. und Carell T.: A comparative repair study of thymine- and uracil-photodimers with model compounds and a photolyase repair enzyme, Chem. Eur. J. 2000, Vol. 6, No. 1, Seiten 62 - 72,
Svoboda J., Schmaderer H. und König B.: Thiourea-enhanced flavin photooxidation of benzyl alcohol; Chem. Eur. J. 2008, 14, Seiten 1854 - 1865
Egorov S.Y., Krasnovsky A.A., Bashtanov M.Y., Mironov E.A., Ludnikova T.A. und Kritsky M.S.; Photosensitization of singlet oxygen formation by pterins and flavins. Time-resolved studies of oxygen phosphorescence under laser excitation. Biochemistry (Mosc)1999, 64 (10), Seiten 1117 - 1121.

## Patentansprüche

1. Dispersion,
umfassend:
(a) wenigstens einen Photosensibilisator, der aus der Gruppe, die aus Phenalenonen, Flavinen und Mischungen davon besteht, ausgewählt wird,
(b) wenigstens eine flüssige, polare Phase, und
(c) wenigstens ein Tensid, und
wobei die Dispersion bei einer Temperatur aus einem Bereich von 2 bis 50°C und einem Druck in einem Bereich von 800 bis 1200 mbar eine Mikroemulsion, ein Gel oder eine Mischung davon umfasst.

2. Dispersion nach Anspruch 1, wobei die wenigstens eine flüssige, polare Phase wenigstens ein polares Lösungsmittel, vorzugsweise Wasser, umfasst.

3. Dispersion nach einem der Ansprüche 1 bis 2, wobei das wenigstens eine Tensid aus der Gruppe, die aus nichtionischen Tensiden, anionischen Tensiden, kationischen Tensiden, amphoteren Tensiden und Mischungen davon, vorzugsweise nichtionischen Tensiden, anionischen Tensiden und Mischungen davon, besteht, ausgewählt wird.

4. Dispersion nach Anspruch 3, wobei kationische Tenside aus der Gruppe, die aus quartären Alkylammoniumsalzen, Esterquats, acylierten Polyaminen, Benzylammoniumsalzen oder Mischungen davon besteht, ausgewählt werden.

5. Dispersion nach einem der Ansprüche 3 oder 4, wobei nichtionische Tenside aus der Gruppe, die aus Polyalkylenglycolethern, Alkylglucosiden, Alkylpolyglycosiden, Alkylglycosidestern und Mischungen davon besteht, ausgewählt werden.

6. Dispersion nach einem der Ansprüche 3 bis 5, wobei anionischen Tenside aus der Gruppe, die aus Alkylcarboxylaten, Alkylsulfonaten, Alkylsulfaten, Alkylphoshaten, Alkylpolyglykolethersulfaten, Sulfonaten von Alkylcarbonsäureestern, N-Alkyl-Sarkosinaten und Mischungen davon besteht, ausgewählt werden.

7. Dispersion nach einem der Ansprüche 1 bis 6, wobei die Dispersion weiterhin wenigstens eine flüssige, unpolare Phase umfasst, wobei die wenigstens eine flüssige, unpolare Phase wenigstens ein unpolares Lösungsmittel, das vorzugsweise aus der Gruppe, die aus Alkanen mit 6 bis 30 Kohlenstoffatomen, Monocarbonsäureestern mit vorzugsweise 4 bis 20 Kohlenstoffatomen, Polycarbonsäureestern mit vorzugsweise 6 bis 20 Kohlenstoffatomen und Mischungen davon besteht, ausgewählt wird, umfasst.

8. Dispersion nach einem der Ansprüche 1 bis 7, wobei die Dispersion weiterhin wenigstens ein Alkanol mit 2 bis 12 Kohlenstoffatomen, und vorzugsweise mit 1 bis 6 OH-Gruppen, enthält.

9. Dispersion nach einem der Ansprüche 1 bis 8, wobei die Dispersion bei einem Druck aus einem Bereich von 800 bis 1200 mbar und einer Temperatur aus einem Bereich von 2 bis 50°C eine Mikroemulsion umfasst oder ist.

10. Dispersion nach Anspruch 9, wobei die Mikroemulsion Tröpfchen mit einer Tröpfchengröße von kleiner als 350 nm aufweist.

11. Dispersion nach Anspruch 9, wobei die Mikroemulsion eine O/W-Mikroemulsion, eine Wasser-in-Öl (W/O) Mikroemulsion oder eine bikontinuierliche Mikroemulsion ist.

12. Dispersion nach einem der Ansprüche 1 bis 6, wobei die Dispersion weiterhin wenigstens eine pH-regulierende Substanz enthält, die vorzugsweise eine anorganische Säure, organische Säure, anorganische Base, organische Base, ein Salz davon oder eine Mischung davon ist.

13. Dispersion nach einem der Ansprüche 1 bis 6 oder 12, wobei die Dispersion weiterhin wenigstens einen Gelbildner, der aus der Gruppe, die aus Carboxyvinylpolymeren, Polyacrylamiden, Polyvinylakoholen, acylierten Polyethylenaminen, Alginaten, Celluloseethern und Mischungen davon ausgewählt wird, umfasst.

14. Dispersion nach einem der Ansprüche 1 bis 6, 12 oder 13, wobei die Dispersion bei einem Druck aus einem Bereich von 800 bis 1200 mbar und einer Temperatur aus einem Bereich von 2 bis 50°C ein Gel umfasst oder ist.

15. Nicht-medizinische Verwendung einer Dispersion nach einem der Ansprüche 1 bis 14 zur photodynamischen Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden.

16. Nicht-medizinische Verwendung nach Anspruch 15 zur Oberflächenreinigung und/oder Oberflächenbeschichtung eines Gegenstandes.

17. Nicht-medizinische Verwendung nach einem der Ansprüche 15 oder 16 zur Oberflächenreinigung und/oder Oberflächenbeschichtung von Medizinprodukten, Lebensmittelverpackungen, Lebensmitteln, Getränkeverpackungen, Getränkebehältern, Textilien, Baustoffen, elektronische Geräten, Haushaltsgeräten, Möbeln, Fenstern, Böden, Wänden oder Hygieneartikeln.

18. Nicht-medizinische Verwendung nach einem der Ansprüche 15 oder 16 zur Entkeimung von Flüssigkeiten.

19. Nicht-medizinisches Verfahren zur photodynamischen Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, wobei das Verfahren folgende Schritte umfasst:
(A) in Kontakt bringen der Mikroorganismen mit wenigstens einer Dispersion nach einem der Ansprüche 1 bis 14, und
(B) Bestrahlen der Mikroorganismen und wenigstens eines, in der Dispersion enthaltenen Photosensibilisators mit elektromagnetischer Strahlung geeigneter Wellenlänge und Energiedichte.

20. Dispersion nach einem der Ansprüche 1 bis 14 zur Anwendung bei der photodynamischen Therapie zur Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden.

21. Dispersion nach einem der Ansprüche 1 bis 14 zur Anwendung nach Anspruch 20 bei der Behandlung und/oder Prophylaxe einer Erkrankung des Zahngewebes und/oder des Zahnhalteapparates.

## Claims

1. Dispersion,
comprising:
(a) at least one photosensitizer selected from the group consisting of phenalenones, flavins and mixtures thereof,
(b) at least one liquid polar phase, and
(c) at least one surfactant, and
wherein the dispersion at a temperature from a range of 2 to 50°C and a pressure in a range of 800 to 1200 mbar comprises a microemulsion, a gel or a mixture thereof.

2. Dispersion according to Claim 1, wherein the at least one liquid polar phase comprises at least one polar solvent, preferably water.

3. Dispersion according to either of Claims 1 and 2, wherein the at least one surfactant is selected from the group consisting of nonionic surfactants, anionic surfactants, cationic surfactants, amphoteric surfactants and mixtures thereof, preferably nonionic surfactants, anionic surfactants and mixtures thereof.

4. Dispersion according to Claim 3, wherein cationic surfactants are selected from the group consisting of quaternary alkylammonium salts, esterquats, acylated polyamines, benzylammonium salts or mixtures thereof.

5. Dispersion according to either of Claims 3 and 4, wherein nonionic surfactants are selected from the group consisting of polyalkylene glycol ethers, alkyl glucosides, alkyl polyglycosides, alkyl glycoside esters and mixtures thereof.

6. Dispersion according to any of Claims 3 to 5, wherein anionic surfactants are selected from the group consisting of alkyl carboxylates, alkyl sulfonates, alkyl sulfates, alkyl phosphates, alkyl polyglycol ether sulfates, sulfonates of alkyl carboxylates, N-alkyl sarcosinates and mixtures thereof.

7. Dispersion according to any of Claims 1 to 6, wherein the dispersion additionally comprises at least one liquid nonpolar phase, wherein the at least one liquid nonpolar phase comprises at least one nonpolar solvent preferably selected from the group consisting of alkanes having 6 to 30 carbon atoms, monocarboxylic esters having preferably 4 to 20 carbon atoms, polycarboxylic esters having preferably 6 to 20 carbon atoms and mixtures thereof.

8. Dispersion according to any of Claims 1 to 7, wherein the dispersion additionally comprises at least one alkanol having 2 to 12 carbon atoms and preferably having 1 to 6 OH groups.

9. Dispersion according to any of Claims 1 to 8, wherein the dispersion at a pressure from a range of 800 to 1200 mbar and a temperature from a range of 2 to 50°C comprises or is a microemulsion.

10. Dispersion according to Claim 9, wherein the microemulsion comprises droplets having a droplet size of less than 350 nm.

11. Dispersion according to Claim 9, wherein the microemulsion is an O/W microemulsion, a water-in-oil (W/O) microemulsion or a bicontinuous microemulsion.

12. Dispersion according to any of Claims 1 to 6, wherein the dispersion additionally comprises at least one pH-regulating substance that is preferably an inorganic acid, organic acid, inorganic base, organic base, a salt thereof or a mixture thereof.

13. Dispersion according to any of Claims 1 to 6 or 12, wherein the dispersion additionally comprises at least one gel-forming agent selected from the group of carboxyvinyl polymers, polyacrylamides, polyvinyl alcohols, acylated polyethylene amines, alginates, cellulose ethers and mixtures thereof.

14. Dispersion according to any of Claims 1 to 6, 12 or 13, wherein the dispersion at a pressure from a range of 800 to 1200 mbar and a temperature from a range of 2 to 50°C comprises or is a gel.

15. Non-medical use of a dispersion according to any of Claims 1 to 14 for the photodynamic inactivation of microorganisms preferably selected from the group consisting of viruses, archaea, bacteria, bacterial spores, fungi, fungal spores, protozoa, algae and blood-borne parasites.

16. Non-medical use according to Claim 15 for the surface-cleaning and/or surface-coating of an article.

17. Non-medical use according to either of Claims 15 and 16 for the surface-cleaning and/or surface-coating of medical devices, food packagings, foodstuffs, beverage packagings, beverage containers, textiles, building materials, electronic devices, household appliances, furniture, windows, floors, walls or toiletries.

18. Non-medical use according to either of Claims 15 and 16 for the disinfection of liquids.

19. Non-medical use for the photodynamic inactivation of microorganisms preferably comprising viruses, archaea, bacteria, bacterial spores, fungi, fungal spores, protozoa, algae, blood-borne parasites or combinations thereof, wherein the process comprises the following steps:
(A) contacting the microorganisms with at least one dispersion according to any of Claims 1 to 14, and
(B) irradiating the microorganisms and at least one photosensitizer present in the dispersion with electromagnetic radiation of suitable wavelength and energy density.

20. Dispersion according to any of Claims 1 to 14 for use in photodynamic therapy for the inactivation of microorganisms preferably selected from the group consisting of viruses, archaea, bacteria, bacterial spores, fungi, fungal spores, protozoa, algae and blood-borne parasites.

21. Dispersion according to any of Claims 1 to 14 for use according to Claim 20 in the treatment and/or prophylaxis of a pathology of the dental tissue and/or of the periodontium.

## Revendications

1. Dispersion,
comprenant :
(a) au moins un photosensibilisant qui est sélectionné dans le groupe constitué de phénalénones, de flavines et de mélanges de ceux-ci,
(b) au moins une phase liquide polaire et
(c) au moins un tensio-actif et
dans laquelle la dispersion comprend, à une température dans un intervalle de 2 à 50 °C et à une pression dans un intervalle de 800 à 1 200 mbar, une microémulsion, un gel ou un mélange de ceux-ci.

2. Dispersion selon la revendication 1, dans laquelle l'au moins une phase liquide polaire comprend au moins un solvant polaire, de préférence de l'eau.

3. Dispersion selon l'une des revendications 1 à 2, dans laquelle l'au moins un tensio-actif est sélectionné dans le groupe constitué de tensio-actifs non ioniques, de tensio-actifs anioniques, de tensio-actifs cationiques, de tensio-actifs amphotères et de mélanges de ceux-ci, de préférence de tensio-actifs non ioniques, de tensio-actifs anioniques et des mélanges de ceux-ci.

4. Dispersion selon la revendication 3, dans laquelle les tensio-actifs cationiques sont sélectionnés dans le groupe constitué de sels d'alkylammonium quaternaires, d'esters quaternaires, de polyamines acylés, de sels de benzylammonium ou de mélanges de ceux-ci.

5. Dispersion selon l'une des revendications 3 ou 4, dans laquelle les tensio-actifs non ioniques sont sélectionnés dans le groupe constitué de polyalkylènes-glyco-éthers, d'alkylglucosides, d'alkylpolyglycosides, d'alkylglycosides-esters et de mélanges de ceux-ci.

6. Dispersion selon l'une des revendications 3 à 5, dans laquelle les tensio-actifs anioniques sont sélectionnés dans le groupe constitué d'alkylcarboxylates, l'akylsulfonates, d'alkylsulfates, d'alkylphosphates, d'alkylpolyglycoléthersulfates, de sulfonates et d'esters d'acides alkylcarboxyliques, de N-alkyl-sarkosinates et de mélanges de ceux-ci.

7. Dispersion selon l'une des revendications 1 à 6, dans laquelle la dispersion comprend en outre au moins une phase liquide non polaire, dans laquelle l'au moins une phase liquide non polaire comprend au moins un solvant non polaire, qui est sélectionné dans le groupe constitué d'alcanes avec 6 à 30 atomes de carbone, d'esters d'acides monocarboxyliques avec de préférence 4 à 20 atomes de carbone, d'esters d'acides polycarboxyliques avec de préférence 6 à 20 atomes de carbone et de mélanges de ceux-ci.

8. Dispersion selon l'une des revendications 1 à 7, dans laquelle la dispersion contient en outre au moins un alkanol avec 2 à 12 atomes de carbone et de préférence avec 1 à 6 groupes OH.

9. Dispersion selon l'une des revendications 1 à 8, dans laquelle la dispersion comprend, à une pression dans un intervalle de 800 à 1 200 mbar et à une température dans un intervalle de 2 à 50 °C, une micro-émulsion.

10. Dispersion selon la revendication 9, dans laquelle la micro-émulsion comprend des gouttelettes avec une taille de gouttelettes inférieure à 350 nm.

11. Dispersion selon la revendication 9, dans laquelle la micro-émulsion est une micro-émulsion huile/eau (O/W), une micro-émulsion eau-dans-huile (W/O) ou une micro-émulsion bicontinue.

12. Dispersion selon l'une des revendications 1 à 6, dans laquelle la dispersion contient en outre au moins une substance régulant le pH, qui est, de préférence un acide anorganique, un acide organique, une base anorganique, une base organique, un sel de ceux-ci ou un mélange de ceux-ci.

13. Dispersion selon l'une des revendications 1 à 6 ou 12, dans laquelle la dispersion comprend au moins un agent gélifiant qui est sélectionné dans le groupe constitué de polymères carboxyvinyles, de polyacrylamides, d'alcools polyvinyliques, de polyétheramines acylés, d'alginates, d'éthers de cellulose et de mélanges de ceux-ci.

14. Dispersion selon l'une des revendications 1 à 6, 12 ou 13, dans laquelle la dispersion comprend ou est un gel à une pression dans un intervalle de 800 à 1 200 mbar et à une température dans un intervalle de 2 à 50 °C.

15. Utilisation non médicale d'une dispersion selon l'une des revendications 1 à 14 pour l'inactivation photodynamique de micro-organismes, qui sont sélectionnés, de préférence, dans le groupe constitué de virus, d'archéobactéries, de bactéries, de spores de bactéries, de champignons, de spores de champignons, de protozoaires, d'algues et de parasites transmissibles par le sang.

16. Utilisation non médicale selon la revendication 15 pour le nettoyage de surfaces et/ou le revêtement de surface d'un objet.

17. Utilisation non médicale selon l'une des revendications 15 ou 16, pour le nettoyage de surfaces et/ou le revêtement de surface de produites médicaux, d'emballages alimentaires, d'aliments, d'emballages de boissons, de récipients de boissons, de textiles, de matériaux de construction, d'appareils électroniques, d'appareils électro-ménagers, de meubles, de fenêtres, de sols, de murs ou d'articles d'hygiène.

18. Utilisation non médicale selon l'une des revendications 15 ou 16, pour la désinfection de liquides.

19. Procédé non médical d'inactivation photodynamique de micro-organismes, qui comprennent de préférence des virus, des archéobactéries, des bactéries, des spores de bactéries, des champignons, des spores de champignons, des protozoaires, des algues, des parasites transmissibles par le sang ou des combinaisons de ceux-ci, dans lequel le procédé comprend les étapes suivantes :
(A) mise en contact des micro-organismes avec au moins une dispersion selon l'une des revendications 1 à 14 et
(B) irradiation des micro-organismes et d'au moins un photosensibilisant contenu dans la dispersion avec un rayonnement électromagnétique d'une longueur d'onde et d'une densité énergétique appropriées.

20. Dispersion selon l'une des revendications 1 à 14, destinée à être utilisée lors d'une thérapie photodynamique pour l'activation de micro-organismes, qui est sélectionnée de préférence dans un groupe constitué de virus, d'archéobactéries, de bactéries, de spores de bactéries, de champignons, de spores de champignons, de protozoaires, d'algues et de parasites transmissibles par le sang.

21. Dispersion selon l'une des revendications 1 à 14, destinée à être utilisée selon la revendication 20 pour le traitement et/ou la prophylaxie d'une maladie du tissu dentaire et/ou du parodonte.
